(19) Europäisches Patentamt
European Patent Office
Office européen des brevets

(11) Veröffentlichungsnummer: **0 285 837 B1**

(12) **EUROPÄISCHE PATENTSCHRIFT**

(45) Veröffentlichungstag der Patentschrift: **08.07.92**

(51) Int. Cl.[5]: **C07D 487/04**, C07D 495/14, A61K 31/55, //(C07D487/04, 243:00,235:00),(C07D495/14, 333:00,243:00,235:00)

(21) Anmeldenummer: **88103739.4**

(22) Anmeldetag: **09.03.88**

Die Akte enthält technische Angaben, die nach dem Eingang der Anmeldung eingereicht wurden und die nicht in dieser Patentschrift enthalten sind.

# (54) Imidazodiazepin-Derivate.

(30) Priorität: **10.03.87 CH 880/87**
**08.01.88 CH 59/88**

(43) Veröffentlichungstag der Anmeldung:
**12.10.88 Patentblatt 88/41**

(45) Bekanntmachung des Hinweises auf die Patenterteilung:
**08.07.92 Patentblatt 92/28**

(84) Benannte Vertragsstaaten:
**AT BE CH DE ES FR GB GR IT LI LU NL SE**

(56) Entgegenhaltungen:
**EP-A- 0 027 214**
**EP-A- 0 059 390**
**EP-A- 0 109 921**
**EP-A- 0 150 040**
**EP-A- 0 202 441**
**DE-A- 2 540 522**

(73) Patentinhaber: **F. HOFFMANN-LA ROCHE AG**
**Postfach 3255**
**CH-4002 Basel(CH)**

(72) Erfinder: **Hunkeler, Walter, Dr.**
**Im Stigler 32**
**CH-4312 Magden(CH)**
Erfinder: **Kyburz, Emilio, Dr.**
**Unterer Rebbergweg 127**
**CH-4153 Reinach(CH)**
Erfinder: **Meier, Marc**
**75 av. Gen. de Gaulle**
**F-68300 Village-Neuf(FR)**

(74) Vertreter: **Lederer, Franz, Dr. et al**
**Patentanwalt Dr. Franz Lederer Lucile-Grahn-Strasse 22**
**W-8000 München 80(DE)**

EP 0 285 837 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Imidazodiazepin-Derivate. Im speziellen betrifft sie Imidazodiazepin-Derivate der allgemeinen Formel

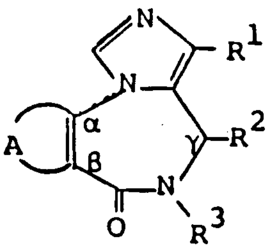

I

worin A zusammen mit den beiden mit $\alpha$ und $\beta$ bezeichneten Kohlenstoffatomen eine der Gruppen

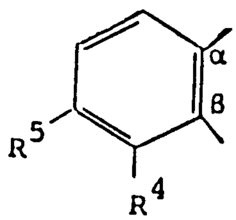

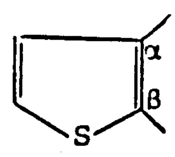

und

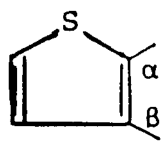

(a) (b) (c)

$R^1$ eine der Gruppen

$$-CH=CH-R^6 \quad \text{und} \quad -C{\equiv}C-R^6,$$

(d) (e)

$R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl und $R^6$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeuten, wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen oder Trimethylen bedeuten, und wobei die in Gruppe (d) vorhandene Doppelbindung die E- und/oder Z-Konfiguration aufweist, wenn $R^6$ von Wasserstoff verschieden ist, und die mit "nieder" bezeichneten Reste bis zu 7 Kohlenstoffatome besitzen.
Diese Verbindungen sind neu und zeichnen sich durch wertvolle pharmakodynamische Eigenschaften aus.

Gegenstand der vorliegenden Erfindung sind die Verbindungen der obigen Formel I als solche und als therapeutische Wirkstoffe, Verfahren und Zwischenprodukte zu deren Herstellung, Arzneimittel, enthaltend eine Verbindung der Formel I und einen therapeutisch inerten Träger, die Herstellung solcher Arzneimittel und die Verwendung von Verbindungen der Formel I bei der Bekämpfung oder Verhütung von Krankheiten (insbesondere bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten) bzw. die Verwendung von

Verbindungen der Formel I zur Herstellung von Arzneimitteln, insbesondere von Arzneimitteln zur Anwendung bei den soeben erwähnten Indikationen.

Der Ausdruck "nieder" wird verwendet, um Reste und Verbindungen mit bis zu 7, vorzugsweise bis zu 4 Kohlenstoffatomen zu bezeichnen. Der Ausdruck "niederes Alkyl" bezeichnet geradkettige oder verzweigte gesättigte Kohlenwasserstoffreste mit höchstens 7, vorzugsweise höchstens 4 Kohlenstoffatomen, wie Methyl, Aethyl, n-propyl, Isopropyl, n-Butyl, Isobutyl, s-Butyl, t-Butyl und dergleichen. Der Ausdruck "niederes Alkoxy" bezeichnet über ein Sauerstoffatom gebundene niedere Alkylreste im Sinne der vorstehenden Definition des Ausdruckes "niederes Alkyl". Falls nichts anderes angegeben ist, bezeichnet der Ausdruck "Halogen" die vier Halogene Fluor, Chlor, Brom und Jod.

Der Ausdruck "gesättigte Kohlenwasserstoffgruppe" bezeichnet offenkettige und cyclische Gruppen und Kombinationen davon. Die offenkettigen Gruppen können geradkettig oder verzweigt sein. Beispiele für gesättigte niedere Kohlenwasserstoffgruppen sind: Methyl, Aethyl, i-Propyl, t-Butyl, 3-Pentyl, Cyclopropyl, Cyclobutyl, Cyclopentyl, Cyclopropylmethyl, Dicyclopropylmethyl und 1-Cyclopropyläthyl. Beispiele für gesättigte niedere Kohlenwasserstoffreste, welche durch Hydroxy, niederes Alkoxy, ($C_3$-$C_7$)-Cycloalkyl oder Oxo mono- oder disubstituiert sind, sind: Hydroxymethyl, Methoxymethyl, Dimethoxymethyl, 1-Hydroxyäthyl, 1-Methoxyäthyl, 1-Hydroxypropyl, 2-Hydroxy-2-propyl, 2-Methoxy-2-propyl, 2-Aethoxy-2-propyl, 3-Hydroxy-3-pentyl, 1-Hydroxy-1-cyclobutyl, 1-Hydroxy-1-cyclopentyl, 1-Methoxy-1-cyclopentyl, 1-Oxoäthyl und Dicyclopropylhydroxymethyl.

Wenn $R^1$ in Formel I eine Gruppe der Formel (d) bedeutet, so steht es beispielsweise für Vinyl, 1-Propenyl, 1-Pentenyl oder 2-Chlorvinyl. Vorzugsweise aber bedeutet $R^1$ in Formel I einen Rest der Formel (e). $R^6$ bedeutet vorzugsweise Wasserstoff, niederes Alkyl, niederes Hydroxyalkyl, niederes Alkoxyalkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl, ($C_3$-$C_7$)-Cycloalkyl-niederes Hydroxyalkyl oder ($C_3$-$C_7$)-Cycloalkyl-niederes Alkoxyalkyl. In einer ganz besonders bevorzugten Ausführungsform bedeutet $R^6$ Wasserstoff, niederes Alkyl, niederes 1-Hydroxyalkyl, niederes 1-Alkoxyalkyl, ($C_3$-$C_7$)-Cycloalkyl, 1-Hydroxy-($C_4$-$C_7$)-cycloalkyl, 1-(niederes Alkoxy)-($C_4$-$C_7$)-cycloalkyl oder 1-[($C_3$-$C_7$)-Cycloalkyl]-niederes 1-Hydroxyalkyl, insbesondere niederes Alkyl, niederes 1-Hydroxyalkyl oder ($C_3$-$C_7$)-Cycloalkyl, beispielsweise Methyl, Aethyl, i-Propyl, t-Butyl, 3-Pentyl, Hydroxymethyl, 1-Hydroxyäthyl, 1-Hydroxypropyl, 2-Hydroxy-2-propyl, 3-Hydroxy-3-pentyl oder Cyclopropyl.

Wenn $R^2$ Wasserstoff und $R^3$ niederes Alkyl bedeuten, dann steht $R^3$ zweckmässigerweise für Methyl. Wenn $R^2$ und $R^3$ zusammen Dimethylen oder Trimethylen bedeuten, dann weist das mit $\gamma$ bezeichnete Kohlenstoffatom vorzugsweise die (S)-Konfiguration auf.

Wenn A einen Rest der Formel (a) bedeutet, dann bedeutet zeckmässigerweise eines von $R^4$ und $R^5$ Wasserstoff und das andere Wasserstoff oder Halogen; dabei bedeuten beispielsweise $R^4$ und $R^5$ beide Wasserstoff oder $R^4$ Wasserstoff und $R^5$ Fluor oder $R^4$ Chlor und $R^5$ Wasserstoff.

Im Rahmen der vorliegenden Erfindung bevorzugte Verbindungen der Formel I sind:

7-Chlor-4,5-dihydro-5-methyl-3-(1-propinyl)-6H-imidazo[1,5-a][1,4]benzodiazepin-6-on;

7-Chlor-4,5-dihydro-3-(3-hydroxy-3-methyl-1-butinyl)-5-methyl-6H-imidazo[1,5-a][1,4]benzodiazepin-6-on,

7-Brom-4,5-dihydro-3-(3-hydroxy-3-methyl-1-butinyl)-5-methyl-6H-imidazo[1,5-a][1,4]benzodiazepin-6-on,

7-Chlor-4,5-dihydro-3-(3-hydroxy-1-butinyl)-5-methyl-6H-imidazo[1,5-a][1,4]benzodiazepin-6-on und

4,5-Dihydro-5-methyl-3-(1-propinyl)-6H-imidazo[1,5-a][1,4]benzodiazepin-6-on,

7-Chlor-4,5-dihydro-5-methyl-3-(3-methyl-1-butinyl)-6H-imidazo[1,5-a][1,4]benzodiazepin-6-on,

7-Chlor-4,5-dihydro-3-(3-hydroxy-1-propinyl)-5-methyl-6H-imidazo[1,5-a][1,4]benzodiazepin-6-on und

7-Chlor-3-(cyclopropyläthinyl)-4,5-dihydro-5-methyl-6H-imidazo[1,5-a][1,4]benzodiazepin-6-on.

Die Verbindungen der Formel I können erfindungsgemäss dadurch hergestellt werden, dass man

a) eine Verbindung der allgemeinen Formel

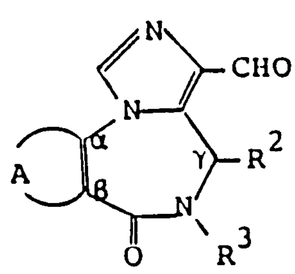

II

worin A, $R^2$ und $R^3$ obige Bedeutung besitzen,
mit einer Verbindung der allgemeinen Formel

$(Ar)_3P=CH\text{-}R^{61}$ III

worin $R^{61}$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch niederes Alkoxy, ($C_3$-$C_7$)-Cycloalkyl oder Oxo mono- oder disubstituierte gesättigte niedere Kohlenwasserstoffgruppe und Ar einen monocyclischen aromatischen Kohlenwasserstoffrest, welcher durch niederes Alkyl, niederes Alkoxy oder Halogen substituiert sein kann, bedeuten,
umsetzt; oder
b) eine Verbindung der allgemeinen Formel

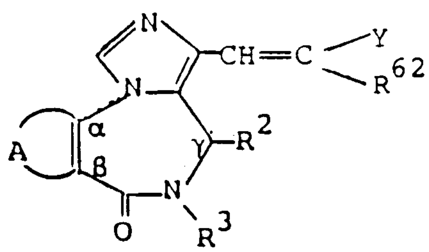

IV

worin $R^{62}$ Wasserstoff oder Halogen und Y Halogen bedeuten, und A, $R^2$ und $R^3$ obige Bedeutung besitzen,
dehydrohalogeniert; oder
c) eine Verbindung der Formel I, worin $R^1$ die Gruppe (e) und $R^6$ Wasserstoff bedeuten, mit einem einen gegebenenfalls durch Hydroxy, niederes Alkoxy, ($C_3$-$C_7$)-Cycloalkyl oder Oxo mono- oder disubstituierten gesättigten niederen Kohlenwasserstoff- oder einen monocyclischen aromatischen Kohlenwasserstoffrest, welcher durch niederes Alkyl, niederes Alkoxy oder Halogen substituiert sein kann, oder Halogen liefernden Mittel behandelt; oder
d) eine Verbindung der allgemeinen Formel

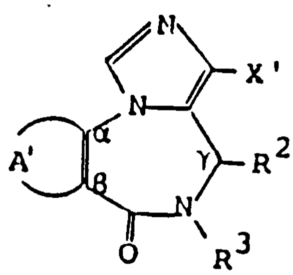

VI

worin $R^2$ und $R^3$ obige Bedeutung besitzen und X' Brom oder Jod und A' einen Rest der Formel (a), (b) oder (c) bedeutet, wobei aber im Falle, dass A' einen Rest der Formel (a) und $R^4$ und/oder $R^5$ Halogen bedeuten, dieses Halogen Fluor oder Chlor ist, wenn X'Brom bedeutet, bzw. Fluor, Chlor oder Brom ist, wenn X'Jod bedeutet, mit einer Verbindung der allgmeinen Formel

$HC\equiv C\text{-}R^{64}$ VII

worin $R^{64}$ Wasserstoff, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeutet,
umsetzt; oder
e) aus einer Verbindung der allgemeinen Formel

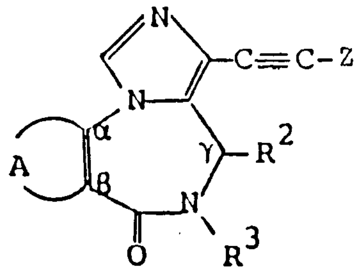

VIII

worin $R^2$, $R^3$ und A obige Bedeutung besitzen und Z eine Schutzgruppe bedeutet,
die Schutzgruppe abspaltet; oder
f) in einer Verbindung der allgemeinen Formel

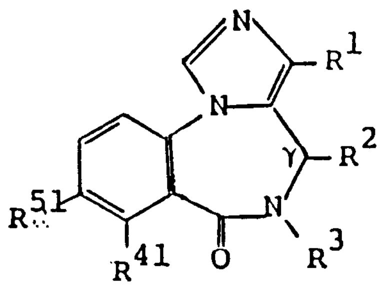

IX

worin $R^1$, $R^2$, $R^3$ und "nieder" obige Bedeutung besitzen und eines von $R^{41}$ und $R^{51}$ Amino und das andere Wasserstoff, Halogen, Trifluormethyl oder niederes Alkyl bedeutet,
die Aminogruppe durch eine Wasserstoff- oder Halogenatom ersetzt; oder
g) eine Verbindung der Formel I, worin $R^1$ einen Rest der Formel (e) bedeutet und worin im Falle, dass A einen Rest der Formel (a) bedeutet, $R^4$ und/oder $R^5$ nicht Jod bedeutet, zur entsprechenden Verbindung der Formel I, worin $R^1$ einen Rest der Formel (d) bedeutet, reduziert; oder
h) eine Verbindung der Formel I, worin $R^1$ einen Rest der Formel (d) oder (e) und $R^6$ eine durch Hydroxy substituierte gesättigte niedere Kohlenwasserstoffgruppe bedeuten, mit einem einen niederen Alkylrest liefernden Mittel behandelt; oder
i) einer Verbindung der Formel I, worin $R^1$ die Gruppe (d) oder (e) und $R^6$ eine durch Oxo substituierte gesättigte niedere Kohlenwasserstoffgruppe bedeuten, die Carbonylgruppe reduziert.

Aspekt a) des erfindungsgemässen Verfahrens liefert Verbindungen der Formel I, worin $R^1$ die Gruppe (d) bedeutet, worin aber $R^6$ nur diejenigen Bedeutungen haben kann, welche oben, im Zusammenhang mit Formel III, für $R^{61}$ angegeben worden sind. Die als Ausgangsprodukte verwendeten Verbindungen der Formel II sind bekannt oder nach an sich bekannten und jedem Fachmann geläufigen Methoden leicht herstellbar; zudem enthalten mehrere der nachfolgenden Beispiele detaillierte Angaben betreffend die Herstellung spezifischer Verbindungen der Formel II. Die Verbindungen der Formel III werden zweckmässigerweise in situ hergestellt, und zwar aus entsprechenden Phosphoniumhalogeniden, wie Methyltriphenylphosphoniumbromid, Aethyltriphenylphosphoniumbromid, Butyltriphenylphosphoniumbromid, Chlormethyltriphenylphosphoniumchlorid etc., und einer starken Base, wie Natriumamid, Butyllithium und dergleichen. Beispielsweise kann man so vorgehen, dass man das betreffende Phosphoniumhalogenid, wie Aethyltriphenylphosphoniumbromid, in einem unter den Reaktionsbedingungen inerten organischen Lösungsmittel, wie Tetrahydrofuran, Aether, N,N-Dimethylformamid, Toluol oder dergleichen, vorlegt und dann eine ungefähr äquimolare Menge oder einen geringen Ueberschuss einer geeigneten starken Base zugibt, beispielsweise indem man eine Butyllithiumlösung in einem unter den Reaktionsbedingungen inerten organischen Lösungsmittel, wie n-Hexan oder dergleichen, zutropft. Nach einer anderen Ausführungsart erfolgt die Herstellung der Ausgangsprodukte der Formel III zweckmässigerweise ausgehend von äquimolaren Mischungen von Natriumamid und einem Phosphoniumhalogenid, wie Methyltriphenylphosphoniumbromid,Butyltriphenylphosphoniumbromid, Chlormethyltriphenylphosphoniumchlorid und dergleichen, welche zum Teil im Handel erhältlich sind; derartige Mischungen können direkt eingesetzt werden, indem man sie in einem unter den Reaktionsbedingungen inerten organischen Lösungsmittel, wie Tetrahydrofuran, Aether, N,N-Dimethylformamid, Toluol, Dioxan oder dergleichen, aufnimmt. Die eine Verbindung der Formel III enthaltende Lösung oder Suspension, welche nach einer der vorstehend beschriebenen Methoden erhalten

worden ist, wird dann mit einer Verbindung der Formel II versetzt. Dabei ist es zweckmässig, die Verbindung der Formel II in fester Form portionenweise zuzugeben oder eine Lösung einer Verbindung der Formel II in einem unter den Reaktionsbedingungen inerten organischen Lösungsmittel, wie Tetrahydrofuran, Dioxan, Aether oder dergleichen, zuzutropfen. Je nach der Natur der als Reaktionskomponenten eingesetzten Verbindungen und des als Reaktionsmedium verwendeten Lösungsmittels oder Lösungsmittelgemisches erfolgt die Umsetzung der Verbindungen der Formel III mit den Verbindungen der Formel II etwa bei oder unterhalb oder oberhalb der Raumtemperatur; im allgemeinen liegt die Reaktionstemperatur zweckmässigerweise in einem Bereich von etwa -50 bis etwa +50°C. Die Reaktionszeit variiert in der Regel zwischen etwa einer halben Stunde und einigen wenigen Stunden.

Aspekt b) des erfindungsgemässen Verfahrens liefert Verbindungen der Formel I, worin $R^1$ die Gruppe (e) bedeutet, wobei aber $R^6$ nur Wasserstoff oder Halogen bedeuten kann. Die Dehydrohalogenierung erfolgt mittels einer Base, beispielsweise mit einer organischen Base, welche möglichst wenig nukleophil ist, zweckmässigerweise also mit Kalium-tert. -Butylat oder dergleichen oder mit einer bicyclischen Verbindung, wie 1,8-Diazabicyclo[5.4.0]undec-7-en, 1,5-Diazabicyclo[4.3.0]non-5-en oder dergleichen, oder auch mit einer anorganischen Base, wie Natriumhydrid, Natriumhydroxid oder dergleichen. Weiterhin erfolgt die Dehydrohalogenierung zweckmässigerweise in einem unter den Reaktionsbedingungen inerten organischen Lösungsmittel, wie N,N-Dimethylformamid, Dimethylsulfoxid, Tetrahydrofuran, tert.-Butanol oder dergleichen, bei erhöhter Temperatur, zweckmässigerweise bei Siedetemperatur des Reaktionssystems; sie kann auch mittels Natriumamid in flüssigem Ammoniak oder mittels einer Lösung von Natrium in einem niederen Alkohol, wie Methanol, erfolgen, und sie dauert mehrere Stunden, beispielsweise etwa 3 bis etwa 8 Stunden.

Gemäss Aspekt c) des erfindungsgemässen Verfahrens wird eine Verbindung der Formel I, worin $R^1$ die Gruppe (e) und $R^6$ Wasserstoff bedeuten, zunächst mittels einer starken Base, wie Natriumhydrid, Kalium-tert.-Butylat, Butyllithium oder dergleichen deprotoniert, was zweckmässigerweise in einem unter den Reaktionsbedingungen inerten organischen Lösungsmittel, wie N,N-Dimethylformamid, Toluol, Tetrahydrofuran oder dergleichen erfolgt; zur Deprotonierung kann aber auch Natriumamid in flüssigem Ammoniak oder Natronlauge in einem niederen Alkohol, wie Methanol, verwendet werden. Anschliessend gibt man dann das den gewünschten Rest liefernde Mittel zu, dessen Natur natürlich von dem gewünschten einzuführenden Rest abhängt. Für die Einführung einer niederen Alkylgruppe verwendet man bei- spielsweise ein niederes Alkylhalogenid, wie Methyljodid, ein niederes Dialkylsulfat oder einen niederen Alkylester einer Sulfonsäure, wie Methansulfonsäure, Benzolsulfonsäure, p-Toluolsulfonsäure oder p-Brombenzolsulfonsäure, usw. In analoger Weise kann man einen niederes-Alkoxy-niederen Alkylrest, einen ($C_4$-$C_7$)-Cycloalkylrest oder einen ($C_3$-$C_7$)-Cycloalkyl-niederen Alkylrest mittels eines entsprechenden Halogenids einführen, beispielsweise also mittels Chlordimethyläther, Cyclohexylbromid, Cyclopropylmethylbromid und dergleichen. Zwecks Einführung eines Hydroxyl-haltigen Restes, in welchem sich die Hydroxygruppe in α-Stellung befindet, kann man eine entsprechende Carbonylverbindung einsetzen; so wird beispielsweise eine Hydroxymethylgruppe mittels Formaldehyd eingeführt, eine 2-Hydroxy-2-propylgruppe mittels Aceton usw. Eine β-Hydroxyalkylgruppe kann zweckmässigerweise mittels eines entsprechenden Epoxids eingeführt werden, eine 2-Hydroxyäthylgruppe also mittels Aethylenoxid. Ein Halogenatom kann mittels elementarem Halogen eingeführt weden. Die Reaktionsbedingungen hängen selbstverständlich von der Natur der zur Einführung des jeweils gewünschten Restes verwendeten Reagens ab. Ist dieses Reagens beispielsweise Methyljodid, so erfolgt die Reaktion zeckmässigerweise in einem unter den Reaktionsbedingungen inerten organischen Lösungsmittel, wie N,N-Dimethylformamid, Tetrahydrofuran, Toluol, Dioxan, Dimethylsulfoxid oder dergleichen, bei Raumtemperatur, und sie dauert einige, beispielsweise 2-5, Stunden.

Die Umsetzung von Verbindungen der Formeln VI und VII gemäss Aspekt d) des erfindungsgemässen Verfahrens erfolgt in Gegenwart eines Palladium(II)-salzes, wie Palladiumchlorid oder Palladiumacetat, eines Organophosphins, wie Triphenylphosphin, von Kupfer(I)-jodid und eines sekundären oder tertiären Amins, wie Diäthylamin oder Triäthylamin; anstatt eines Palladium(II)-salzes und eines Organophosphins kann auch ein geeigneter entsprechender Komplex verwendet werden, wie z.B. Bis-(triphenylphosphin)-palladium(II)-dichlorid. Als Lösungsmittel kann das erwähnte sekundäre oder tertiäre Amin selbst, ein halogenierter Kohlenwasserstoff, wie Methylenchlorid oder dergleichen, N,N-Dimethylformamid oder dergleichen verwendet werden. Als Verbindung der Formel VII verwendet man beispielsweise Propin, 3,3-Dimethyl-1-butin, Phenylacetylen, Propargylalkohol, 2-Methyl-3-butin-2-ol usw. Je nach der Natur der verwendeten Verbindung der Formel VII erfolgt die Reaktion unter Druck und bei Temperaturen in einem Bereich zwischen etwa Raumtemperatur und etwa 120°C; die Reaktionszeit beträgt, je nach den übrigen Reaktionsparametern, etwa 1 bis etwa 70 Stunden. Die Ausgangsprodukte der Formel VI sind bekannt oder nach an sich bekannten und jedem Fachmann geläufigen Methoden leicht herstellbar; zudem enthalten einige der nachfolgenden Beispiele ausführliche Angaben über die Herstellung bestimmter Verbindungen der Formel

VI.

Aspekt e) des erfindungsgemässen Verfahrens liefert Verbindungen der Formel I, worin $R^1$ einen Rest der Formel (e) und $R^6$ Wasserstoff bedeuten. Als Schutzgruppe, welche in Formel VIII mit Z bezeichnet werden, kommen natürlich nur solche Reste in Betracht, welche selektiv entfernt werden können, ohne dass dabei andere im Molekül vorhandene Gruppen in Mitleidenschaft gezogen werden. Reste, welche diesen Anforderungen genügen, und Methoden zu deren selektiven Entfernung sind dem Fachmann geläufig. Geeignet sind beispielsweise Trialkylsilylgruppen, wie Trimethylsilyl, α-Hydroxyalkylgruppen, wie 2-Hydroxy-2-propyl usw. Die Abspaltung von Trialkylsilylgruppen kann beispielsweise mittels Kaliumfluorid in Wasser, mittels eines Alkalimetallhydroxids, wie Kaliumhydroxid, in einem niederen Alkanol, wie Aethanol, und/oder Wasser oder dergleichen erfolgen, und die Abspaltung von Gruppen wie 2-Hydroxy-2-propyl zweckmässigerweise unter alkalischen Be- dingungen, beispielsweise mittels eines Alkalimetallhydroxids, wie Natriumhydroxid, eines Alkalimetallhydrids, wie Natriumhydrid, oder dergleichen in einem unter den Reaktionsbedingungen inerten organischen Lösungsmittel, beispielsweise in einem aromatischen Kohlenwasserstoff, wie Toluol, Benzol, Xylol oder dergleichen. Diejenigen unter den Ausgangsprodukten der Formel VIII, welche nicht unter den Umfang von Formel I fallen, sind ebenfalls neu und gleichfalls Gegenstand der vorliegenden Erfindung. Die Herstellung solcher Verbindungen kann in analoger Weise wie die Herstellung entsprechender Verbindungen der Formel I erfolgen, beispielsweise in Analogie zu Aspekt d) des erfindungsgemässen Verfahrens.

Der Ersatz einer Aminogruppe durch ein Halogenatom gemäss Aspekt f) des erfindungsgemässen Verfahrens kann dadurch erfolgen, dass man die Aminoverbindung der Formel IX in ein entsprechendes Diazoniumsalz überführt und dieses, gegebenenfalls ohne vorgängige Isolierung, mit einem Halogenid, z.B. mit einem Chlorid oder Bromid, in Gegenwart eines Kupfer(I)-salzes umsetzt; die Herstellung entsprechender Jodverbindungen erfolgt in analoger Weise, jedoch ist die Anwesenheit eines Kupfer(I)-salzes nicht erforderlich. Entsprechende Fluorverbindungen werden zweckmässigerweise über die entsprechenden Diazonium-tetrafluoroborate hergestellt, beispielsweise durch Bestrahlen mit UV-Licht. Die vorstehend erwähnten Reaktionen werden in wässrigen Lösungen bei Temperaturen von etwa -10 °C bis etwa Raumtemperatur durchgeführt.

Der Ersatz der Aminogruppe durch ein Wasserstoffatom gemäss Aspekt f) des erfindungsgemässen Verfahrens kann durch Reduktion eines entsprechenden Diazoniumsalzes bewerkstelligt werden, beispielsweise durch Erhitzen in einem cyclischen Aether, wie Tetrahydrofuran oder Dioxan, oder in Aethanol, N,N-Dimethylformamid oder dergleichen; dabei arbeitet man vorzugsweise bei Siedetemperatur der Reaktionsmischung. Man kann aber auch ein Amin der Formel IX in einem cyclischen Aether, wie Tetrahydrofuran oder Dioxan, mit t-Butylnitrit, Isopentylnitrit und dergleichen umsetzen, wobei man vorzugsweise bei etwa Siedetemperatur des Reaktionsgemisches arbeitet.

Die Ausgangsprodukte der Formel IX sind neu und ebenfalls Gegenstand der vorliegenden Erfindung. Ihre Herstellung erfolgt durch Reduktion entsprechender Nitroverbindungen, und diese können ihrerseits in Analogie zur Herstellung entsprechender Verbindungen der Formel I erhalten werden.

Gemäss Aspekt g) des erfindungsgemässen Verfahrens wird eine Kohlenstoff-Kohlenstoff-Dreifachbindung partiell zu einer Kohlenstoff-Kohlenstoff-Doppelbindung reduziert. Eine derartige partielle Reduktion kann nach an sich üblichen und jedem Fachmann geläufigen Methoden bewerkstelligt werden, zweckmässigerweise durch Hydrierung in Gegenwart eines teilweise inaktivierten Katalysators, beispielsweise in Gegenwart eines mittels Chinolin und/oder Blei vorbehandelten Palladiumkatalysators. Die partielle Hydrierung erfolgt zweckmässigerweise bei Raumtemperatur und Atmosphärendruck in einem unter den Reaktionsbedingungen inerten organischen Lösungsmittel, beispielsweise in Essigester, Methanol, N,N-Dimethylformamid, Dichlormethan usw.

Gemäss Aspekt h) des erfindungsgemässen Verfahrens wird eine Hydroxygruppe in eine niedere Alkoxygruppe übergeführt. Es handelt sich also hierbei um eine Verätherung einer Hydroxygruppe, und Methoden zur Durchführung einer derartigen Verätherung sind an sich bekannt und jedem Fachmann geläufig. Die erfindungsgemässe Verätherung erfolgt zweckmässigerweise mittels eines niederen Alkylhalogenids, wie Methyljodid, eines niederen Dialkylsulfats oder eines niederen Alkylesters einer organischen Sulfonsäure, wie Methansulfonsäure, Benzolsulfonsäure, p-Toluolsulfonsäure, p-Brombenzolsulfonsäure usw. Man arbeitet zweckmässigerweise in Gegenwart einer Base, beispielsweise eines Alkalimetallhydroxids, wie Kaliumhydroxid, und in Gegenwart eines unter den Reaktionsbedingungen inerten organischen Lösungsmittels, beispielsweise in N,N-Dimethylformamid, Dimethylsulfoxid, Toulol und dergleichen.

Gemäss Aspekt i) des erfindungsgemässen Verfahrens wird eine Carbonylgruppe zur entsprechenden Alkoholgruppe reduziert. Diese Reduktion kann nach an sich bekannten und jedem Fachmann geläufigen Methoden durchgeführt werden. Als Reduktionsmittel kommt beispielsweise ein Alkalimetallborhydrid, wie Natriumborhydrid, in Frage. Geeignet Lösungsmittel sind z.B. niedere Alkohole, wie Methanol und Aethanol,

und Dimethylformamid und Mischungen davon. Die Reduktion wird zweckmässigerweise bei Raumtemperatur durchgeführt.

Wie eingangs erwähnt sind die Verbindungen der Formel I neu. Sie besitzen wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben entweder ausgeprägte anxiolytische, antikonvulsive, muskelrelaxierende und sedativ-hypnotische Eigenschaften, und/oder sie antagonisieren selektiv einige oder alle Wirkungen, welche tranquilisierend wirksame 1,4-Benzodiazepine oder andere Substanzen über die zentralen Benzodiazepin-Rezeptoren entfalten, partiell oder vollständig.

Die Affinität von Verbindungen der allgemeinen Formel I zu den zentralen Benzodiazepin-Rezeptoren wurde nach der in Life Science 20, 2101-2110 (1977) und Science 198, 849-851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung des tritiierten Diazepams an die spezifischen Benzodiazepin-Rezeptoren im cerebralen Cortex bewirkt.

Die zentralen Eigenschaften der erfindungsgemässen Verbindungen der Formel I können beispielsweise im nachfolgend beschriebenen und für die Erfassung antikonvulsiver Eigenschaften allgemein anerkannten Antipentetrazoltest nachgewiesen werden.

In diesem Tierversuch verabreicht man 60-80 g schweren, weiblichen Ratten die zu testende Verbindung oral und 30 Minuten später 120 mg/kg i.p. Pentetrazol, das in ungeschützten Versuchstieren 1 bis 4 Minuten nach der Injektion Emprosthotonus und tonische Streckungen der vorderen und/oder hinteren Gliedmassen bewirkt. Man verwendet zehn Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt.

Eine der typischen Eigenschaften von tranquilisierend wirksamen 1,4-Benzodiazepinen in Versuchstieren ist ihre ausgeprägte antikonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antagonisieren vermögen.

In diesem Test verabreicht man Mäusen eine Stunde vor dem Pentetrazol (120 mg/kg, i.p.) 5 mg/kg (i.p.) Diazepam (d.h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p.o. Die antagonistische Wirksamkeit der untersuchten Verbindungen, d.h. ihr Vermögen, die Wirkung des Diazepams im Pentetrazol-Test aufzuheben, wird ermittelt, indem man die Mäuse auszählt, die in diesem Test krampfartige Anfälle erleiden. Als $ED_{50}$ bezeichnet man die Menge der jeweiligen Testverbindung in mg/kg (p.o.), die bei 50% der Tiere den Diazepam-Effekt in obigem Versuch aufhebt.

In der nachfolgenden Tabelle werden die Resultate dargestellt, welche mit repräsentativen Vertretern der durch die allgemeine Formel I definierten Verbindungsklasse in den vorstehend geschilderten Versuchen erhalten worden sind. Ausserdem enthält die Tabelle Angaben über die akute Toxzität einiger dieser Verbindungen ($DL_{50}$ in mg/kg bei einmaliger oraler Verabreichung an Ratten).

| Verbindung | Affinität zu Benzodiazapin-Rezeptoren IC 50, nMol/l | Antipentetrazoltest ED 50 mg/kg p.o. | Antagonismus von Diazepam ED 50 mg/kg p.o. | Toxizität DL 50 mg/kg p.o. |
|---|---|---|---|---|
| A | 4,5 | | 0,24 | 312-625 |
| B | 24 | | 1,5 | 312-625 |
| C | 2,3 | | 0,36 | >5000 |
| D | 2,4 | 1,5 | >50 | >4000 |
| E | 2,0 | 0,29 | | 500-1000 |
| F | 7,6 | | 1,2 | 1250-2500 |

A = 7-Chlor-3-äthinyl-4,5-dihydro-5-methyl-6H-imidazo[1,5-a][1,4]benzodiazepin-6-on
B = 3-Aethinyl-8-fluor-4,5-dihydro-5-methyl-6H-imidazo[1,5-a][1,4]benzodiazepin-6-on
C = 7-Chlor-4,5-dihydro-5-methyl-3-(1-propinyl)-6H-imidazo[1,5-a][1,4]benzodiazepin-6-on
D = (S)-8-Chlor-1-äthinyl-11,12,13,13a-tetrahydro-9H-imidazo[1,5-a]pyrrolo[2,1-c][1,4]benzodiazepin-9-on
E = 7-Brom-4,5-dihydro-3-(3-hydroxy-3-methyl-1-butinyl)-5-methyl-6H-imidazo[1,5-a][1,4]benzodiazepin-6-on
F = 7-Chlor-4,5-dihydro-5-methyl-3-vinyl-6H-imidazo[1,5-a][1,4]benzodiazepin-6-on

Eine selektive antagonistische Komponente, wie sie bei vielen der Verbindungen der Formel I nachgewiesen werden kann, ist von grosser therapeutischer Bedeutung, indem sie die Nutzung erwünschter Eigenschaften (z.B. anxiolytische oder antikonvulsive Wirkung) der erfindungsgemässen Stoffe erlaubt, unter Zurückdrängung der im bestimmten Anwendungsfall unerwünschten, zusätzlichen Eigenschaften (z.B. sedierende, muskelrelaxierende und die motorische Koordination störende Wirkungen).

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I und ein therapeutisch inertes Excipiens, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I und gegebenenfalls einen oder mehrere andere therapeutisch wertvolle Stoffe zusammen mit einem oder mehreren therapeutisch inerten Trägern in eine galenische Darreichungsform bringt.

Wie eingangs erwähnt, können die Verbindungen der Formel I erfindungsgemäss bei der Bekämpfung bzw. Verhütung von Krankheiten verwendet werden und zwar insbesondere bei der Bekämpfung von Konvulsionen und Angstzuständen und/oder bei der partiellen oder vollständigen Antagonisierung einiger oder aller Wirkungen, welche tranquilisierend wirksame 1,4-Benzodiazepine oder andere Substanzen über die zentralen Benzodiazepin-Rezeptoren entfalten. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,1 mg bis 100 mg angemessen sein.

Ebenfalls Gegenstand der Erfindung ist schliesslich, wie eingangs erwähnt, die Verwendung von Verbindungen der Formel I für die Herstellung von Arzneimitteln, insbesondere von Arzneimitteln zur Anwendung bei der Bekämpfung bzw. Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

a) 14,14 g (49,5 mMol) Aethyl 5,6-dihydro-5-methyl-6-oxo-4H-imidazo[1,5-a][1,4]benzodiazepin -3-carboxylat werden in 100 ml Tetrahydrofuran bei Siedetemperatur portionenweise mit 1,35 g (62 mMol) Lithiumborhydrid versetzt, worauf während 6 Stunden am Rückfluss gekocht wird. Man kühlt dann das Reaktionsgemisch ab, tropft unter Rühren vorsichtig eine Mischung von 20 ml Wasser und 20 ml konzentrierter Salzsäure zu, erhitzt, rührt 30 Minuten bei Siedetemperatur, kühlt wieder ab und versetzt bis zur alkalischen Reaktion mit konzentriertem Ammoniak. Das organische Lösungsmittel wird am Rotationsverdampfer abdestilliert, und die erhaltene wässrige Suspension wird abgekühlt und filtriert. Der Filterrückstand wird mit Wasser gewaschen und getrocknet; man erhält 4,5-Dihydro-3-hydroxymethyl-5-methyl-6H -imidazo[1,5-a][1,4]benzodiazepin-6-on vom Schmelzpunkt 219-221°.

b) 6,73 g (27,6 mMol) 4,5-Dihydro-3-hydroxymethyl-5-methyl-6H -imidazo[1,5-a][1,4]benzodiazepin-6-on werden zusammen mit 33 g (380 mMol) Mangandioxid in 100 ml Methylenchlorid während 4 Stunden bei Raumtemperatur gerührt. Man filtriert, wäscht den Filterrückstand gründlich mit ca. 1,5 l Methylenchlorid nach und dampft das Filtrat ein. Man erhält 5,6-Dihydro-5-methyl-6-oxo-4H-imidazo[1,5-a][1,4]-benzodiazepin -3-carboxaldehyd vom Schmelzpunkt 202-203°.

c) 13,50 g einer äquimolaren Mischung von Chlormethyltriphenylphosphoniumchlorid und Natriumamid werden während 15 Minuten mit 50 ml Tetrahydrofuran gerührt, wobei die Temperatur auf 42° steigt. Man gibt dann portionenweise 6,2 g (25,7 mMol) 5,6-Dihydro-5-methyl-6-oxo-4H-imidazo[1,5-a][1,4]-benzodiazepin -3-carboxaldehyd zu, rührt eine weitere Stunde bei Raumtemperatur, filtriert und dampft das Filtrat ein. Nach Chromatographieren des Rückstandes an Kieselgel unter Eluieren mit Cyclohexan/Aether/Isopropanol (3:3:1) erhält man 3-[(Z)-2-Chlorvinyl]-4,5-dihydro-5-methyl-6H -imidazo-[1,5-a][1,4]benzodiazepin-6-on vom Schmelzpunkt 197-199°.

Beispiel 2

2,20 (8 mMol) 3-[(Z)-2-Chlorvinyl]-4,5-dihydro-5-methyl-6H-imidazo[1,5-a][1,4]benzodiazepin-6-on werden zusammen mit 1,43 ml (9,6 mMol) 1,8-Diazabicyclo[5.4.0]undec-7-en in 30 ml N,N-Dimethylformamid während 6 Stunden bei 145° gerührt. Man giesst anschliessend das Reaktionsgemisch auf Wasser und extrahiert fünfmal mit Methylenchlorid. Die organischen Auszüge werden fünfmal mit Wasser gewaschen, über Magnesiumsulfat getrocknet und eingedampft. Nach Chromatographieren des Rückstandes an Kieselgel unter Eluieren mit Essigester und anschliessendem Umkristallisieren aus Essigester erhält man 3-Aethinyl-4,5-dihydro-5-methyl-6H-imidazo[1,5-a][1,4]benzodiazepin-6-on vom Schmelzpunkt 191-193°.

Beispiel 3

100 g einer äquimolaren Mischung von Chlormethyltriphenylphosphoniumchlorid und Natriumamid werden in 450 ml Tetrahydrofuran während 45 Minuten verrührt, wobei die Temperatur auf 37° steigt. Bei Raumtemperatur gibt man dann portionenweise 62,23 g (229,7 mMol) 7-Chlor-5,6-dihydro-5-methyl-6-oxo-4H -imidazo[1,5-a][1,4]benzodiazepin-3-carboxaldehyd zu und rührt nach Beendigung der Zugabe noch 1 Stunde weiter. Man filtriert anschliessend das Reaktionsgemisch und dampft das Filtrat ein. Nach Chromatographieren des Rückstandes an Kieselgel unter Eluieren mit Cyclohexan/Aether/Isopropanol (3:3:1) erhält

man 7-Chlor-3-[(Z)-2-chlorvinyl]-4,5-dihydro-5-methyl-6H -imidazo[1,5-a][1,4]benzodiazepin-6-on vom Schmelzpunkt 205-207°.

Beispiel 4

20,2 g (65,5 mMol) 7-Chlor-3-[(Z)-2-chlorvinyl]-4,5-dihydro-5-methyl-6H -imidazo[1,5-a][1,4]-benzodiazepin-6-on werden zusammen mit 11,7 ml (78,5 mMol) 1,8-Diazabicyclo[5.4.0]undec-7-en in 200 ml N,N-Dimethylformamid während 5 Stunden unter Rückfluss zum Sieden erhitzt. Man giesst anschliessend das Reaktionsgemisch auf 800 ml Wasser und extrahiert viermal mit Methylenchlorid. Die organischen Auszüge werden viermal mit Wasser gewaschen, über Magnesiumsulfat getrocknet und eingedampft. Nach Chromatographieren des Rückstandes an Kieselgel unter Eluieren mit Essigester und zwei aufeinanderfolgenden Kristallisationen aus Acetonitril und aus Essigester erhält man 7-Chlor-3-äthinyl-4,5-dihydro-5-methyl-6H -imidazo[1,5-a][1,4]benzodiazepin-6-on vom Schmelzpunkt 201-202°.

Beispiel 5

2,72 g (10 mMol) 7-Chlor-3-äthinyl-4,5-dihydro-5-methyl-6H -imidazo[1,5-a][1,4]benzodiazepin-6-on werden in 20 ml N,N-Dimethylformamid gelöst. 1,31 g (30 mMol) Natriumhydrid-Dispersion (55%ig in Oel) werden mit n-Hexan gewaschen und dann bei Raumtemperatur in die obige Lösung eingetragen. Nach 10 Minuten gibt man 0,95 ml (15 mMol) Methyljodid zu und rührt während 3 Stunden bei Raumtemperatur weiter. Man giesst das Reaktionsgemisch auf 300 ml Wasser und extrahiert viermal mit Methylenchlorid. Die organischen Auszüge werden viermal mit Wasser gewaschen und über Magnesiumsulfat getrocknet. Nach Chromatographieren des Rückstandes an Kieselgel unter Eluieren mit Essigester und Umkristallisieren aus Essigester erhält man 7-Chlor-4,5-dihydro-5-methyl-3-(1-propinyl)-6H -imidazo[1,5-a][1,4]benzodiazepin-6-on vom Schmelzpunkt 243-244°.

Beispiel 6

6,25 g einer äquimolaren Mischung von Methyltriphenylphosphoniumbromid und Natriumamid werden während 15 Minuten in 40 ml Tetrahydrofuran gerührt. Zur gelben Suspension gibt man dann 4,13 g (15 mMol) 7-Chlor-5,6-dihydro-5-methyl-6-oxo-4H -imidazo[1,5-a][1,4]benzodiazepin-3-carboxaldehyd, wobei die Temperatur auf 43° steigt und sich die Suspension entfärbt. Man rührt während 1 Stunde weiter, filtriert und dampft das Filtrat ein. Nach Chromatographieren des Rückstandes an Kieselgel unter Eluieren mit Cyclohexan/Aether/Isopropanol (3:3:1) und Umkristallisieren aus Essigester erhält man 7-Chlor-4,5-dihydro-5-methyl-3-vinyl-6H -imidazo[1,5-a][1,4]benzodiazepin-6-on vom Schmelzpunkt 205-207°.

Beispiel 7

15 g (40,4 mMol) Aethyltriphenylphosphoniumbromid werden in 60 ml Tetrahydrofuran vorgelegt und bei -40° tropfenweise mit 28 ml (45 mMol) 1,6-molarer Butyllithiumlösung in n-Hexan versetzt. Man rührt die erhaltene orange Suspension während 20 Minuten bei -40° weiter und tropft anschliessend innerhalb von 50 Minuten bei -40° bis -50° eine Lösung von 10 g (36 mMol) 7-Chlor-5,6-dihydro-5-methyl-6-oxo-4H -imidazo[1,5-a][1,4]benzodiazepin-3-carboxaldehyd in 250 ml Tetrahydrofuran zu. Man rührt während 2 Stunden bei Raumtemperatur weiter und filtriert anschliessend das Reaktionsgemisch. Das Filtrat wird eingedampft und an Kieselgel unter Eluieren mit Cyclohexan/Aether/Isopropanol (3:3:1) chromatographiert. Man erhält 7-Chlor-4,5-dihydro-5-methyl-3-[(Z)-propenyl]-6H -imidazo[1,5-a][1,4]benzodiazepin-6-on vom Schmelzpunkt 169,5-170,5° (aus Essigester/Hexan) und 7-Chlor-4,5-dihydro-5-methyl-3-[(E)-propenyl]-6H -imidazo[1,5-a][1,4]benzodiazepin-6-on vom Schmelzpunkt 200-201° (aus Acetonitril).

Beispiel 8

25 g einer äquimolaren Mischung von Chlormethyltriphenylphosphoniumchlorid und Natriumamid werden in 120 ml Tetrahydrofuran während 20 Minuten bei Raumtemperatur gerührt. Man gibt dann 12,9 g (50 mMol) 8-Fluor-5,6-dihydro-5-methyl-6-oxo-4H -imidazo[1,5-a][1,4]benzodiazepin-3-carboxaldehyd zu und rührt während 1 Stunde bei Raumtemperatur und 10 Minuten bei Seidetemperatur. Man kühlt ab, filtriert und dampft das Filtrat ein. Nach Chromtographieren des Rückstandes an Kieselgel unter Eluieren mit Cyclohexan/Aether/Isopropanol (3:3:1) und Umkristallisieren aus Acetonitril erhält man 3-[(Z)-2-Chlorvinyl]-8-fluor-4,5-dihydro-5-methyl-6H -imidazo[1,5-a][1,4]benzodiazepin-6-on vom Schmelzpunkt 220-221°.

Beispiel 9

3,70 g (12,7 mMol) 3-[(Z)-2-Chlorvinyl]-8-fluor-4,5-dihydro-5-methyl-6H -imidazo[1,5-a][1,4]-benzodiazepin-6-on werden zusammen mit 2,26 ml (15,2 mMol) 1,8-Diazabicyclo[5.4.0]undec-7-en in 30 ml N,N-Dimethylformamid während 4 Stunden unter Rückfluss zum Sieden erhitzt. Man giesst anschliessend das Reaktionsgemisch auf 400 ml Wasser und extrahiert fünfmal mit Methylenchlorid. Die organischen Auszüge werden viermal mit Wasser gewaschen, über Magnesiumsulfat getrocknet und eingedampft. Der Rückstand wird in Essigester gelöst, mit Kohle behandelt und aus Essigester umkristallisiert. Man erhält 3-Aethinyl-8-fluor-4,5-dihydro-5-methyl-6H -imidazo[1,5-a][1,4]benzodiazepin-6-on vom Schmelzpunkt 207-208°.

Beispiel 10

40,70 g einer äquimolaren Mischung von Butyltriphenylphosphoniumbromid und Natriumamid werden in 150 ml Tetrahydrofuran während 15 Minuten bei Raumtemperatur gerührt. Man gibt dann 20,73 g (80 mMol) 8-Fluor-5,6-dihydro-5-methyl-6-oxo-4H-imidazo[1,5-a][1,4]benzodiazepin-3-carboxaldehyd zu, rührt während 1,5 Stunden bei Raumtemperatur weiter, filtriert und dampft das Filtrat ein. Nach Chromatographieren des Rückstandes an Kieselgel unter Eluieren mit Cyclohexan/Aether/Isopropanol (3:3:1) und Umkristallisieren aus Essigester und n-Hexan erhält man 8-Fluor-4,5-dihydro-5-methyl-3-(1-pentenyl)-6H -imidazo[1,5-a][1,4]benzodiazepin-6-on vom Schmelzpunkt 150-151°.

Beispiel 11

17,5 g einer äquimolaren Mischung von Chlormethyltriphenylphosphoniumchlorid und Natriumamid werden in 100 ml Tetrahydrofuran während 15 Minuten gerührt, wobei die Temperatur auf 33° steigt. Man gibt dann portionenweise 11,1 g (38,58 mMol) (S)-8-Chlor-12,12a-dihydro-9-oxo-9H,11H-azeto[2,1-c]-imidazo[1,5-a][1,4]benzodiazepin-1-carboxaldehyd zu, wobei die Temperatur unter Ammoniak-Entwicklung auf 45° ansteigt. Man rührt während einer Stunde bei Raumtemperatur weiter, filtriert und dampft das Filtrat ein. Nach Chromatographieren an Kieselgel unter Eluieren mit Cyclohexan/Aether/Isopropanol (3:3:1) und anschliessendem Umkristalliesieren aus Essigester erhält man (S)-8-Chlor--1-[(Z)-2-chlorvinyl]-12,12a-dihydro -9H,11H-azeto[2,1-c]imidazo[1,5-a][1,4]benzadiazepin -9-on vom Schmelzpunkt 189-191°.

Beispiel 12

2.21 g (6.9 mMol) (S)-8-Chlor-1-[(Z) -2-chlorvinyl]-12,12a-dihydro-9H,11H-azeto[2,1-c] imidazo[1,5-a]-[1,4]benzodiazepin-9-on werden zusammen mit 1,23 ml (8,3 mMol) 1,8-Diazabicyclo[5.4.0]undec-7-en in 30 ml N,N-Dimethylformamid während 4 Stunden unter Rückfluss zum Sieden erhitzt. Man giesst anschliessend das Reaktionsgemisch auf 300 ml Wasser und extrahiert fünfmal mit Methylenchlorid. Die organischen Auszüge werden viermal mit Wasser gewaschen, über Magnesiumsulfat getrocknet und eingedampft. Nach Chromatographieren an Kieselgel unter Eluieren mit Essigester und anschliessendem Umkristallisieren aus Essigester erhält man (S)-8-Chlor-1-äthinyl-12,12a-dihydro -9H,11H-azeto[2,1-c]imidazo[1,5-a][1,4]-benzodiazepin-9-on vom Schmelzpunkt 249-250°.

Beispiel 13

8,70 g einer äquimolaren Mischung von Methyltriphenylphosphoniumbromid und Natriumamid werden während 20 Minuten in 60 ml Tetrahydrofuran gerüht. Man gibt dann portionenweise 6 g (20 mMol) (S)-8-Chlor-12,12a-dihydro -9-oxo-9H,11H-azeto[2,1-c]imidazo[1,5-a][1,4] benzodiazepin-1-carboxaldehyd zu und rührt während 1 Stunde weiter. Man filtriert anschliessend und dampft das Filtrat ein. Nach Chromatographieren des Rückstandes an Kieselgel unter Eluieren mit Cyclohexan/Aether/Isopropanol (3:3:1) und anschliessendem Umkristallisieren aus Essigester erhält man (S)-8-Chlor-12,12a-dihydro -1-vinyl-9H,11H-azeto[2,1-c]imidazo[1,5-a][1,4]benzodiazepin -9-on vom Schmelzpunkt 171-172°.

Beispiel 14

15 g einer äquimolaren Mischung von Chlormethyltriphenylphosphoniumchlorid und Natriumamid werden in 60 ml Tetrahydrofuran während 20 Minuten gerührt. Man gibt dann 10 g (33 mMol) (S)-8-Chlor-11,12,13,13a-tetrahydro -9-oxo-9H-imidazo[1,5-a]pyrrolo[2,1-c][1,4]benzodiazepin -1-carboxaldehyd zu, wo-bei die Temperatur unter heftiger Ammoniak--Entwicklung rasch auf 54° steigt. Man rührt während 45 Minuten weiter, filtriert und dampft ein. Der Rückstand wird an Kieselgel unter Eluieren mit Cyclohexan/Aether/Isopropanol (3:3:1) chromatographiert. Nach Umkristallisieren aus Essigester erhält man (S)-8-Chlor-1-[(Z)-2-chlorvinyl]-11,12,13,13a-tetrahydro -9H-imidazo[1,5-a]pyrrolo[2,1-c][1,4]benzodiazepin-9-on vom Schmelzpunkt 209-210°.

Beispiel 15

2,5 g (7,5 mMol) (S)-8-Chlor-1-[(Z)-2-chlorvinyl]-11,12,13,13a-tetrahydro -9H-imidazo[1,5-a]pyrrolo[2,1-c][1,4]benzodiazepin -9-on werden zusammen mit 1,66 ml (11,2 mMol) 1,8-Diazabicyclo[5.4.0]undec-7-en in 30 ml N,N-Dimethylformamid während 5 Stunden unter Rückfluss zum Sieden erhitzt. Man giesst anschliessend das Reaktionsgemisch auf 400 ml Wasser und filtriert die erhaltenen Kristalle ab. Nach Trocknen und Umkristallisieren aus N,N-Dimethylformamid erhält man (S)-8-Chlor-1-äthinyl-11,12,13,13a-tetrahydro -9H-imidazo[1,5-a]pyrrolo[2,1-c][1,4]benzodiazepin-9-on vom Schmelzpunkt 324-325°.

Beispiel 16

250 mg (0,85 mMol) (S)-8-Chlor-1-äthinyl-11,12,13,13a-tetrahydro -9H-imidazo[1,5-a]pyrrolo[2,1-c][1,4]-benzodiazepin-9-on werden in 5 ml N,N-Dimethylformamid suspendiert. 50 mg (1 mMol) Natriumhydrid-Dispersion (55%ig in Oel) werden mit n-Hexan gewaschen und dann in die obige Suspension eingetragen. Nach 10 Minuten gibt man 0,1 ml (1,5 mMol) Methyljodid zu und rührt während 4,5 Stunden bei Raumtemperatur weiter. Man giesst auf 50 ml Wasser und extrahiert viermal mit Methylenchlorid. Die organischen Auszüge werden viermal mit Wasser gewaschen, über Magnesiumsulfat getrocknet und eingedampft. Nach Chromatographieren des Rückstandes an Kieselgel unter Eluieren mit Essigester und Umkristallisieren aus Essigester und Hexan erhält man (S)-8-Chlor-11,12,13,13a-tetrahydro-1-propinyl -9H-imidazo[1,5-a]pyrrolo[2,1-c][1,4]benzodiazepin-9-on vom Schmelzpunkt 205-20°.

Beispiel 17

20, 8 g einer äquimolaren Mischung von Methyltriphenylphosphoniumbromid und Natriumamid werden während 20 Minuten in 80 ml Tetrahydrofuran gerührt. Man gibt dann portionenweise 15,08 g (50 mMol) (S)-8-Chlor-11,12,13,13a-tetrahydro -9-oxo-9H-imidazo[1,5-a]pyrrolo[2,1-c][1,4]benzodiazepin -1-carboxaldehyd zu, wobei die Temperatur unter Ammoniak-Entwicklung auf 43° steigt. Man rührt während 1 Stunde bei Raumtemperatur, filtriert und dampft das Filtrat ein. Nach Chromatographieren des Rückstandes an Kieselgel unter Eluieren mit Cyclohexan/Aether/Isopropanol (3:3:1) und Umkristallisieren aus Essigester erhält man (S)-8-Chlor-11,12,13,13a-tetrahydro -1-vinyl-9H-imidazo[1,5-a]pyrrolo[2,1-c][1,4]benzodiazepin -9-on vom Schmelzpunkt 213-215°.

Beispiel 18

a) 2,31 g 8-Fluor-4,5-dihydro-5-methyl-6H -imidazo[1,5-a][1,4]benzodiazepin-6-on werden mit 8,88 g (35 mMol) Jod in 25 ml N,N-Dimethylformamid während 1,5 Stunden bei 95° gerührt. Man giesst dann das Reaktionsgemisch auf 300 ml Wasser, entfärbt es mit einer Natriumthiosulfat-Lösung und extrahiert es viermal mit Methylenchlorid. Die organischen Auszüge werden dreimal mit Wasser gewaschen, über Magnesiumsulfat getrocknet und eingedampft. Nach Umkristallisieren des Rückstandes aus Essigester erhält man 8-Fluor-4,5-dihydro-3-jod-5-methyl-6H -imidazo[1,5-a][1,4]benzodiazepin-6-on vom Schmelzpunkt 187-188°.

b) 2,08 g (5,9 mMol) 8-Fluor-4,5-dihydro-3-jod-5-methyl-6H -imidazo[1,5-a][1,4]benzodiazepin-6-on werden in einem verschliessbaren Kolben mit 50 mg Bis-(triphenylphosphin)-palladium(II)-dichlorid und 7 mg Kupfer(I)-jodid in 20 ml Diäthylamin vermischt. Man kühlt mit einem Aceton/Trockeneis-Bad ab und gibt einige Tropfen Propin zu. Man schliesst den Kolben dicht zu und rührt das Gemisch während 20 Stunden bei Raumtemperatur. Man kühlt dann wieder auf -70° ab, öffnet den Kolben und lässt auf Raumtemperatur erwärmen. Man verdünnt das Reaktionsgemisch mit Methylenchlorid und wäscht zweimal mit Wasser. Die organische Phase wird über Magnesiumsulfat getrocknet und eingeengt. Nach zweimaligem Umkristallisieren aus Essigester erhält man 8-Fluor-4,5-dihydro-5-methyl-3-(1-propinyl)-6H -imidazo[1,5-a][1,4]benzodiazepin-6-on vom Schmelzpunkt 219-220°.

Beispiel 19

a) 27,3 g (100 mMol) (S)-8-Chlor-11,12,13,13a-tetrahydro-9H -imadazo[1,5-a]pyrrolo[2,1-c][1,4]-benzodiazepin-9-on werden mit 88 g (350 mMol) Jod in 200 ml N,N-Dimethylformamid während 3 Stunden bei 100° gerührt. Man kühlt das Reaktionsgemisch ab, filtriert das ausgefallene Produkt ab, wäscht es mit Essigester nach und erhält nach Trocknen (S)-8-Chlor-11,12,13,13a-tetrahydro-1-jod-9H -imidazo[1,5-a]pyrrolo[2,1-c][1,4]benzodiazepin-9-on vom Schmelzpunkt 298-300°.

b) 3,0 g (7,5 mMol) (S)-8-Chlor-11,12,13,13a-tetrahydro-1-jod-9H -imidazo[1,5-a]pyrrolo[2,1-c][1,4]-benzodiazepin-9-on, 70 mg Bis-(triphenylphosphin)-palladium(II)-dichlorid und 10 mg Kupfer(I)-jodid werden mit 30 ml Diäthylamin in einem Druckrohr auf ca. -60° gekühlt und mit ca. 2 ml Propin versetzt. Man schliesst das Druckrohr und erhitzt während 20 Stunden auf 100°. Nach Abkühlen und Oeffnen des Druckrohres nimmt man das Reaktionsgemisch in Methylenchlorid auf, filtriert und wäscht das Filtrat zweimal mit Wasser. Man trocknet die organische Phase über Magnesiumsulfat und dampft sie ein. Nach Chromatographieren des Rückstandes an Kieselgel unter Eluieren mit Essigester und Kristallisieren aus Essigester und Hexan erhält man (S)-8-Chlor-11,12,13,13a-tetrahydro-1-(1-propinyl)-9H -imidazo[1,5-a]pyrrolo[2,1-c][1,4]benzodiazepin-9-on vom Schmelzpunkt 208-209°.

## Beispiel 20

a) 12,38 g (50 mMol) 7-Chlor-4,5-dihydro-5-methyl-6H-imidazo[1,5-a][1,4]benzodiazepin-6-on werden mit 9,80 g (55 mMol) N-Bromsuccinimid in 80 ml N,N-Dimethylformamid während 40 Minuten bei Raumtemperatur gerührt. Man giesst das Reaktionsgemisch auf 800 ml Wasser und filtriert die erhaltene Suspension. Man wäscht den Filterrückstand mit Wasser nach und nimmt ihn in Methylenchlorid auf. Die organische Phase wird über Magnesiumsulfat getrocknet und eingedampft. Nach Chromatographieren des Rückstandes an Kieselgel unter Eluieren mit Essigester und Umkristallisieren aus Essigester und Hexan erhält man 3-Brom-7-Chlor-4,5-dihydro-5-methyl-6H-imidazo[1,5-a][1,4]benzodiazepin-6-on vom Schmelzpunkt 178-179°.

b) 3,26 g (10 mMol) 3-Brom-7-chlor-4,5-dihydro-5-methyl-6H-imidazo[1,5-a][1,4]benzodiazepin-6-on werden mit 1,10 g (12 mMol) 2-Methyl-3-butin-2-ol, 70 mg Bis-(triphenylphosphin)-palladium(II)-dichlorid und 10 mg Kupfer(I)-jodid in 20 ml Diäthylamin und 15 ml Methylenchlorid über Nacht unter Rückfluss zum Sieden erhitzt. Man dampft das Reaktionsgemisch ein und löst den Rückstand in Methylenchlorid. Nach Chromatographieren der Lösung an Kieselgel unter Eluieren mit Essigester und Kristallisieren aus Essigester erhält man 7-Chlor-4,5-dihydro-3-(3-hydroxy-3-methyl-1-butinyl)-5-methyl-6H-imidazo[1,5-a]-[1,4]benzodiazepin-6-on vom Schmelzpunkt 194-195°.

## Beispiel 21

3,73 g (10 mMol) 7-Chlor-4,5-dihydro-3-jod-5-methyl -6H-imidazo[1,5-a][1,4]benzodiazepin-6-on werden mit 1,10 g (12 mMol) 2-Methyl-3-butin-2-ol und 20 ml Diäthylamin vermischt. Man gibt dann 70 mg Bis-(triphenylphosphin)-palladium(II)-dichlorid und 10 mg Kupfer(I)-jodid zu und rührt während 60 Stunden bei Raumtemperatur. Man dampft das Reaktionsgemisch ein und löst den Rückstand in Methylenchlorid. Die Lösung wird zweimal mit Wasser gewaschen, über Magnesiumsulfat getrocknet und eingedampft. Nach Umkristallisieren des Rückstandes aus Essigester erhält man 7-Chlor-4,5-dihydro-3-(3-hydroxy-3-methyl-1-butinyl)-5-methyl-6H-imidazo[1,5-a][1,4]benzodiazepin-6-on vom Schmelzpunkt 193-194°.

## Beispiel 22

660 mg (2 mMol) 7-Chlor-4,5-dihydro-3-(3-hydroxy-3-methyl-1-butinyl)-5 -methyl-6H-imidazo[1,5-a][1,4]-benzodiazepin-6-on werden mit 80 mg Natriumhydroxid in 10 ml Toluol über Nacht unter Rückfluss zum Sieden erhitzt. Nach Eindampfen des Lösungsmittels löst man den Rückstand in Methylenchlorid und wäscht die Lösung zweimal mit Wasser, trocknet sie über Magnesiumsulfat und dampft sie ein. Nach Umkristallisieren des Rückstandes aus Essigester erhält man 7-Chlor-3-äthinyl-4,5-dihydro-5-methyl-6H -imidazo[1,5-a][1,4]benzodiazepin-6-on vom Schmelzpunkt 200-201°.

## Beispiel 23

3,73 g (10 mMol) 7-Chlor-4,5-dihydro-3-jod-5-methyl-6H -imidazo[1,5-a][1,4]benzodiazepin-6-on werden mit 1,46 ml (12 mMol) 3,3-Dimethyl-1-butin, 70 mg Bis-(triphenylphosphin)-palladium(II)-dichlorid und 10 mg Kupfer(I)-jodid in 30 ml Diäthylamin während 24 Stunden bei Siedetemperatur gerührt. Nach Eindampfen des Reaktionsgemisches nimmt man den Rückstand in Methylenchlorid auf und wäscht zweimal mit

Wasser. Man trocknet die organische Phase über Magnesiumsulfat, dampft sie ein und chromatographiert den Rückstand an Kieselgel unter Eluieren mit Cyclohexan/Aether/Isopropanol (3:3:1). Nach Kristallisieren aus Essigester und Hexan erhält man 7-Chlor-3-(3,3-dimethyl-1-butinyl)-4,5-dihydro-5-methyl -6H-imidazo-[1,5-a][1,4]benzodiazepin-6-on vom Schmelzpunkt 126-128°.

Beispiel 24

3,73 g (10 mMol) 7-Chlor-4,5-dihydro-3-jod-5-methyl -6H-imidazo[1,5-a][1,4]benzodiazepin-6-on werden während 20 Stunden mit 1,12 g (11 mMol) Phenylacetylen, 70 mg Bis-(triphenylphosphin)-palladium(II)-dichlorid und 10 mg Kupfer(I)-jodid in 20 ml Diäthylamin bei Raumtemperatur gerührt. Man verdünnt das Reaktionsgemisch mit Methylenchlorid und wäscht zweimal mit Wasser. Die organische Phase wird über Magnesiumsulfat getrocknet und eingedampft. Nach Umkristallisieren des Rückstandes aus Essigester erhält man 7-Chlor-4,5-dihydro-5-methyl-3-(phenyläthinyl) -6H-imidazo[1,5-a][1,4]benzodiazepin-6-on vom Schmelzpunkt 205-206°.

Beispiel 25

a) 19,1 g (56,8 mMol) 7-Brom-5,6-dihydro-5-methyl-6-oxo -4H-imidazo[1,5-a][1,4]benzodiazepin-3-carbonsäure werden bei 290-300° decarboxyliert. Man nimmt die Schmelze in Methylenchlorid auf, verdünnt die Lösung mit Essigester und Aethanol und entfärbt sie mit Tierkohle. Nach Eindampfen und Umkristallisieren aus Essigester und Aethanol erhält man 7-Brom-4,5-dihydro-5-methyl -6H-imidazo[1,5-a][1,4]benzodiazepin-6-on vom Schmelzpunkt 196-197°.

b) 12,80 g (44 mMol) 7-Brom-4,5-dihydro-5-methyl -6H-imidazo[1,5-a][1,4]benzodiazepin-6-on werden mit 39 g (154 mMol) Jod in 80 ml N,N-Dimethylformamid während 3,5 Stunden bei 95° gerührt. Man dampft das Reaktionsgemisch ein, nimmt den Rückstand in Methylenchlorid und Wasser auf und entfärbt durch Zugabe von Natriumthiosulfat. Man filtriert und dampft das Filtrat ein. Nach Chromatographieren des Rückstandes an Kieselgel unter Eluieren mit Essigester und Umkristallisieren aus Methylenchlorid und Essigester erhält man 7-Brom-4,5-dihydro-3-jod-5-methyl -6H-imidazo[1,5-a][1,4]-benzodiazepin-6-on vom Schmelzpunkt 203-204°.

c) 3,74 g (8,95 mMol) 7-Brom-4,5-dihydro-3-jod-5-methyl -6H-imidazo[1,5-a][1,4]benzodiazepin-6-on werden während 1,5 Stunden mit 0,80 g (9,5 mMol) 2-Methyl-3-butin-2-ol, 70 mg Bis-(triphenylphosphin)-palladium(II)-dichlorid und 10 mg Kupfer(I)-jodid in 30 ml Diäthylamin bei Siedetemperatur gerührt. Man dampft das Reaktionsgemisch ein und chromatographiert den Rückstand an Kieselgel unter Eluieren mit Essigester. Nach Umkristallisieren des beim Eindampfen des Eluats verbleibenden Rückstandes aus Essigester erhält man 7-Brom-4,5-dihydro-3-(3-hydroxy-3-methyl-1-butinyl)-5-methyl -6H-imidazo[1,5-a]-[1,4]benzodiazepin-6-on vom Schmelzpunkt 207-208°.

Beispiel 26

a) 109,03 g (300 mMol) (S)-8-Brom-11,12,13,13a-tetrahydro-9-oxo-9H -imidazo[1,5-a]pyrrolo[2,1-c][1,4]-benzodiazepin-1-carbonsäure werden bei 290° decarboxyliert. Man löst die Schmelze in ca. 400 ml N,N-Dimethylformamid und giesst die Lösung auf 2,5 l Wasser. Nach 30-minütigem Rühren wird das ausgefallene Produkt abfiltriert, mit Wasser nachgewaschen und getrocknet. Man erhält (S)-8-Brom-11,12,13,13a-tetrahydro-9H -imidazo[1,5-a]pyrrolo[2,1-c][1,4]benzodiazepin-9-on vom Schmelzpunkt 232-233°.

b) 15,90 g (50 mMol) (S)-8-Brom-11,12,13,13a-tetrahydro-9H -imidazo[1,5-a]pyrrolo[2,1-c][1,4]-benzodiazepin-9-on werden mit 44 g (175 mMol) Jod und 14 g (100 mMol) Kaliumcarbonat in 100 ml N,N-Dimethylformamid während 3 Stunden bei 100° gerührt. Man giesst das Reaktionsgemisch auf 1 l Wasser und filtriert nach 30-minütigem Rühren das ausgefallene Produkt ab. Der Filterrückstand wird mit Wasser nachgewaschen, getrocknet und aus N,N-Dimethylformamid umkristallisiert. Man erhält (S)-8-Brom-11,12,13,13a-tetrahydro-1-jod-9H -imidazo[1,5-a]pyrrolo[2,1-c][1,4]benzodiazepin-9-on vom Schmelzpunkt 301-303°.

c) 2,22 g (5 mMol) (S)-8-Brom-11,12,13,13a-tetrahydro-1-jod -9H-imidazo[1,5-a]pyrrolo[2,1-c][1,4]-benzodiazepin-9-on werden mit 0,44 g (5 mMol) 2-Methyl-3-butin-2-ol, 25 mg Palladium(II)-acetat, 100 mg Triphenylphosphin und 10 mg Kupfer(I)-jodid in 20 ml Triäthylamin und 20 ml N,N-Dimethylformamid während 8 Stunden unter Rückfluss bei Siedetemperatur gerührt. Man dampft das Reaktionsgemisch ein und chromatographiert den Rückstand an Kieselgel unter Eluieren mit Essigester. Nach Eindampfen des Eluats und Umkristallisieren des Rückstandes aus Essigester erhält man (S)-8-Brom-11,12,13,13a-tetrahydro-1-(3-hydroxy-3-methyl -1-butinyl)-9H-imidazo[1,5-a]pyrrolo[2,1-c][1,4]benzodiazepin-9-on vom Schmelzpunkt 227-228°.

Beispiel 27

2,83 g (7 mMol) (S)-8-Chlor-11,12,13,13a-tetrahydro 1-jod-9H-imidazo[1,5-a]pyrrolo[2,1-c][1,4]-benzodiazepin-9-on werden mit 1,10 g (11 mMol) Phenylacetylen, 25 mg Palladium(II)-acetat, 100 mg Triphenylphosphin und 15 mg Kupfer(I)-jodid in 40 ml Triäthylamin und 20 ml N,N-Dimethylformamid während 60 Stunden bei 105° gerührt. Man dampft dann das Gemisch zur Trockene ein und chromatographiert den Rückstand an Kieselgel unter Eluieren mit Essigester. Nach Umkristallisieren aus Methylenchlorid und Essigester erhält man (S)-8-Chlor-11,12,13,13a-tetrahydro-1-(phenyläthinyl) -9H-imidazo[1,5-a][pyrrolo-[2,1-c][1,4]benzodiazepin-9-on vom Schmelzpunkt 241-242°.

Beispiel 28

3,99 g (10 mMol) (S)-8-Chlor-11,12,13,13a-tetrahydro-1 -jod-9H-imidazo[1,5-a]pyrrolo[2,1-c][1,4]-benzodiazepin-9-on werden über Nacht mit 0,88 g (10,5 mMol) 2-Methyl-3-butin-2-ol, 25 mg Palladium(II)-acetat, 100 mg Triphenylphosphin und 10 mg Kupfer(I)-jodid in 40 ml Triäthylamin und 20 ml N,N-Dimethylformamid bei 100° gerührt. Man dampft dann das Gemisch zur Trockene ein und chromatographiert den Rückstand an Kieselgel unter Eluieren mit Essigester. Nach Umkristallisieren aus Essigester erhält man (S)-8-Chlor-11,12,13,13a-tetrahydro-1-(3-hydroxy-3-methyl -1-butinyl)-9H-imidazo[1,5-a]pyrrolo-[2,1-c][1,4]benzodiazepin -9-on vom Schmelzpunkt 234-235°.

Beispiel 29

4,18 g (10 mMol) 7-Brom-4,5-dihydro-3-jod-5-methyl-6H -imadazo[1,5-a][1,4]benzodiazepin-6-on werden während 2 Stunden mit 1,12 g (11 mMol) Phenylacetylen, 70 mg Bis-(triphenylphosphin)-palladium(II)-dichlorid und 10 mg Kupfer(I)-jodid in 30 ml Diäthylamin unter Rückfluss zum Sieden erhitzt. Man dampft dann das Lösungsmittel ab und chromatographiert den Rückstand an Kieselgel unter Eluieren mit Essigester. Nach Umkristallisieren aus Essigester erhält man 7-Brom-4,5-dihydro-5-methyl-3-(phenyläthinyl) -6H-imidazo[1,5-a][1,4]benzodiazepin-6-on vom Schmelzpunkt 207-209°.

Beispiel 30

3,40 g (10 mMol) 4,5-Dihydro-3-jod-5-methyl-6H -imidazo[1,5-a][1,4]benzodiazepin-6-on werden mit 0,925 g (11 mMol) 2-Methyl-3-butin-2-ol, 70 mg Bis-(triphenylphosphin)-palladium(II)-dichlorid und 10 mg Kupfer(I)-jodid während 1,5 Stunden in 30 ml Diäthylamin unter Rückfluss zum Sieden erhitzt. Man dampft dann das Lösungsmittel ab und chromatographiert den Rückstand an Kieselgel unter Eluiren mit Essigester. Nach Umkristallisieren aus Essigester erhält man 4,5-Dihydro-3-(3-hydroxy-3-methyl-1-butinyl)-5-methyl -6H-imidazo[1,5-a][1,4]benzodiazepin-6-on vom Schmelzpunkt 168-169°.

Beispiel 31

3,90 g (11,5 mMol) 4,5-Dihydro-3-jod-5-methyl-6H -imidazo[1,5-a][1,4]benzodiazepin-6-on werden mit 0,67 g (12 mMol) Propargylalkohol, 70 mg Bis-(triphenylphosphin)-palladium(II)-dichloridund 10 mg Kupfer-(I)-jodid während 5 Stunden in 30 ml Diäthylamin unter Rückfluss zum Sieden erhitzt. Man dampft dann das Lösungsmittel ab, nimmt den Rückstand im Methylenchlorid auf, filtriert den Festkörper ab und wäscht ihn mit Essigester nach. Nach Trocknung erhält man 4,5-Dihydro-3-(3-hydroxy-1-propinyl)-5-methyl-6H -imidazo[1,5-a][1,4]benzodiazepin-6-on vom Schmelzpunkt 240-241°.

Beispiel 32

1,20 g (18,6 mMol) frisch pulverisiertes Kaliumhydroxid werden in 15 ml Dimethylsulfoxid während 5 Minuten bei Raumtemperatur gerührt. Dann gibt man nacheinander 1,65 g (5 mMol) 7-Chlor-4,5-dihydro-3-(3-hydroxy-3-methyl-1-butinyl) -5-methyl-6H-imidazo[1,5-a][1,4]benzodiazepin-6-on und 1,42 g (10 mMol) Methyljodid zu, wobei die Temperatur auf 35° steigt. Man rührt während 45 Minuten und giesst dann das Reaktionsgemisch auf 50 ml Wasser. Man extrahiert fünfmal mit Methylenchlorid, trocknet die vereinigten organischen Phasen über Magnesiumsulfat und dampft sie ein. Nach Kristallisieren aus Diisopropyläther

erhält man 7-Chlor-4,5-dihydro-3-(3-methoxy-3-methyl-1-butinyl) -5-methyl-6H-imidazo[1,5-a][1,4]-benzodiazepin-6-on vom Schmelzpunkt 172-174°.

Beispiel 33

7,47 g (20 mMol) 7-Chlor-4,5-dihydro-3-jod-5-methyl-6H -imidazo[1,5-a][1,4]benzodiazepin-6-on werden mit 1,75 g (25 mMol) 3-Butin-2-ol, 70 mg Bis-(triphenylphosphin)-palladium(II)-dichlorid und 10 mg Kupfer-(I)-jodid in 50 ml Diäthylamin und 20 ml Aethylenchlorid während 4 Stunden unter Rückfluss zum Sieden erhitzt. Nach Abdampfen des Lösungsmittels nimmt man den Rückstand in Methylenchlorid auf und nutscht die so erhaltene Suspension ab. Man wäscht das erhaltene Material mit Methylenchlorid und erhält nach Umkristallisieren aus Essigester 7-Chlor-4,5-dihydro-3-(3-hydroxy-1-butinyl) -5-methyl-6H-imidazo[1,5-a]-[1,4]benzodiazepin-6-on vom Schmelzpunk 251-252°C.

Beispiel 34

3,73 g (10 mMol) 7-Chlor-4,5-dihydro-3-jod-5-methyl-6H-imidazo[1,5-a][1,4]benzodiazepin-6-on werden mit 1,40 g (12,5 mMol) 3-Aethyl-1-pentin-3-ol, 70 mg Bis-(triphenylphosphin)-palladium(II)-dichlorid und 10 mg Kupfer(I)-jodid in 30ml Diäthylamin während 2 Stunden unter Rückfluss zum Sieden erhitzt. Man dampft das Reaktionsgemisch ein und chromatographiert den Rückstand an Kieselgel unter Eluieren mit Essigester. Nach Umkristallisieren aus Essigester erhält man 7-Chlor-3-(3-äthyl-3-hydroxy-1-pentinyl)-4,5-dihydro-5-methyl-6H-imidazo[1,5-a][1,4]benzodiazepin-6-on vom Schmelzpunkt 186-188°C.

Beispiel 35

9,57 g (25,5 mMol) 7-Chlor-4,5-dihydro-3-jod-5-methyl-6H-imidazo[1,5-a][1,4]benzodiazepin-6-on werden mit 3,52 g (32 mMol) 1-Aethinylcyclopentanol, 170 mg Bis-(triphenylphosphin)-palladium(II)-dichlorid und 30 mg Kupfer(I)-jodid während 3 Stunden in 60 ml Diäthylamin unter Rückfluss zum Sieden erhitzt. Man dampft das Reaktionsgemisch ein und chromatographiert den Rückstand an Kieselgel unter Eluieren mit Essigester. Nach Kristallisieren aus Essigester erhält man 7-Chlor-4,5-dihydro-3-[(1-hydroxycyclopentyl)äthinyl]-5-methyl-6H-imidazo[1,5-a][1,4]benzodiazepin-6-on vom Schmelzpunkt 207-208°C.

Beispiel 36

3,73 g (10 mMol) 7-Chlor-4,5-dihydro-3-jod-5-methyl-6H-imidazo[1,5-a][1,4]benzodiazepin-6-on werden mit 1,09 g (13 mMol) Aethinyl-äthylcarbinol, 70 mg Bis-(triphenylphosphin)-palladium(II)-dichlorid und 10 mg Kupfer(I)-jodid in 30 ml Diäthylamin während 5 Stunden unter Rückfluss erhitzt. Man dampft das Reaktionsgemisch ein und chromatographiert den Rückstand an Kieselgel unter Eluieren mit Essigester. Nach Umkristallisieren aus Essigester erhält man 7-Chlor-4,5-dihydro-3-(3-hydroxy-1-pentinyl)-5-methyl-6H-imidazo[1,5-a][1,4]benzodiazepin-6-on vom Schmelzpunkt 178°C.

Beispiel 37

10,0 g (26,7 mMol) 7-Chlor-4,5-dihydro-3-jod-5-methyl-6H-imidazo[1,5-a][1,4]benzodiazepin-6-on, 140 mg Bis-(triphenylphosphin)-palladium(II)-dichlorid, 40 mg Kupfer(I)-jodid und 3,4 ml (40,3 mMol) Methylpropargyläther werden in 100 ml Diäthylamin während 3,5 Stunden unter Rückfluss zum Sieden erhitzt. Man dampft das Reaktionsgemisch ein und chromatographiert den Rückstand an Kieselgel unter Eluieren mit Essigester. Nach Umkristallisieren aus Essigester erhält man 7-Chlor-4,5-dihydro-3-(3-methoxy-1-propinyl)-5-methyl-6H-imidazo[1,5-a][1,4]benzodiazepin-6-on vom Schmelzpunkt 154-156°C.

Beispiel 38

3,73 g (10 mMol) 7-Chlor-4,5-dihydro-3-jod-5-methyl-6H-imidazo[1,5-a][1,4]benzodiazepin-6-on werden mit 0,87 g 3-Methyl-1-butin, 70 mg Bis-(triphenylphosphin)-palladium(II)-dichloridund 19 mg Kupfer(I)-jodid während 20 Stunden in 20 ml Diäthylamin und 10 ml Aethylenchlorid in einem Druckrohr auf 100°C erhitzt. Durch Eindampfen des Reaktionsgemisches und Chromatographieren des Rückstandes an Kieselgel unter Eluieren mit Essigester erhält man ein Gemisch aus Produkt und Ausgangsstoff von etwa 2:1. Um den Ausgangsstoff zu entfernen, wird das Gemisch mit 0,8 ml 2-Methyl-3-butin-2-ol, 70 mg Bis-(triphenylphosphin)-palladium(II)-dichlorid und 15 mg Kupfer(I)-jodid in 15 ml Diäthylamin während 5

Stunden unter Rückfluss erhitzt. Durch Eindampfen des Reaktionsgemisches und Chromatographieren des Rückstandes an Kieselgel unter Eluieren mit Essigester kann das Nebenprodukt vom gewünschten Produkt abgetrennt werden. Man erhält nach Umkristallisieren aus Aether 7-Chlor-4,5-dihydro-5-methyl-3-(3-methyl-1-butinyl)-6H-imidazo[1,5-a][1,4]benzodiazepin-6-on vom Schmelzpunkt 128-130 °C.

Beispiel 39

3,37 g (10 mMol) 7-Chlor-4,5-dihydro-3-jod-5-methyl-6H-imidazo[1,5-a][1,4]benzodiazepin-6-on werden mit 1,47 g Cyclopropylacetylen, 70 mg Bis-(triphenylphosphin)-palladium(II)-dichlorid und 20 mg Kupfer(I)-jodid während 16 Stunden in 20 ml Diäthylamin und 10 ml Aethylenchlorid auf 75 °C erhitzt. Durch Eindampfen des Reaktionsgemisches und Chromatographieren des Rückstandes an Kieselgel unter Eluieren mit Essigester erhält man ein Gemisch aus Produkt und Ausgangsstoff. Um den Ausgangsstoff zu entfernen, wird das Gemisch mit 1 ml 2-Methyl-3-butin-2-ol, 70 mg Bis-(triphenylphosphin)-palladium(II)-dichlorid und 20 mg Kupfer(I)-jodid in 10 ml Diäthylamin und 10 ml Aethylenchlorid während 4 Stunden unter Rückfluss erhitzt. Durch Eindampfen des Reaktionsgemisches und Chromatographieren des Rückstandes an Kieselgel unter Eluieren mit Essigester kann das Nebenprodukt vom gewünschten Produkt abgetrennt werden. Man erhält nach Kristallisieren aus Essigester 7-Chlor-3-(cyclopropyläthinyl)-4,5-dihydro-5-methyl-6H-imidazo[1,5-a][1,4]benzodiazepin-6-on vom Schmelzpunkt 177-178 °C.

Beispiel 40

4,07 g (15 mMol) 7-Chlor-3-äthinyl-4,5-dihydro-5-methyl-6H-imidazo[1,5-a][1,4]benzodiazepin-6-on werden in 30 ml Tetrahydrofuran suspendiert und innert 35 Minuten bei maximal -5 °C tropfenweise mit 20 ml (30 mMol) 1,5M-Butyllithium in Hexan versetzt. Nach 1,5-stündigen Rühren im Eisbad kühlt man auf -74 °C ab und gibt 5 ml Hexamethyl-phosphorsäuretriamid und nach 10 Minuten 3,30 g (30 mMol) Dicyclopropylketon dazu. Man lässt während 6 Stunden auf Raumtemperatur kommen und rührt über das Wochenende weiter. Das Reaktionsgemisch wird dann auf 200 ml Wasser gegossen, mit 4N Salzsäure auf pH 7 angesäuert und viermal mit Essigester extrahiert. Man wäscht die vereinigten organischen Extrakte zweimal mit Wasser, trocknet sie über Magnesiumsulfat und dampft sie ein. Nach Chromatographieren des Rückstandes an Kieselgel unter Eluieren mit Methylenchlorid/Methanol (19/1) und anschliessendem Umkristallisieren aus Essigester erhält man 7-Chlor-3-(3,3-dicyclopropyl-3-hydroxy-1-propinyl)-4,5-dihydro-5-methyl-6H-imidazo[1,5-a][1,4]benzodiazepin-6-on vom Schmelzpunkt 179-181 °C.

Beispiel 41

2,72 g (10 mMol) 7-Chlor-3-äthinyl-4,5-dihydro-5-methyl-6H-imidazo[1,5-a][1,4]benzodiazepin-6-on werden in 30 ml Methanol suspendiert und auf 10 °C abgekühlt. Man gibt dann gleichzeitig innert ca. 10 Minuten 5 ml 28%ige Natronlauge und 3 g Jod portionenweise dazu. Nach 1-stündigem Rühren bei Raumtemperatur wird die Suspension mit ca. 30 ml Wasser verdünnt, abgenutscht, und der Nutschkuchen wird getrocknet. Durch zwei Umkristallisationen aus Methylenchlorid und Essigester und aus Dioxan erhält man 7-Chlor-4,5-dihydro-3-(2-jodäthinyl)-5-methyl-6H-imidazo[1,5-a][1,4]benzodiazepin-6-on vom Schmelzpunkt 215-216 °C.

Beispiel 42

4, 0 g (69,5 mMol) frisch pulverisiertes Kaliumhydroxid werden in 40 ml N,N-dimethylformamid während 10 Minuten verrührt und auf 10 °C abgekühlt. Man gibt nacheinander 5,48 g (17,3 mMol) 7-Chlor-4,5-dihydro-3-(3-hydroxy-1-butinyl)-5-methyl-6H-imidazo[1,5-a][1,4]benzodiazepin-6-on und 4,94 g (35 mMol) Methyljodid dazu, entfernt das Eisbad und rührt 1 Stunde weiter. Man giesst das Reaktionsgemisch auf 200 ml Wasser und extrahiert es fünfmal mit Methylenchlorid. Die vereinigten organischen Extrakte werden viermal mit Wasser gewaschen, über Magnesiumsulfat getrocknet und eingedampft. Nach Umkristallisieren des Rückstandes aus Essigester erhält man 7-Chlor-4,5-dihydro-3-(3-methoxy-1-butinyl)-5-methyl-6H-imidazo[1,5-a][1,4]benzodiazepin-6-on vom Schmelzpunkt 139-141 °C.

Beispiel 43

2,35 g (42 mMol) frisch pulverisiertes Kaliumhydroxid werden während 5 Minuten in 30 ml Dimethylsulfoxid verrührt. Man gibt dann nacheinander 4,0 g (11,25 mMol) 7-Chlor-4,5-dihydro-3-[(1-hydroxycyclope-

ntyl)äthinyl]-5-methyl-6H-imidazo[1,5-a][1,4]benzodiazepin-6-on und 3,12 g (22,5 mMol) Methyljodid dazu und rührt während 1 Stunde weiter, bevor man das Dimethylsulfoxid abdampft. Den Rückstand nimmt man in Wasser auf und extrahiert ihn dreimal mit Methylenchlorid. Man trocknet die vereinigten organischen Extrakte über Magnesiumsulfat, dampft sie ein und chromatographiert den Rückstand an Kieselgel unter Eluieren mit Essigester/Hexan (1/1). Nach Umkristallisieren aus Esisgester erhält man 7-Chlor-4,5-dihydro-3-[(1-methoxycyclopentyl)äthinyl]-5-methyl-6H-imidazo[1,5-a][1,4]benzodiazepin-6-on vom Schmelzpunkt 174-175 ° C.

Beispiel 44

a) 3,73 g (10 mMol) 7-Chlor-4,5-dihydro-3-jod-5-methyl-6H-imidazo[1,5-a][1,4]benzodiazepin-6-on werden mit 1,30 g (12,5 mMol) Aethinyltrimethylsilan, 70 mg Bis-(triphenylphosphin)-palladium(II)-dichlorid und 10 mg Kupfer(I)-jodid in 30 ml Diäthylamin während 3 Stunden unter Rückfluss zum Sieden erhitzt. Man dampft das Reaktionsgemisch ein und chromatographiert den Rückstand an Kieselgel unter Eluieren mit Essigester/Hexan (1/1). Nach Kristallisieren aus Essigester und Hexan erhält man 7-Chlor-4,5-dihydro-5-methyl-3-[(trimethylsilyl)äthinyl]-6H-imidazo[1,5-a][1,4]benzodiazepin-6-on vom Schmelzpunkt 186-187 ° C.

b) 12,7 g (37 mMol) 7-Chlor-4,5-dihydro-5-methyl-3-[(trimethylsilyl)äthinyl]-6H-imidazo[1,5-a][1,4]-benzodiazepin-6-on werden in 40 ml Methanol gelöst und mit 40 ml 1-normaler Kalilauge versetzt. Nach 1-stündigem Rühren wird der Methanol abgedampft und die wässrige Suspension abgekühlt und abgenutscht. Nach Trocknen erhält man 7-Chlor-3-äthinyl-4,5-dihydro-5-methyl-6H-imidazo[1,5-a][1,4]-benzodiazepin-6-on vom Schmelzpunkt 194-195 ° C.

Beispiel 45

a) 3,0 g (10,9 mMol) 7-Chlor-5,6-dihydro-5-methyl-6-oxo-4H-imidazo[1,5-a][1,4]benzodiazepin-3-carboxaldehyd und 5,7 g (21,7 mMol) Triphenylphosphin werden in 80 ml Methylenchlorid bei Raumtemperatur gelöst. Danach wird mit einem Eisbad auf 0 ° C abgekühlt und bei dieser Temperatur eine Lösung von 4,0 g (12,0 mMol) Tetrabrommethan in 15 ml Methylenchlorid dazugetropft. Danach wird noch 6 Stunden bei Raumtemperatur gerührt und anschliessend im Vakuum eingedampft. Der Rückstand wird in Essigester suspendiert und 30 Minuten unter Rückfluss erhitzt, unter Rühren auf 10 ° C abgekühlt, genutscht und getrocknet. Das kristalline Rohprodukt wird aus Alkohol umkristallisiert, und man erhält 7-Chlor-3-(2,2-dibromvinyl)-4,5-dihydro-5-methyl-6H-imidazo[1,5-a][1,4]benzodiazepin-6-on-hydrobromid, als weisse Kristalle vom Schmelzpunkt 215-217 ° C.

b) 3,3 g (6,44 mMol) der so erhaltenen Verbindung werden im 100 ml Methanol gelöst. Dazu gibt man eine Lösung von 0,31 g (13,4 mMol) Natrium in 30 ml Methanol. Das Reaktionsgemisch wird danach 16 Stunden unter Rückfluss erhitzt und anschliessend im Vakuum eingedampft. Der Rückstand wird zwischen Wasser und Methylenchlorid verteilt, und die wässrige Phase wird mit Methylenchlorid extrahiert. Die vereinigten organischen Phasen werden mit Wasser gewaschen, über Magnesiumsulfat getrocknet, filtriert und eingedampft. Der Rückstand wird in Methylenchlorid gelöst und über 120 g Kieselgel (in Methylenchlorid) chromatographiert. Eluiert wird mit einem Gemisch aus Methylenchlorid und Essigester im Verhältnis 9:1, 8:2 und 7:3 (v/v). Die nach Dünnschichtchromatogramm reinen Fraktionen werden vereinigt und aus Essigester/Hexan umkristallisiert. Man erhält 3-(Bromäthinyl)-7-Chlor-4,5-dihydro-5-methyl-6H-imidazo[1,5-a][1,4]benzodiazepin-6-on als weisse Kristalle vom Schmelzpunkt >190 ° C (Zers.).

Beispiel 46

10,3 g (29 mMol) Acetonyltriphenylphosphoniumchlorid werden unter Argon in 100 ml Tetrahydrofuran suspendiert. Dazu gibt man 3,3 g (29,4 mMol) Kalium-tert.-butylat und rührt danach noch 30 Minuten bei Raumtemperatur. Nach dem Abkühlen auf 10 ° C gibt man 4,0 g (14,5 mMol) 7-Chlor-5,6-dihydro-5-methyl-6-oxo-4H-imidazo[1,5-a][1,4]benzodiazepin-3-carboxaldehyd dazu und rührt 1 Stunde bei 20 ° C, sowie 6 Stunden unter Rückfluss. Das Reaktionsgemisch wird eingedampft. Der Rückstand wird zwischen Methylenchlorid und Wasser verteilt. Die organische Phase wird mit Wasser gewaschen und anschliessend über Magnesiumsulfat getrocknet, filtriert und eingedampft. Nach Chromatographieren des Rückstandes an Kieselgel mit Methylenchlorid/Essigester 8:2, 7:3 und 1:1 und anschliessendem Umkristallisieren der vereinigten, reinen Fraktionen aus Essigester/Hexan erhält man 7-Chlor-4,5-dihydro-3-[(E)-3-oxo-1-butenyl]-5-methyl-6H-imidazo[1,5-a][1,4]benzodiazepin-6-on, als weisse Kristalle vom Schmelzpunkt 235-237 ° C.

Beispiel 47

2,4 g (7,6 mMol) 7-Chlor-4,5-dihydro-3-[(E)-3-oxo-1-butenyl]-5-methyl-6H-imidazo[1,5-a][1,4]-benzodiazepin-6-on werden in 50 ml Dimethylformamid unter Erwärmen gelöst. Danach wird mit 280 ml Aethanol verdünnt. Zu dieser Lösung gibt man 0,6 g (15,8 mMol) Natriumborhydrid und rührt 2,5 Stunden bei 20 ° C, danach gibt man nochmals 0,2 g Natriumborhydrid dazu. Nach einer Reaktionsdauer von total 4,5 Stunden wird im Vakuum eingedampft. Der noch Dimethylformamid enthaltende Rückstand wird auf Eiswasser gegossen und 1 Stunde bei 0 ° C gerührt. Das Kristallisat wird abgenutscht und getrocknet. Man erhält 7-Chlor-4,5-dihydro-3-[(E)-3-hydroxy-1-butenyl]-5-methyl-6H-imidazo[1,5-a][1,4]benzodiazepin-6-on, als weisse Kristalle vom Schmelzpunkt 232-233 ° C.

Beispiel 48

3,57 g (10 mMol) 8-Fluor-4,5-dihydro-3-jod-5-methyl-6H-imidazo[1,5-a][1,4]benzodiazepin-6-on werden mit 0,925 g (11 mMol) 2-Methyl-3-butin-2-ol, 70 mg Bis-(triphenylphosphin)-palladium(II)-dichlorid und 10 mg Kupfer(I)-jodid in 30 ml Diäthylamin während 6,5 Stunden unter Rückfluss zum Sieden erhitzt. Das Reaktionsgemisch wird dann eingedampft, und der Rückstand wird an Kieselgel chromatographiert unter Eluieren mit Essigester. Nach Umkristallisieren aus Essigester erhält man 8-Fluor-4,5-dihydro-3-(3-hydroxy-3-methyl-1-butinyl)-5-methyl-6H-imidazo[1,5-a][1,4]benzodiazepin-6-on vom Schmelzpunkt 165-167 ° C.

Beispiel 49

8,93 g (25 mMol) 8-Fluor-4,5-dihydro-3-jod-5-methyl-6H-imidazo[1,5-a][1,4]benzodiazepin-6-on werden mit 3,03 g (27 mMol) 3-Aethyl-1-pentin-3-ol, 100 mg Bis-(triphenylphosphin-palladium(II)-dichlorid und 20 mg Kupfer(I)-jodid in 60 ml Diäthylamin während 24 Stunden bei Raumtemperatur gerührt. Man gibt noch 0.87 g (7,7 mMol) 3-Aethyl-1-pentin-3-ol sowie 50 mg Bis-(triphenylphosphin)-palladium(II)-dichlorid und 50 mg Kupfer(I)-jodid dazu und rührt 0,5 Stunden bei Siede- temperatur. Man dampft anschliessend das Reaktionsgemisch ein und chromatographiert den Rückstand an Kieselgel unter Eluieren mit Essigester. Nach Entfärben des Rohproduktes mit Aktivkohle und Kristallisieren aus Essigester erhält man 8-Fluor-3-(3-äthyl-3-hydroxy-1-pentinyl)-4,5-dihydro-5-methyl-6H-imidazo[1,5-a][1,4]benzodiazepin-6-on vom Schmelzpunkt 135-136 ° C.

Beispiel 50

3,57 g (10 mMol) 8-Fluor-4,5-dihydro-3-jod-5-methyl-6H-imidazo[1,5-a][1,4]benzodiazepin-6-on werden mit 1,22 g (12,5 mMol) 3-Methoxy-3-methyl-1-butin, 70 mg Bis-(triphenylphosphin)-palladium(II)-dichlorid und 10 mg Kupfer(I)-jodid in 30 ml Diäthylamin während 4 Stunden unter Rückfluss zum Sieden erhitzt. Man dampft das Reaktionsgemisch ein und chromatographiert den Rückstand an Kieselgel unter Eluieren mit Essigester. Nach Umkristallisieren aus Essigester erhält man 8-Fluor-4,5-dihydro-3-(3-methoxy-3-methyl-1-butinyl)-5-methyl-6H-imidazo[1,5-a][1,4]benzodiazepin-6-on vom Schmelzpunkt 149-150 ° C.

Beispiel 51

7 g (19,9 mMol) 8-Fluor-4,5-dihydro-3-jod-5-methyl-6H-imidazo[1,5-a][1,4]benzodiazepin-6-on werden mit 2,5 ml (29 mMol) Propargyläther, 100 mg Bis-(triphenylphosphin)-palladium(II)-dichloridund 30 mg Kupfer(I)-jodid während 3 Stunden in 70 ml Diäthylamin unter Rückfluss zum Sieden erhitzt. Man dampft das Reaktionsgemisch ein und chromatographiert den Rückstand an Kieselgel unter Eluieren mit Essigester. Nach Umkristallisieren aus Essigester erhält man 8-Fluor-4,5-dihydro-3-(3-methoxy-1-propinyl)-5-methyl-6H-imidazo[1,5-a][1,4]benzodiazepin-6-on vom Schmelzpunkt 146-147 ° C.

Beispiel 52

3,6 g (mMol) 8-Fluor-4,5-dihydro-3-jod-5-methyl-6H-imidazo[1,5-a][1,4]benzodiazepin-6-on werden mit 1,5 g (11 mMol) p-Chlorphenylacetylen, 70 mg Bis-(triphenylphosphin)-palladium(II)-dichlorid und 15 mg Kupfer(I)-jodid während 16 Stunden in 35 ml Diäthylamin bei Raumtemperatur verrührt. Das Reaktionsgemisch wird mit 175 ml Methylenchlorid verdünnt und dreimal mit Wasser gewaschen. Man trocknet die organische Phase über Magnesiumsulfat und dampft sie ein. Durch Chromatographieren des Rückstandes an Kieselgel und anschliessendes Umkristallisieren aus Methylenchlorid und Essigester erhält man 3-[(p-Chlorphenyl)äthinyl]-8-fluor-4,5-dihydro-5-methyl-6H-imidazo[1,5-a][1,4]benzodiazepin-6-on vom Schmelzpunkt 221-222 ° C.

Beispiel 53

3,6 g (10 mMol) 8-Fluor-4,5-dihydro-3-jod-5-methyl-6H-imidazo[1,5-a][1,4]benzodiazepin-6-on werden mit 1,5 g (11 mMol) o-Chlorphenylacetylen, 70 mg Bis-(triphenylphosphin)-palladium(II)-dichlorid und 15 mg Kupfer(I)-jodid in 20 ml Diäthylamin über Nacht bei Raumtemperatur gerührt. Man verdünnt das Reaktionsgemisch mit 175 ml Methylenchlorid und wäscht es dreimal mit Wasser. Nach Trocknen und Eindampfen der Lösung wird der Rückstand an Kieselgel chromatographiert unter Eluieren mit Methylenchlorid /Methanol (99/1). Durch Umkristallisieren des eingedampften Eluates erhält man 3-[(o-Chlorphenyl)äthinyl]-8-fluor-4,5-dihydro-5-methyl-6H-imidazo[1,5-a][1,4]benzodiazepin-6-on vom Schmelzpunkt 259-261 ° C.

Beispiel 54

1,27 g (5 mMol) 3-Aethinyl-8-fluor-4,5-dihydro-5-methyl-6H-imidazo[1,5-a][1,4]benzodiazepin-6-on werden in 10 ml Tetrahydrofuran suspendiert und bei maximal -5 ° C mit 6,4 ml (10 mMol) Butyllithium 1,6m in Hexan innert 20 Minuten tropfenweise versetzt. Nach 2-stündigem Rühren im Eisbad wird auf -70 ° C gekühlt und 1,7 ml Hexamethyl-phosphorsäuretriamid und 0.6 g (10 mMol) Aceton nacheinander dazugegeben. Man lässt innert 4,5 Stunden auf Raumtemperatur kommen, über Nacht stehen und giesst auf ca. 120 ml Wasser. Nach Ansäuern mit 4-normaler Salzsäure extrahiert man viermal mit Aether und sechsmal mit Essigester, trocknet die vereinigten Extrakte über Magnesiumsulfat und dampft sie ein. Man chromatographiert den Rückstand an Kieselgel unter Eluieren mit Essigester und erhält nach Kristallisieren aus Essigester 8-Fluor-4,5-dihydro-3-(3-hydroxy-3-methyl-1-butinyl)-5-methyl-6H-imidazo[1,5-a][1,4]-benzodiazepin-6-on vom Schmelzpunkt 163-164 ° C.

Beispiel 55

2,55 g (10 mMol) 3-Aethinyl-8-fluor-4,5-dihydro-5-methyl-6H-imidazo[1,5-a][1,4]benzodiazepin-6-on werden in 20 ml Tetrahydrofuran suspendiert und bei -5 ° C mit 13 ml (20 mMol) 1,6-Butyllithium in Hexan innert 30 Minuten tropfenweise versetzt. Nach 2-stündigem Rühren im Eisbad wird auf -70 ° C gekühlt, und 3,4 ml Hexamethyl-phosphorsäuretriamid und 1,68 g (20 mMol) Cyclopropyl-methylketon werden nacheinander dazugegeben. Man lässt innert 4 Stunden auf Raumtemperatur kommen, über Nacht rühren und giesst auf ca. 200 ml Wasser. Nach Ansäuern mit 4-normaler Salzsäure extrahiert man fünfmal mit Essigester, trocknet die vereinigten Extrakte über Magnesiumsulfat und dampft sie ein. Man chromatographiert den Rückstand an Kieselgel unter Eluieren mit Essigester und erhält nach Kristallisieren aus Essigester 8-Fluor-3-(3-cyclopropyl-3-hydroxy-1-butinyl)-4,5-dihydro-5-methyl-6H-imidazo[1,5-a][1,4]-benzodiazepin-6-on vom Schmelzpunkt 191-192 ° C.

Beispiel 56

3,83 g (15 mMol) 3-Aethinyl-8-fluor-4,5-dihydro-5-methyl-6H-imidazo[1,5-a][1,4]benzodiazepin-6-on werden in 30 ml Tetrahydrofuran suspendiert und bei maximal -5 ° C mit 20 ml (30 mMol) 1,6M-Butyllithium in Hexan innert 30 Minuten tropfenweise versetzt. Nach 2-stündigem Rühren im Eisbad wird auf -70 ° C gekühlt, und 5 ml Hexamethyl-phosphorsäuretriamid und 2 g (28 mMol) Cyclobutanon werden nacheinander dazugegeben. Man lässt innert 5 Stunden auf Raumtemperatur kommen, über Nacht rühren und giesst auf 300 ml Wasser. Nach Ansäuern mit 4-normaler Salzsäure extrahiert man fünfmal mit Essigester. Man wäscht die vereinigten organischen Extrakte mit Wasser, trocknet sie über Magnesiumsulft und dampft sie ein. Durch Chromatographieren des Rückstandes an Kieselgel unter Eluieren mit Essigester und Kristallisieren aus Essigester erhält man 8-Fluor-4,5-dihydro-3-[(1-hydroxycyclobutyl)äthinyl]-5-methyl-6H-imidazo[1,5-a][1,4]benzodiazepin-6-on vom Schmelzpunkt 177-179 ° C.

Beispiel 57

3,0 g (9,5 mMol) 8-Fluor-4,5-dihydro-3-(3-hydroxy-3-methyl-1-butinyl)-5-methyl-6H-imidazo[1,5-a][1,4]-benzodiazepin-6-on werden in 20 ml N,N-Dimethylformamid gelöst und bei 3 ° C mit 2.97 g (19 mMol) Aethyljodid und 2,13 g frisch pulverisiertem Kaliumhydroxid (38 mMol) versetzt. Nach 5-minütigem Rühren entfernt man das Kühlbad und lässt auf Raumtemperatur kommen. Das Reaktionsgemisch wird dann auf

200 ml Wasser gegossen, mit 4-normaler Salzsäure angesäuert und viermal mit Methylenchlorid extrahiert. Man wäscht die vereinigten Extrakte fünfmal mit Wasser, trocknet über Magnesiumsulfat und dampft ein. Durch Umkristallisieren des Rohproduktes aus Essigester und Hexan erhält man 8-Fluor-3-(3-äthoxy-3-methyl-1-butinyl)-4,5-dihydro-5-methyl-6H-imidazo[1,5-a][1,4]benzodiazepin-6-on vom Schmelzpunkt 136-138 ° C.

Beispiel 58

a) 67.8 g (190 mMol) 8-Fluor-4,5-dihydro-3-jod-5-methyl-6H-imidazo[1,5-a][1,4][benzodiazepin-6-on werden mit 23.4 g (238 mMol) Aethinyl-trimethylsilan, 300 mg Bis-(triphenylphosphin)-palladium(II)-dichlorid und 70 mg Kupfer(I)-jodid während 8 Stunden in 280 ml Diäthylamin und 25 ml Aethylenchlorid unter Rückfluss zum Sieden erhitzt. Man dampft das Reaktionsgemisch ein und Chromatographiert den Rückstand an Keiselgel unter Eluieren mit Chloroform/Essigester (3/1). Durch Umkristallisieren aus Essigester erhält man 8-Fluor-4-5-dihydro-5-methyl-3-[(trimethylsilyl)äthinyl]-6H-imidazo[1,5-a][1,4]-benzodiazepin-6-on vom Schmelzpunkt 184-185 ° C.

b) In Analogie zu Biespiel 44b) erhält man aus obiger Verbindung 3-Aethinyl-8-fluor-4,5-dihydro-5-methyl-6H-imidazo[1,5-a][1,4]benzodiazepin-6-on (vgl. Beispiel 9).

Beispiel 59

a) 4,0 g (15,43 mMol) 8-Fluor-5,6-dihydro-5-methyl-6-oxo-4H-imidazo[1,5-a][1,4]benzodiazepin-3-carboxaldehyd und 8,1 g (30,88 mMol) Triphenylphosphin werden in 150 ml Methylenchlorid bei Raumtemperatur gelöst. Danach wird mit einem Eisbad auf 0 ° C abgekühlt, und bei dieser Temperatur wird eine Lösung von 5,6 g (16,88 mMol) Tetrabrommethan in 80 ml Methylenchlorid dazugetropft. Danach wird noch 4 Stunden bei Raumtemperatur gerührt und anschliessend eingedampft. Die erhaltene Suspension wird auf 0 ° C abgekühlt, genutscht und im Vakuum getrocknet. Das Rohprodukt wird aus Aethanol umkristallisiert. Man erhält 3-(2,2-Dibromvinyl)-8-fluor-4,5-dihydro-5-methyl-6H-imidazo[1,5-a][1,4]-benzodiazepin-6-on-Hydrobromid, als weisse Kristalle vom Smp. >260 ° C (Zers.).

b) 3,4 g (6,85 mMol) obiger Verbindung werden in 100 ml Methanol gelöst. Dazu gibt man eine Lösung von 0,33 g (14,35 mMol) Natrium in 30 ml Methanol. Das Reaktionsgemisch wird danach 16 Stunden unter Rückfluss erhitzt und anschliessend im Vakuum eingedampft. Der Rückstand wird zwischen Wasser und Methylenchlorid verteilt, und die wässerige Phase wird mit Methylenchlorid extrahiert. Die vereinigten organischen Phasen werden mit Wasser gewaschen, über Magnesiumsulfat getrocknet, filtriert und eingedampft. Der Rückstand wird aus Essigester/Hexan umkristallisiert. Man erhält 3-(Bromäthinyl)-8-fluor-4,5-dihydro-5-methyl-6H-imidazo[1,5-a][1,4]benzodiazepin-6-on als weisse Kristalle vom Smp. 167 ° C.

Beispiel 60

6,78 g (20 mMol) 4,5-Dihydro-3-jod-5-methyl-6H-imidazo[1,5-a][1,4]benzodiazepin-6-on werden in einem Druckrohr mit 80 mg Bis-(triphenylphospin)-palladium(II)-dichlorid, 30 mg Kupfer(I)-jodid und ca. 2 ml Propin in 60 ml Diäthylamin über Nacht auf 100 ° C erhitzt. Das Reaktionsgemisch wird dann eingedampft, und der Rückstand wird an 300 g Kieselgel chromatographiert unter Eluieren mit Essigester. Durch Umkristallisieren des Rohproduktes aus Aethanol erhält man 4,5-Dihydro-5-methyl-3-(1-propinyl)-6H-imidazo[1,5-a][1,4]-benzodiazepin-6-on vom Schmelzpunkt 206-207 ° C.

Beispiel 61

6,78 g (20 mMol) 4,5-Dihydro-3-jod-5-methyl-6H-imidazo[1,5-a][1,4]benzodiazepin-6-on werden mit 1,75 g (25 mMol) 3-Butin-2-ol, 70 mg Bis-(triphenylphosphin)-palladium(II)-dichlorid und 15 mg Kupfer(I)-jodid in 50 ml Diäthylamin und 30 ml Aethylenchlorid während 7 Stunden unter Rückfluss zum Sieden erhitzt. Das Reaktionsgemisch wird eingedampft, und der Rückstand wird an Kieselgel chromatographiert unter Eluieren mit Essigester. Nach Umkristallisieren aus Essigester und Hexan erhält man 4,5-Dihydro-3-(3-hydroxy-1-butinyl)-5-methyl-6H-imidazo[1,5-a][1,4]benzodiazepin-6-on vom Schmelzpunkt 161-162 ° C.

Beispiel 62

2,92 g (10 mMol) 3-Brom-4,5-dihydro-5-methyl-6H-imidazo[1,5-a][1,4]benzodiazepin-6-on werden mit 70

mg Bis-(triphenylphosphin)-palladium(II)-dichlorid, 30 mg Kupfer(I)-jodid und 2,5 ml (20 mMol) 3,3-Dimethyl-1-butin in 30 ml Diäthylamin und 10 ml Aethylenchlorid in einem Druckrohr während 24 Stunden auf 80 ° C erhitzt. Das Reaktionsgemisch wird eingedampft, in Methylenchlorid aufgenommen und mit Wasser gewaschen. Man trocknet die organische Lösung über Magnesiumsulfat und filtriert sie durch ein Kieselgel-Polster. Durch Kristallisieren aus Hexan erhält man 3-(3,3-Dimethyl-1-butinyl)-4-dihydro-5-methyl-6H-imidazo[1,5-a][1,4]benzodiazepin-6-on vom Schmelzpunkt 148-150 ° C.

Beispiel 63

5,05 g (90 mMol) frisch pulverisiertes Kaliumhydroxid werden in 50 ml N,N-Dimethylformamid suspendiert und auf 3 ° C gekühlt. Man gibt nacheinander 6,0 g (22,5 mMol) 4,5-Dihydro-3-(3-hydroxy-1-propinyl)-5-methyl-6H-imidazo[1,5-a][1,4]benzodiazepin-6-on und 6,39 g (45 mMol) Methyljodid dazu, entfernt das Eisbad und rührt während 1 Stunde weiter. Das Reaktionsgemisch wird auf 200 ml Wasser gegossen, mit 4-normaler Salzsäure auf pH 7 angesäuert und fünfmal mit Methylenchlorid extrahiert. Man trocknet die vereinigten organischen Extrakte über Magnesiumsulfat, dampft sie ein und erhält nach Kristallisieren des Rückstandes aus Essigester 4,5-Dihydro-3-(3-methoxy-1-propinyl)-5-methyl-6H-imidazo[1,5-a][1,4]-benzodiazepin-6-on vom Schmelzpunkt 114-115 ° C.

Beispiel 64

4,58 g (80 mMol) frisch pulverisiertes Kaliumhydroxid werden in 65 ml Dimethylsulfoxid suspendiert und auf 5 ° C gekühlt. Man gibt nacheinander 5,67 g (19,2 mMol) 4,5-Dihydro-3-(3-hydroxy-3-methyl-1-butinyl)-5-methyl-6H-imidazo[1,5-a][1,4]benzodiazepin-6-on und 5,47 g (38,5 mMol) Methyljodid dazu. Man entfernt das Kühlbad und rührt 1 Stunde weiter. Das Reaktionsgemisch wird eingedampft und der Rückstand an Kieselgel chromatographiert unter Eluieren mit Methylenchlorid/Methanol 19,5/0,5. Nach Kristallisieren aus tert.-Butylmethyläther erhält man 4,5-Dihydro-3-(3-methoxy-3-methyl-1-butinyl)-5-methyl-6H-imidazo[1,5-a]-[1,4]benzodiazepin-6-on vom Schmelzpunkt 136-137 ° C.

Beispiel 65

a) 14,55 g (43 mMol) Aethyl 7-chlor-8-fluor-5,6-dihydro-5-methyl-6-oxo-4H-imidazo[1,5-a][1,4]-benzodiazepin-3-carboxylat werden mit 1,90 g (47,7 mMol) Natriumhydroxid in 80 ml Aethanol und 30 ml Wasser während 15 Minuten unter Rückfluss zum Sieden erhitzt. Das Reaktionsgemisch wird dann abgekühlt und mit 10,4 ml 4-normaler Salzsäure versetzt. Durch Nutschen der erhaltenen Suspension und Trocknen des Produktes erhält man 7-Chlor-8-fluor-5,6-dihydro-6-oxo-4H-imidazo[1,5-a][1,4]-benzodiazepin-3-carbonsäure vom Zersetzungspunkt 260 ° C.

b) 11,64 g (37,5 mMol) 7-Chlor-8-fluor-5,6-dihydro-5-methyl-6-oxo-4H-imidazo[1,5-a][1,4]benzodiazepin-3-carbonsäure werden in einem Metallbad bis zum Ende der $CO_2$-Abspaltung auf 285 ° C erhitzt. Die Schmelze wird auf 50 ml Aethanol gegossen, und das ausgefallene Produkt wird abgenutscht und mit Aethanol gewaschen. Nach Trocknen erhält man 7-Chlor-8-fluor-4,5-dihydro-5-methyl-6H-imidazo[1,5-a]-[1,4]benzodiazepin-6-on vom Schmelzpunkt 234-235 ° C.

c) 6,08 g (22,8 mMol) 7-Chlor-8-fluor-4,5-dihydro-5-methyl-6H-imidazo[1,5-a][1,4]benzodiazepin-6-on werden während 4 Stunden mit 20 g (79,5 mMol) Jod in 50 ml N,N-Dimethylformamid bei 100 ° C gerührt. Das Reaktionsgemisch wird eingedampft, der Rückstand in Methylenchlorid und Wasser aufgenommen, mit Natriumthiosulfat geklärt und mit Natriumbicarbonat neutralisiert. Die wässrige Phase wird abgetrennt und viermal mit Methylenchlorid extrahiert. Man trocknet die vereinigten organischen Phasen über Magnesiumsulfat, dampft sie ein und chromatographiert den Rückstand an Kieselgel unter Eluieren mit Essigester/Hexan (1:1). Man erhält 7-Chlor-8-fluor-4,5-dihydro-3-jod-5-methyl-6H-imidazo[1,5-a][1,4]-benzodiazepin-6-on vom Schmelzpunkt 218-219 ° C.

d) 4,40 g (11,2 mMol) 7-Chlor-8-fluor-4,5-dihydro-3-jod-5-methyl-6H-imidazo[1,5-a][1,4]benzodiazepin-6-on werden mit 1,4 g (17 mMol) 2-Methyl-3-butin-2-ol, 70 mg Bis-(triphenylphosphin)-palladium(II)-dichlorid und 20 mg Kupfer(I)-jodid in 30 ml Diäthylamin während 1,5 Stunden unter Rückfluss zum Sieden erhitzt. Das Reaktionsgemisch wird eingedampft, der Rückstand in Methylenchlorid und Methanol gelöst und mit Aktivkohle entfärbt. Durch Kristallisieren aus Methanol und Wasser erhält man 7-Chlor-8-fluor-4,5-dihydro-3-(3-hydroxy-3-methyl-1-butinyl)-5-methyl-6H-imidazo[1,5-a][1,4]benzodiazepin-6-on vom Schmelzpunkt 208-209 ° C.

Beispiel 66

a) 25 g (85 mMol) Aethyl 5,6-dihydro-5,7-dimethyl-6-oxo-4H-imidazo[1,5-a][1,4]benzodiazepin-3-carboxylat werden mit 3,40 g (85 mMol) Natriumhydroxid in 200 ml Aethanol und 15 ml Wasser während 1 Stunde unter Rückfluss zum Sieden erhitzt. Nach Eindampfen des Aethanols wird mit Wasser verdünnt und mit 21 ml 4-normaler Salzsäure angesäuert. Man nutscht die erhaltene Suspension ab und wäscht sie mit Wasser. Nach Trocknen des Nutschkuchens erhält man 5,6-Dihydro-5,7-dimethyl-6-oxo-4H-imidazo[1,5-a][1,4]benzodiazepin-3-carbonsäure vom Zersetzungspunkt 274-275 °C.

b) 22,20 g (81,8 mMol) 5,6-Dihydro-5,7-dimethyl-6-oxo-4H-imidazo[1,5-a][1,4]benzodiazepin-3-carbonsäure werden bis zur Beendigung der $CO_2$-Abspaltung im Metallbad auf 290-300 °C erhitzt. Die Schmelze wird in Methylenchlorid und Aethanol gelöst und die Lösung konzentriert bis kein Methylenchlorid mehr überdestilliert. Aus dieser Lösung kristallisiert das 4,5-Dihydro-5,7-dimethyl-6H-imidazo[1,5-a][1,4]benzodiazepin-6-on vom Schmelzpunkt 224-225 °C.

c) 15,85 g (69,7 mMol) 4,5-Dihydro-5,7-dimethyl-6H-imidazo[1,5-a][1,4]benzodiazepin-6-on werden mit 67 g (264 mMol) Jod in 100 ml N,N-Dimethylformamid während 2,5 Stunden auf 95 °C erhitzt. Das Reaktionsgemisch wird dann auf 450 ml Wasser gegossen, mit Methylenchlorid versetzt mit Natriumthiosulfat entfärbt und mit Natriumbicarbonat neutralisiert. Die wässrige Phase wird abgetrennt und sechsmal mit Methylenchlorid extrahiert. Man wäscht die vereinigten organischen Phasen dreimal mit Wasser, trocknet sie über Magnesiumsulfat und dampft sie ein. Durch Chromatographieren des Rückstandes an Kieselgel unter Eluieren mit Essigester und Umkristalisieren aus Essigester und Hexan erhält man 4,5-Dihydro-3-jod-5,7-dimethyl-6H-imidazo[1,5-a][1,4]benzodiazepin-6-on vom Schmelzpunkt 106-108 °C.

d) 5 g (14,2 mMol) 4,5-Dihydro-3-jod-5,7-dimethyl-6H-imidazo[1,5-a][1,4-benzodiazepin-6-on werden mit 1,50 g (17,8 mMol) 2-Methyl-3-butin-2-ol, 70 mg Bis-(triphenylphosphin)-palladium(II)-dichlorid und 20 mg Kupfer(I)-jodid in 40 ml Diäthylamin während 4,5 Stunden unter Rückfluss zum Sieden erhitzt. Das Reaktionsgemisch wird dann eingedampft, und der Rückstand wird an Kieselgel chromatographiert unter Eluieren mit Essigester. Durch Umkristallisieren aus Essigester erhält man 4,5-Dihydro-3-(3-hydroxy-3-methyl-1-butinyl)-5,7-dimethyl-6H-imidazo[1,5-a][1,4]benzodiazepin-6-on vom Schmelzpunkt 165-167 °C.

Beispiel 67

5,20 g (14 mMol) 7-Chlor-4,5-dihydro-3-jod-5-methyl-6H-imidazo[1,5-a][1,4]benzodiazepin-6-on werden mit 1,04 g (18,5 mMol) Propargylalkohol, 70 mg Bis-(triphenylphosphin)-palladium(II)-dichlorid und 10 mg Kupfer(I)-jodid in 35 ml Diäthylamin während 4 Stunden unter Rückfluss zum Sieden erhitzt. Das Reaktionsgemisch wird eingedampft, und der Rückstand wird in Methylenchlorid aufgeschlämmt. Man nutscht die Suspension ab und wäscht den Nutschkuchen mit Essigester. Nach zwei aufeinanderfolgenden Umkristallisationen aus Aethanol bzw. N,N-Dimethylformamid erhält man 7-Chlor-4,5-dihydro-3-(3-hydroxy-1-propinyl)-5-methyl-6H-imidazo[1,5-a][1,4]benzodiazepin-6-on vom Schmelzpunkt 250-252 °C.

Beispiel 68

2,0 g (5 mMol) (S)-8-chlor-11,12,13,13a-tetrahydro-1-jod-9H-imidazo[1,5-a]pyrrolo[2,1-c][1,4]-benzodiazepin-9-on werden mit 0,73 g (5,5 mMol) 4-Methoxyphenylacetylen, 50 mg Bis-(triphenylphosphin)-palladium(II)-dichlorid und 50 mg Kupfer(I)-jodid während 6 Stunden in 25 ml Diäthylamin und 25 ml N,N-Dimethylformamid unter Rückfluss zum Sieden erhitzt. Man dampft das Reaktionsgemisch ein und chromatographiert den Rückstand an Kieselgel unter Eluieren mit Methylenchlorid/Methanol (99/1). Nach Umkristallisieren aus Methylenchlorid und Essigester erhält man (S)-8-Chlor-11,12,13,13a-tetrahydro-1-[(p-methoxyphenyl)äthinyl]-9H-imidazo[1,5-a]pyrrolo[2,1-c][1,4]benzodiazepin-9-on vom Schmelzpunkt 186-187 °C.

Beispiel 69

2,0 g (5 mMol) (S)-8-chlor-11,12,13,13a-tetrahydro-1-jod-9H-imidazo[1,5-a]pyrrolo[2,1-c][1,4]-benzodiazepin-9-on werden mit 0,73 g (5,6 mMol) 3-Methoxyphenylacetylen, 65 mg Bis-(triphenylphosphin)-palladium(II)-dichlorid und 65 mg Kupfer(I)-jodid während 9 Stunden in 20 ml Diäthylamin und 20 ml N,N-Dimethylformamid unter Rückfluss zum Sieden erhitzt. Das Reaktionsgemisch wird eingedampft, und der Rückstand wird an Kieselgel chromatographiert unter Eluieren mit Methylenchlorid/Methanol (99/1). Durch Kristallisieren aus Methylenchlorid und Essigester erhält man (S)-8-Chlor-11,12,13,13a-tetrahydro-1-[(m-methoxyphenyl)äthinyl]-9H-imidazo[1,5-a]pyrrolo[2,1-c][1,4]benzodiazepin-9-on vom Schmelzpunkt 204-205 °C.

Beispiel 70

2,0 g (5 mMol) (S)-8-chlor-11,12,13,13a-tetrahydro-1-jod-9H-imidazo[1,5-a]pyrrolo[2,1-c][1,4]-benzodiazepin-9-on werden mit 0,75 g (5,5 mMol) 3-Chlorophenylacetylen, 65 mg Bis-(triphenylphosphin)-palladium(II)-dichlorid und 65 mg Kupfer(I)-jodid während 9 Stunden in 20 ml Diäthylamin und 20 ml N,N-Dimethylformamid unter Rückfluss zum Sieden erhitzt. Das Reaktionsgemisch wird eingedampft, und der Rückstand wird an Kieselgel chromatographiert unter Eluieren mit Methylenchlorid/Methanol (99,2/0,8). Durch Kristallisieren aus Methylenchlorid und Essigester erhält man (S)-8-Chlor-1-[(m-chlorphenyl)äthinyl]-11,12,13,13a-tetrahydro-9H-imidazo[1,5-a]pyrrolo[2,1-c][1,4]benzodiazepin-9-on vom Schmelzpunkt 207-209 ° C.

Beispiel 71

1,0 g (4,9 mMol) (S)-8-Chlor-11,12,13,13a-tetrahydro-1-jod-9H-imidazo[1,5-a]pyrrolo[2,1-c][1,4]-benzodiazepin-9-on werden mit 0,71 g (5,2 mMol) 2-Chlorphenylacetylen, 65 mg Bis-(triphenylphosphin)-palladium(II)-dichlorid und 65 mg Kupfer(I)-jodid während 3,5 Stunden in 25 ml Diäthylamin und 25 ml N,N-Dimethylformamid unter Rückfluss zum Sieden erhitzt. Man dampft das Reaktionsgemisch ein und chromatographiert den Rückstand an Kieselgel unter Eluieren mit Methylenchlorid/Methanol (99/1). Durch Umkristallisieren aus Methylenchlorid und Essigester erhält man (S)-8-Chlor-1-[(o-chlorphenyl)äthinyl]-11,12,13,13a-tetrahydro-9H-imidazo[1,5-a]pyrrolo-[2,1-c][1,4]benzodiazepin-9-on vom Schmelzpunkt 217-219 ° C.

Beispiel 72

a) 44,26 g (185 mMol) (S)-11,12,12,13a-Tetrahydro-9H-imidazo[1,5-a]pyrrolo[2,1-c][1,4]benzodiazepin-9-on werden mit 162,8 g (645 mMol) Jod in 300 ml N,N-Dimethylformamid während 4 Stunden bei 100 ° C gerührt. Das Reaktionsgemisch wird eingedampft, der Rückstand wird in Wasser und Methylenchlorid aufgenommen, mit Natriumthiosulfat entfärbt und mit Natriumbicarbonat neutralisiert. Die wässrige Phase wird abgetrennt und viermal mit Methylenchlorid extrahiert. Die vereinigten organischen Phasen werden über Magnesiumsulfat getrocknet und eingedampft. Durch Chromatographieren des Rohproduktes an Kieselgel unter Eluieren mit Essigester und anschliessendes Umkristallisieren aus Methylenchlorid und Methanol erhält man (S)-11,12,13,13a-Tetrahydro-1-jod-9H-imidazo[1,5-a]pyrrolo[2,1-c][1,4]-benzodiazepin-9-on vom Schmelzpunkt 222-223 ° C.

b) 3,65 (10 mMol) (S)-11,12,13,13a-Tetrahydro-1-jod-9H-imidazo[1,5-a]pyrrolo[2,1-c][1,4]benzodiazepin-9-on werden mit 1,26 g (15 mMol) 2-Methyl-3-butin-2-ol, 70 mg Bis-(triphenylphosphin)-palladium(II)-dichlorid und 20 mg Kupfer(I)-jodid in 30 ml Diäthylamin während 1,5 Stunden unter Rückfluss zum Sieden erhitzt. Man dampft das Reaktionsgemisch ein und chromatographiert den Rückstand an Kieselgel unter Eluieren mit Essigester. Durch Umkristallisieren des Rohproduktes aus Methanol und Wasser erhält man (S)-11,12, 13,13a-Tetrahydro-1-(3-hydroxy-3-methyl-1-butinyl)-9H-imidazo[1,5-a]-pyrrolo[2,1-c][1,4]benzodiazepin-9-on vom Schmelzpunkt 128 ° C.

Beispiel 73

a) 27 g 5,6-Dihydro-5-methyl-4-oxo-4H-imidazo[1,5-a]thieno[3,2-f][1,4]diazepin-7-carbonsäure werden in einem mit Argon gefüllten Kolben in ein auf 280 ° C vorgewärmtes Heizbad getaucht. Die Substanz schmilzt unter Decarboxylierung. Nach Beendigung der Kohlendioxid-Entwicklung (ca. 7 bis 8 Min.) wird der Kolben leicht abgekühlt. Das Reaktionsgemisch wird, noch bevor es erstarrt, mit 250 ml Trichlormethan, vermischt. Die Lösung wird im Vakuum eingedampft, und der Rückstand wird aus Essigsäureäthylester/Diisopropyläther umkristallisiert. Man erhält 5,6-Dihydro-5-methyl-4H-imidazo[1,5-a]thieno[3,2-f][1,4]diazepin-4-on vom Schmelzpunkt 160-163 ° C. Nach Umkristallisieren aus Essigsäureäthylester beträgt der Schmelzpunkt 163-164 ° C.

b) 22 g 5,6-Dihydro-5-methyl-4H-imidazo[1,5-a]thieno[3,2-f][1,4]diazepin-4-on werden in 330 ml Dimethylformamid gelöst und mit 50,6 g Jod versetzt. Das Gemisch wird 24 Stunden bei Raumtemperatur und dann 18 Stunden bei 30 ° C gerührt. Es wird sodann in 9 l gesättigte wässrige Natriumhydrogencarbonatlösung gegossen. Diese Lösung wird viermal mit Trichlormethan extrahiert. Die Trichlormethan-Lösungen werden nacheinander mit wässriger Natriumthiosulfatlösung und mit wässriger Natriumchloridlösung gewaschen, dann über Natriumsulfat getrocknet und im Vakuum eingedampft. Der Rückstand wird durch 2,5 kg Kieselgel chromatographiert. Zunächst wird mit Trichlormethan eine Verunreinigung eluiert. Dann wird das Hauptprodukt mit Trichlormethan/Aethanol-Gemischen 199:1 und 99:1 eluiert. Diese Eluate werden im Vakuum eingedampft, und der Rückstand wird aus Essigsäureäthylester/Diisopropyläther,

dann aus Essigsäureäthylester mehrmals kristallisiert. Man erhält 5,6-Dihydro-7-jod-5-methyl-4H-imidazo-[1,5-a]thieno[3,2-f][1,4]diazepin-4-on vom Schmelzpunkt 175-177 ° C.

c) 5,52 g 5,6-Dihydro-7-jod-5-methyl-4H-imidazo[1,5-a]thieno[3,2-f][1,4]diazepin-4-on werden in 30 ml Diäthylamin mit 1,92 ml 2-Methyl-3-butin-2-ol, 75 mg Bis-(triphenylphosphin)-palladium(II)-chlorid und 13 mg Kupfer(I)-jodid versetzt. Man rührt 2 Stunden bei Rückflusstemperatur. Sodann wird das Reaktionsgemisch im Vakuum eingedampft. Der Rückstand wird in Dichlormethan gelöst, und diese Lösung wird zweimal mit Wasser gewaschen. Nach Trocknen über Natriumsulfat wird diese Lösung durch 300 g Kieselgel chromatographiert. Mit Dichlormethan/Aethanol-Gemischen 99:1, 98:2 und 97:3 wird zunächst nicht umgesetztes Ausgangsmaterial zurückgewonnen. Sodann wird mit Dichlormethan/Aethanol-Gemischen 96:4 und 95:5 eine ölige Substanz eluiert, welche aus Essigsäureäthylester/Diäthyläther kristallisiert. Man erhält 5,6-Dihydro-7-(3-hydroxy-3-methyl-1-butinyl)-5-methyl-4H-imidazo[1,5-a]thieno[3,2-f]-[1,4]diazepin-4-on vom Schmelzpunkt 180-181 ° C.

Auf analoge Weise erhält man folgende Endprodukte:

d) (S)-10,11,12,12a-Tetrahydro-1-(3-hydroxy-3-methyl-1-butinyl)-8H-imidazo[5,1-c]pyrrolo[1,2-a]thieno-[2,3-e][1,4]diazepin-8-on vom Schmelzpunkt 145-147 ° C;

e) 4,5-Dihydro-3-(3-hydroxy-3-methyl-1-butinyl)-5-methyl-6H-imidazo[1,5-a]thieno[2,3-f][1,4]diazepin-6-on vom Schmelzpunkt 198-200 ° C;

f) (S)-10,11,12,12a-Tetrahydro-1-(3-hydroxy-3-methyl-1-butinyl)-8H-imidazo[5,1-c]pyrrolo[1,2-a]thieno[3,2-e][1,4]diazepin-8-on vom Schmelzpunkt 142-144 ° C.

## Beispiel 74

a) 5,1 g 5,6-Dihydro-7-jod-5-methyl-4H-imidazo[1,5-a]thieno[3,2-f][1,4]diazepin-4-on (hergestellt gemäss Beispiel 73) werden in 47 ml Diäthylamin und 20 ml Dimethylformamid gelöst. Dann werden 1,75 ml Phenylacetylen, 105 mg Bis-(triphenylphosphin)-palladium(II)chlorid und 17 mg Kupfer(I)jodid zugegeben. Das Gemisch wird 2 Stunden bei Rückflusstemperatur gerührt und sodann im Vakuum eingeengt. Der Rückstand wird mit 200 ml Dichlormethan verdünnt und zweimal mit Wasser gewaschen. Die Waschwasser werden zweimal mit Dichlormethan extrahiert. Die vereinigten Dichlormethan-Lösungen werden über Natriumsulfat getrocknet und durch 300 g Kieselgel chromatographiert. Zunächst wird mit Trichlormethan und Trichlormethan/Aethanol-Gemischen 199:1 und 99:1 eluiert. Sodann wird mit einem Trichlormethan/Aethanol-Gemisch 97:3 eine ölige Substanz eluiert, welche aus Isopropanol/Wasser kristallisiert werden kann. Nach mehrmaligem Umkristallisieren aus Isopropanol/Wasser erhält man 5,6-Dihydro-5-methyl-7-(phenyläthinyl)-4H-imidazo[1,5-a]thieno[3,2-f][1,4]-diazepin-4-on vom Schmelzpunkt 143-144 ° C.

Auf analoge Weise erhält man folgende Endprodukte:

b) (S)-10,11,12,12a-Tetrahydro-1-(phenyläthinyl)-8H-imidazo[5,1-c]pyrrolo[1,2-a]thieno[2,3-e][1,4]-diazepin-8-on vom Schmelzpunkt 159-160 ° C;

c) 4,5-Dihydro-5-methyl-3-(phenyläthinyl)-6H-imidazo[1,5-a]thieno[2,3-f][1,4]diazepin-6-on vom Schmelzpunkt 200-202 ° C;

d) (S)-10,11,12,12a-Tetrahydro-1-(phenyläthinyl)-8H-imidazo[5,1-c]pyrrolo[1,2-a]thieno[3,2-e][1,4]-diazepin-8-on vom Schmelzpunkt 218-220 ° C.

## Beispiel A

Es werden in üblicher Weise Tabletten folgender Zusammensetzung hergestellt:

| | mg/Tablette |
|---|---|
| 7-Chlor-4,5-dihydro-3-(3-hydroxy-3-methyl-1-butinyl)-5-methyl-6H-imidazo-[1,5-a][1,4]benzodiazepin-6-on | 0,2 |
| Milchzucker | 140 |
| Maisstärke | 50,8 |
| Polyvinylpyrrolidin | 8 |
| Magnesiumstearat | 1 |
| Tablettengewicht | 200 |

Beispiel B

Es werden in üblicher Weise Kapseln folgender Zusammensetzung hergestellt:

| | mg/Kapsel |
|---|---|
| 7-Chlor-4,5-dihydro-3-(3-hydroxy-3-methyl-1-butinyl)-5-methyl-6H-imidazo-[1,5-a][1,4]benzodiazepin-6-on | 0,5 |
| Milchzucker | 40 |
| Maisstärke | 8 |
| Talk | 1 |
| Magnesiumstearat | 0,5 |
| Kapselfüllgewicht | 50 |

Beispiel C

Es werden Injektionslösungen folgender Zusammensetzung hergestellt:

| | |
|---|---|
| 7-Chlor-4,5-dihydro-5-methyl-3-(1-propinyl)-6H-imidazo[1,5-a][1,4]benzodiazepin-6-on | 0,1 mg |
| Natriumchlorid | 45,0 mg |
| SESQUESTREN $Na_2$ | 0,5 mg |
| Essigsäure p.a. | 0,5 mg |
| NaOH 1N ad pH 4,5 | q.s. |
| Wasser zu Injektionszwecken q.s. ad | 5,0 ml |

## Patentansprüche

**Patentansprüche für folgende Vertragsstaaten : AT, BE, CH, DE, FR, GB, IT, LI, LU, NL, SE**

**1.** Verbindungen der allgemeinen Formel

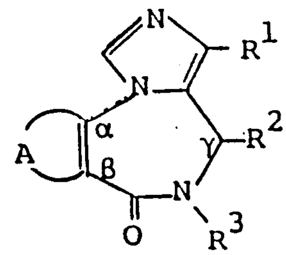

I

worin A zusammen mit den beiden mit α und β bezeichneten Kohlenstoffatomen eine der Gruppen

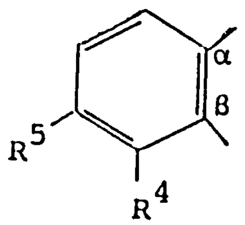

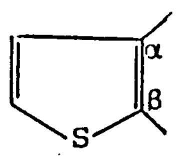

und

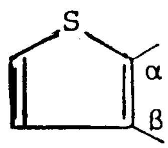

(a) (b) (c)

$R^1$ eine der Gruppen

$-CH{=}CH{-}R^6$ und $-C{\equiv}C{-}R^6$,

(d) (e)

$R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl und $R^6$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeuten, wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten, und wobei die in Gruppe (d) vorhandene Doppelbindung die E- und/oder Z-Konfiguration aufweist, wenn $R^6$ von Wasserstoff verschieden ist, und die mit "nieder" bezeichneten Reste bis zu 7 Kohlenstoffatome besitzen.

**2.** Verbindungen gemäss Anspruch 1, worin $R^1$ die Gruppe $-CH{=}CH{-}R^6$ (d) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl, Phenyl oder Halogen bedeuten, oder $R^1$ die Gruppe $-C{\equiv}C{-}R^6$ (e) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl oder Phenyl bedeuten.

**3.** Verbindungen gemäss Anspruch 1 oder 2, worin $R^1$ die Gruppe $-C{\equiv}C{-}R^6$ (e) bedeutet.

**4.** Verbindungen gemäss Anspruch 3, worin $R^6$ Wasserstoff, niederes Alkyl, niederes Hydroxyalkyl, niederes Alkoxyalkyl, ($C_3$-$C_7$)-Cycloalkyl, Hydroxy-($C_4$-$C_7$)-Cycloalkyl, niederes Alkoxy-($C_4$-$C_7$)-Cycloalkyl, ($C_3$-$C_7$)-Cycloalkyl-niederes Alkyl, ($C_3$-$C_7$)-Cycloalkyl-niederes Hydroxyalkyl oder ($C_3$-$C_7$)-

Cycloalkyl-niederes Alkoxyalkyl bedeutet.

**5.** Verbindungen gemäss Anspruch 4, worin $R^6$ Wasserstoff, niederes Alkyl, niederes 1-Hydroxyalkyl, niederes 1-Alkoxyalkyl, ($C_3$-$C_7$)-Cycloalkyl, 1-Hydroxy-($C_4$-$C_7$)-cycloalkyl, 1-(niederes Alkoxy)-($C_4$-$C_7$)-cycloalkyl oder 1-[($C_3$-$C_7$)-Cycloalkyl]-niederes 1-Hydroxyalkyl, insbesondere niederes Alkyl, niederes 1-Hydroxyalkyl oder ($C_3$-$C_7$)-Cycloalkyl bedeutet.

**6.** Verbindungen gemäss einem der Ansprüche 1 bis 5, worin $R^2$ Wasserstoff und $R^3$ Methyl bedeuten oder worin $R^2$ und $R^3$ zusammen Dimethylen oder Trimethylen bedeuten und das mit $\gamma$ bezeichnete Kohlenstoffatom die (S)-Konfiguration aufweist.

**7.** Verbindungen gemäss einem der Ansprüche 1 bis 6, worin A einen Rest der Formel (a) und eines von $R^4$ und $R^5$ Wasserstoff und das andere Wasserstoff oder Halogen bedeuten.

**8.** Verbindungen gemäss Anspruch 7, worin $R^4$ und $R^5$ beide Wasserstoff oder $R^4$ Wasserstoff und $R^5$ oder $R^4$ Chlor oder Brom und $R^5$ Wasserstoff bedeuten.

**9.** 7-Chlor-4,5-dihydro-5-methyl-3-(1-propinyl)-6H-imidazo[1,5-a][1,4]benzodiazepin-6-on.

**10.** 7-Chlor-4,5-dihydro-3-(3-hydroxy-3-methyl-1-butinyl)-5-methyl-6H-imidazo[1,5-a][1,4]benzodiazepin-6-on.

**11.** 7-Brom-4,5-dihydro-3-(3-hydroxy-3-methyl-1-butinyl)-5-methyl-6H-imidazo[1,5-a][1,4]benzodiazepin-6-on.

**12.** 7-Chlor-4,5-dihydro-3-(3-hydroxy-1-butinyl)-5-methyl-6H-imidazo[1,5-a][1,4]benzodiazepin-6-on.

**13.** 4,5-Dihydro-5-methyl-3-(1-propinyl)-6H-imidazo[1,5-a] [1,4]benzodiazepin-6-on.

**14.** 7-Chlor-4,5-dihydro-5-methyl-3-(3-methyl-1-butinyl)-6H-imidazo[1,5-a][1,4]benzodiazepin-6-on.

**15.** 7-Chlor-4,5-dihydro-3-(3-hydroxy-1-propinyl)-5-methyl-6H-imidazo[1,5-a][1,4]benzodiazepin-6-on.

**16.** 7-Chlor-3-(cyclopropyläthinyl)-4,5-dihydro-5-methyl-6H-imidazo[1,5-a][1,4]benzodiazepin-6-on.

**17.** Verbindungen der allgemeinen Formel

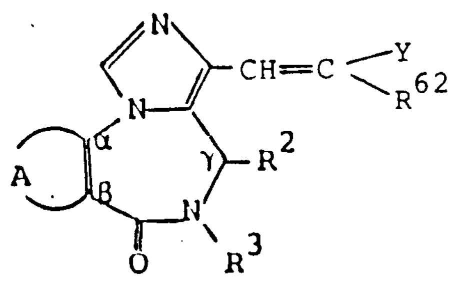

IVa

worin Y und $R^{62}$ je Halogen bedeuten, und A, $R^2$ und $R^3$ die in Anspruch 1 angegebene Bedeutung haben.

**18.** Verbindungen der allgemeinen Formel

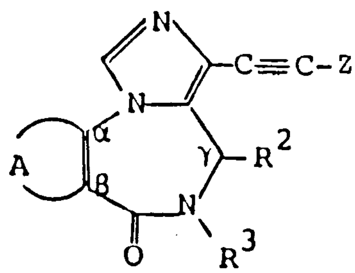

VIII

worin Z eine Schutzgruppe bedeutet, und A, $R^2$ und $R^3$ die in Anspruch 1 angegebene Bedeutung haben.

**19.** Verbindungen der allgemeinen Formel

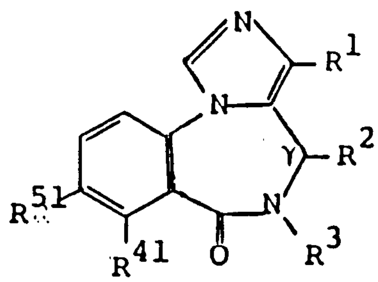

IX

worin eines von $R^{41}$ und $R^{51}$ Amino und das andere Wasserstoff, Halogen, Trifluormethyl oder niederes Alkyl bedeutet, und $R^1$, $R^2$, $R^3$ und "nieder" die in Anspruch 1 angegebene Bedeutung haben.

**20.** Verbindungen gemäss einem der Ansprüche 1 bis 16 zur Anwendung als therapeutische Wirkstoffe.

**21.** Verbindungen gemäss einem der Ansprüche 1 bis 16 zur Anwendung als therapeutische Wirkstoffe, welche antikonvulsiv, anxiolytisch, muskelrelaxierend und sedativ-hypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren.

**22.** Verfahren zur Herstellung von Verbindungen gemäss einem der Ansprüche 1 bis 16, dadurch gekennzeichnet, dass man
a) eine Verbindung der allgemeinen Formel

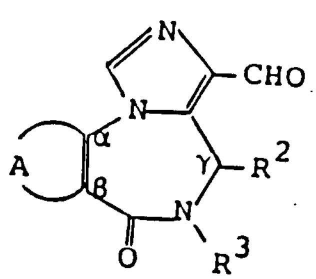

II

worin A, $R^2$ und $R^3$ die in Anspruch 1 angegebene Bedeutung besitzen,
mit einer Verbindung der allgemeinen Formel

$(Ar)_3P=CH\text{-}R^{61}$ III

worin $R^{61}$ Wasserstoff, Halogen, einen monocyclischen aromatischen Kohlenwasserstoffrest, welcher

durch niederes Alkyl, niederes Alkoxy oder Halogen substituiert sein kann, oder eine gegebenenfalls durch niederes Alkoxy, ($C_3$-$C_7$)-Cycloalkyl oder Oxo mono- oder disubstituierte gesättigte niedere Kohlenwasserstoffgruppe und Ar einen monocyclischen aromatischen Kohlenwasserstoffrest, welcher durch niederes Alkyl, niederes Alkoxy oder Halogen substituiert sein kann, bedeuten,
umsetzt; oder
b) eine Verbindung der allgemeinen Formel

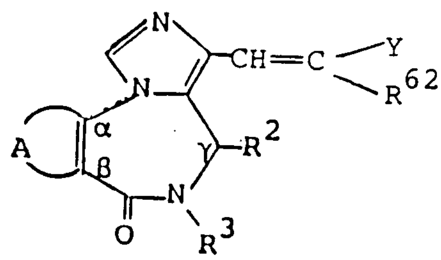

IV

worin $R^{62}$ Wasserstoff oder Halogen und Y Halogen bedeuten, und A, $R^2$ und $R^3$ die in Anspruch 1 angegebene Bedeutung besitzen,
dehydrohalogeniert; oder
c) eine Verbindung der Formel I, worin $R^1$ die Gruppe (e) und $R^6$ Wasserstoff bedeuten, mit einem einen gegebenenfalls durch Hydroxy, niederes Alkoxy, ($C_3$-$C_7$)-Cycloalkyl oder Oxo mono- oder disubstituierten gesättigten niederen Kohlenwasserstoff- oder einen monocyclischen aromatischen Kohlenwasserstoffrest, welcher durch niederes Alkyl, niederes Alkoxy oder Halogen substituiert sein kann, oder Halogen liefernden Mittel behandelt; oder
d) eine Verbindung der allgemeinen Formel

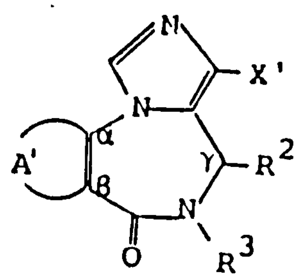

VI

worin $R^2$ und $R^3$ die in Anspruch 1 angegebene Bedeutung besitzen und X' Brom oder Jod und A' einen Rest der Formel (a), (b) oder (c) bedeutet, wobei aber im Falle, dass A' einen Rest der Formel (a) und $R^4$ und/oder $R^5$ Halogen bedeuten, dieses Halogen Fluor oder Chlor ist, wenn X Brom bedeutet, bzw. Fluor, Chlor oder Brom ist, wenn X Jod bedeutet,
mit einer Verbindung der allgmeinen Formel

$HC \equiv C-R^{64}$ VII

worin $R^{64}$ Wasserstoff, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeutet,
umsetzt; oder
e) aus einer Verbindung der allgemeinen Formel

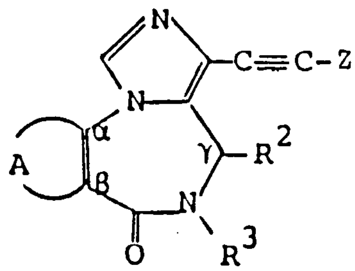

VIII

worin $R^2$, $R^3$ und A die in Anspruch 1 angegebene Bedeutung besitzen und Z eine Schutzgruppe bedeutet,
die Schutzgruppe abspaltet; oder
f) in einer Verbindung der allgemeinen Formel

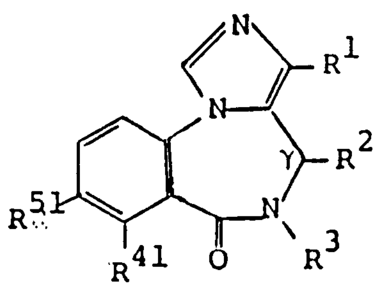

IX

worin $R^1$, $R^2$ und $R^3$ die in Anspruch 1 angegebene Bedeutung besitzen und eines von $R^{41}$ und $R^{51}$ Amino und das andere Wasserstoff, Halogen, Trifluormethyl oder niederes Alkyl bedeutet, die Aminogruppe durch ein Wasserstoff- oder Halogenatom ersetzt; oder
g) eine Verbindung der Formel I, worin $R^1$ einen Rest der Formel (e) bedeutet und worin im Falle, dass A einen Rest der Formel (a) bedeutet, $R^4$ und/oder $R^5$ nicht Jod bedeutet, zur entsprechenden Verbindung der Formel I, worin $R^1$ einen Rest der Formel (d) bedeutet, reduziert; oder
h) eine Verbindung der Formel I, worin $R^1$ einen Rest der Formel (d) oder (e) und $R^6$ eine durch Hydroxy substituierte gesättigte niedere Kohlenwasserstoffgruppe bedeuten, mit einem einen niederen Alkylrest liefernden Mittel behandelt; oder
i) einer Verbindung der Formel I, worin $R^1$ die Gruppe (d) oder (e) und $R^6$ eine durch Oxo substituierte gesättigte niedere Kohlenwasserstoffgruppe bedeuten, die Carbonylgruppe reduziert.

**23.** Arzneimittel, enthaltend eine Verbindung gemäss einem der Ansprüche 1 bis 16 und einen therapeutisch inerten Träger.

**24.** Arzneimittel gemäss Anspruch 23, welche antikonvulsiv, anxiolytisch, muskelrelaxierend und sedativhypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren.

**25.** Verwendung von Verbindungen gemäss einem der Ansprüche 1 bis 16 für die Herstellung von Arzneimitteln zur Anwendung bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

**Patentansprüche für folgenden Vertragsstaat : ES**

**1.** Verfahren zur Herstellung von Verbindungen der allgemeinen Formel

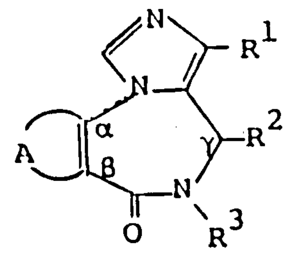

I

worin A zusammen mit den beiden mit $\alpha$ und $\beta$ bezeichneten Kohlenstoffatomen eine der Gruppen

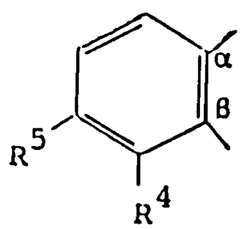

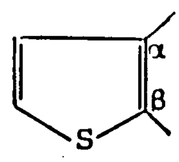

und

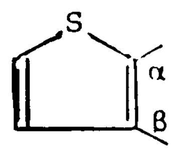

(a) (b) (c)

$R^1$ eine der Gruppen

$-CH{=}CH-R^6$ und $-C{\equiv}C-R^6$,

(d) (e)

$R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl und $R^6$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeuten, wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen oder Trimethylen bedeuten, und wobei die in Gruppe (d) vorhandene Doppelbindung die E- und/oder Z-Konfiguration aufweist, wenn $R^6$ von Wasserstoff verschieden ist, und die mit "nieder" bezeichneten Reste bis zu 7 Kohlenstoffatome besitzen

dadurch gekennzeichnet, dass man

a) eine Verbindung der allgemeinen Formel

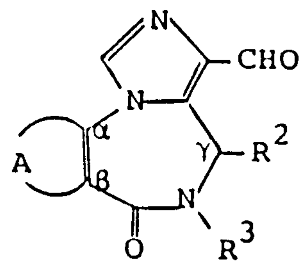

II

worin A, $R^2$ und $R^3$ obige Bedeutung besitzen,
mit einer Verbindung der allgemeinen Formel

$(Ar)_3P=CH-R^{61}$ III

worin $R^{61}$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch niederes Alkoxy, $(C_3-C_7)$-Cycloalkyl oder Oxo mono- oder disubstituierte gesättigte niedere Kohlenwasserstoffgruppe und Ar einen monocyclischen aromatischen Kohlenwasserstoffrest, welcher durch niederes Alkyl, niederes Alkoxy oder Halogen substituiert sein kann, bedeuten,
umsetzt; oder
b) eine Verbindung der allgemeinen Formel

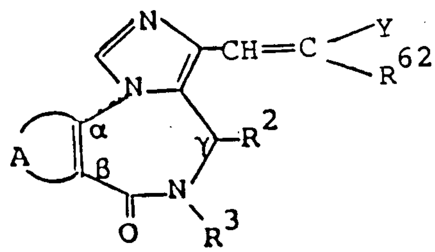

IV

worin $R^{62}$ Wasserstoff oder Halogen und Y Halogen bedeuten, und A, $R^2$ und $R^3$ obige Bedeutung besitzen,
dehydrohalogeniert; oder
c) eine Verbindung der Formel I, worin $R^1$ die Gruppe (e) und $R^6$ Wasserstoff bedeuten, mit einem einen gegebenenfalls durch Hydroxy, niederes Alkoxy, $(C_3-C_7)$-Cycloalkyl oder Oxo mono- oder disubstituierten gesättigten niederen Kohlenwasserstoff- oder einen monocyclischen aromatischen Kohlenwasserstoffrest, welcher durch niederes Alkyl, niederes Alkoxy oder Halogen substituiert sein kann, oder Halogen liefernden Mittel behandelt; oder
d) eine Verbindung der allgemeinen Formel

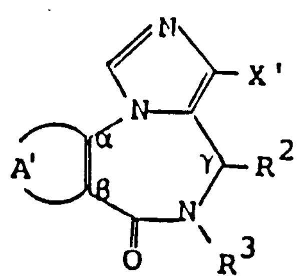

VI

worin $R^2$ und $R^3$ obige Bedeutung besitzen und X' Brom oder Jod und A' einen Rest der Formel (a), (b) oder (c) bedeutet, wobei aber im Falle, dass A' einen Rest der Formel (a) und $R^4$ und/oder $R^5$ Halogen bedeuten, dieses Halogen Fluor oder Chlor ist, wenn X Brom bedeutet, bzw. Fluor, Chlor

oder Brom ist, wenn X Jod bedeutet,
mit einer Verbindung der allgmeinen Formel

$HC\equiv C\text{-}R^{64}$ VII

worin $R^{64}$ Wasserstoff, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeutet,
umsetzt; oder
e) aus einer Verbindung der allgemeinen Formel

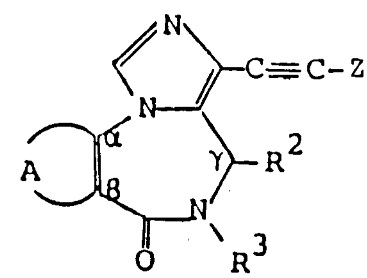

VIII

worin $R^2$, $R^3$ und A obige Bedeutung besitzen und Z eine Schutzgruppe bedeutet,
die Schutzgruppe abspaltet; oder
f) in einer Verbindung der allgemeinen Formel

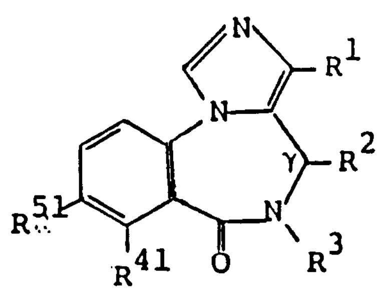

IX

worin $R^1$, $R^2$ $R^3$ und "nieder" obige Bedeutung besitzen und eines von $R^{41}$ und $R^{51}$ Amino und das andere Wasserstoff, Halogen, Trifluormethyl oder niederes Alkyl bedeutet,
die Aminogruppe durch ein Wasserstoff- oder Halogenatom ersetzt; oder
g) eine Verbindung der Formel I, worin $R^1$ einen Rest der Formel (e) bedeutet und worin im Falle, dass A einen Rest der Formel (a) bedeutet, $R^4$ und/oder $R^5$ nicht Jod bedeutet, zur entsprechenden Verbindung der Formel I, worin $R^1$ einen Rest der Formel (d) bedeutet, reduziert; oder
h) eine Verbindung der Formel I, worin $R^1$ einen Rest der Formel (d) oder (e) und $R^6$ eine durch Hydroxy substituierte gesättigte niedere Kohlenwasserstoffgruppe bedeuten, mit einem einen niederen Alkylrest liefernden Mittel behandelt; oder
i) einer Verbindung der Formel I, worin $R^1$ die Gruppe (d) oder (e) und $R^6$ eine durch Oxo substituierte gesättigte niedere Kohlenwasserstoffgruppe bedeuten, die Carbonylgruppe reduziert.

**2.** Verfahren gemäss Anspruch 1 zur Herstellung von Verbindungen der Formel I, worin $R^1$ die Gruppe $-CH=CH-R^6$ (d) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl, Phenyl oder Halogen bedeuten, oder $R^1$ die Gruppe $-C\equiv C-R^6$ (e) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl oder Phenyl bedeuten, dadurch gekennzeichnet, dass man eine der Verfahrensvarianten a) bis h) anwendet und in Verfahrensvariante a) eine Verbindung der Formel III, worin $R^{61}$ Wasserstoff, niederes Alkyl,

niederes Alkoxy-niederes Alkyl, ($C_3$-$C_7$)-Cycloalkyl, niederes Alkoxy-($C_4$-$C_7$)-cycloalkyl, ($C_3$-$C_7$)-Cycloalkyl-niederes Alkyl, Phenyl oder Halogen bedeutet, in Verfahrensvariante b) eine Verbindung der Formel IV, worin $R^{62}$ Wasserstoff bedeutet, in Verfahrensvariante c) ein einen niederen Alkyl-, einen niederen Hydroxyalkyl-, einen niederen Alkoxy-niederen Alkyl-, einen ($C_4$-$C_7$)-Cycloalkyl-, einen Hydroxy-($C_4$-$C_7$)-cycloalkyl-, einen niederen Alkoxy-($C_4$-$C_7$)-cycloalkyl-, einen ($C_3$-$C_7$)-Cycloalkyl-niederen Alkyl- oder ein einen Phenylrest lieferndes Mittel, in Verfahrensvariante d) eine Verbindung der Formel VII, worin $R^{64}$ die obige im Falle der Gruppe (e) für $R^6$ angegebene Bedeutung besitzt, und in Verfahrensvariante h) eine Verbindung der Formel I, worin $R^1$ einen Rest der Formel (d) oder (e) und $R^6$ niederes Hydroxyalkyl oder Hydroxy-($C_4$-$C_7$)-cycloalkyl bedeuten, als Ausgangsstoff verwendet.

**3.** Verfahren gemäss Anspruch 1 oder 2, worin $R^1$ die Gruppe $-C{\equiv}C-R^6$ (e) bedeutet.

**4.** Verfahren gemäss Anspruch 3, worin $R^6$ Wasserstoff, niederes Alkyl, niederes Hydroxyalkyl, niederes Alkoxyalkyl, ($C_3$-$C_7$)-Cycloalkyl, Hydroxy-($C_4$-$C_7$)-Cycloalkyl, niederes Alkoxy-($C_4$-$C_7$)-Cycloalkyl, ($C_3$-$C_7$)-Cycloalkyl-niederes Alkyl, ($C_3$-$C_7$)-Cycloalkyl-niederes Hydroxyalkyl oder ($C_3$-$C_7$)-Cycloalkyl-niederes Alkoxyalkyl bedeutet.

**5.** Verfahren gemäss Anspruch 4, worin $R^6$ Wasserstoff, niederes Alkyl, niederes 1-Hydroxyalkyl, niederes 1-Alkoxyalkyl, ($C_3$-$C_7$)-Cycloalkyl, 1-Hydroxy-($C_4$-$C_7$)-cycloalkyl, 1-(niederes Alkoxy)-($C_4$-$C_7$)-cycloalkyl oder 1-[($C_3$-$C_7$)-Cycloalkyl]-niederes 1-Hydroxyalkyl, insbesondere niederes Alkyl, niederes 1-Hydroxyalkyl oder ($C_3$-$C_7$)-Cycloalkyl bedeutet.

**6.** Verfahren gemäss einem der Ansprüche 1 bis 5, worin $R^2$ Wasserstoff und $R^3$ Methyl bedeuten oder worin $R^2$ und $R^3$ zusammen Dimethylen oder Trimethylen bedeuten und das mit $\gamma$ bezeichnete Kohlenstoffatom die (S)-Konfiguration aufweist.

**7.** Verfahren gemäss einem der Ansprüche 1 bis 6, worin A einen Rest der Formel (a) und eines von $R^4$ und $R^5$ Wasserstoff und das andere Wasserstoff oder Halogen bedeuten.

**8.** Verfahren gemäss Anspruch 7, worin $R^4$ und $R^5$ beide Wasserstoff oder $R^4$ Wasserstoff und $R^5$ Fluor oder $R^4$ Chlor oder Brom und $R^5$ Wasserstoff bedeuten.

**9.** Verfahren nach Anspruch 1, dadurch gekennzeichnet, dass man 7-Chlor-4,5-dihydro-5-methyl-3-(1-propinyl)-6H-imidazo[1,5-a][1,4]benzodiazepin-6-on herstellt.

**10.** Verfahren nach Anspruch 1, dadurch gekennzeichnet, dass man 7-Chlor-4,5-dihydro-3-(3-hydroxy-3-methyl-1-butinyl)-5-methyl-6H-imidazo[1,5-a][1,4]benzodiazepin-6-on herstellt.

**11.** Verfahren nach Anspruch 1, dadurch gekennzeichnet, dass man 7-Brom-4,5-dihydro-3-(3-hydroxy-3-methyl-1-butinyl)-5-methyl-6H-imidazo[1,5-a][1,4]benzodiazepin-6-on herstellt.

**12.** Verfahren nach Anspruch 1, dadurch gekennzeichnet, dass man 7-Chlor-4,5-dihydro-3-(3-hydroxy-1-butinyl)-5-methyl-6H-imidazo[1,5-a][1,4]benzodiazepin-6-on herstellt.

**13.** Verfahren nach Anspruch 1, dadurch gekennzeichnet, dass man 4,5-Dihydro-5-methyl-3-(1-propinyl)-6H-imidazo[1,5-a]-[1,4]benzodiazepin-6-on herstellt.

**14.** Verfahren nach Anspruch 1, dadurch gekennzeichnet, dass man 7-Chlor-4,5-dihydro-5-methyl-3-(3-methyl-1-butinyl)-6H-imidazo[1,5-a][1,4]benzodiazepin-6-on herstellt.

**15.** Verfahren nach Anspruch 1, dadurch gekennzeichnet, dass man 7-Chlor-4,5-dihydro-3-(3-hydroxy-1-propinyl)-5-methyl-6H-imidazo[1,5-a][1,4]benzodiazepin-6-on herstellt.

**16.** Verfahren nach Anspruch 1, dadurch gekennzeichnet, dass man 7-Chlor-3-(cyclopropyläthinyl)-4,5-dihydro-5-methyl-6H-imidazo[1,5-a][1,4]benzodiazepin-6-on herstellt.

**17.** Verwendung von Verbindungen der in Anspruch 1 definierten Formel I für die Herstellung von Arzneimitteln zur Anwendung bei der Bekämpfung oder Verhütung von Konvulsionen, Angstzuständen,

Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

**Patentansprüche für folgenden Vertragsstaat : GR**

**1.** Verfahren Zur Herstellung von Verbindungen der allgemeinen Formel

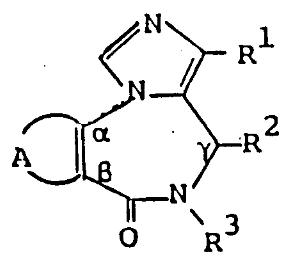

I

worin A zusammen mit den beiden mit $\alpha$ und $\beta$ bezeichneten Kohlenstoffatomen eine der Gruppen

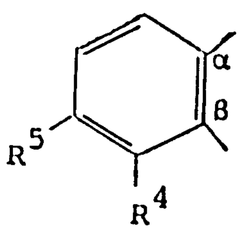

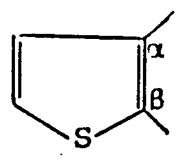

und

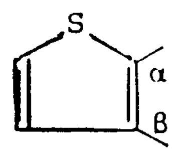

(a) (b) (c)

$R^1$ eine der Gruppen

$$-CH{=}CH{-}R^6 \quad \text{und} \quad -C{\equiv}C{-}R^6,$$

(d) (e)

$R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl und $R^6$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeuten, wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen oder Trimethylen bedeuten, und wobei die in Gruppe (d) vorhandene Doppelbindung die E- und/oder Z-Konfiguration aufweist, wenn $R^6$ von Wasserstoff verschieden ist, und die mit "nieder" bezeichneten Reste bis zu 7 Kohlenstoffatome besitzen

dadurch gekennzeichnet, dass man

a) eine Verbindung der allgemeinen Formel

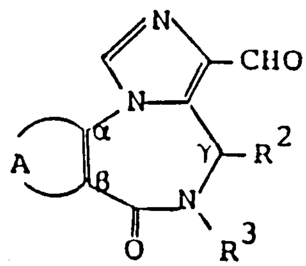

II

worin A, $R^2$ und $R^3$ obige Bedeutung besitzen, mit einer Verbindung der allgemeinen Formel

$(Ar)_3P = CH\text{-}R^{61}$     III

worin $R^{61}$ Wasserstoff, Halogen, einen monocyclischen aromatischen Kohlenwasserstoffrest, welcher durch niederes Alkyl, niederes Alkoxy oder Halogen substituiert sein kann, oder eine gegebenenfalls durch niederes Alkoxy, $(C_3\text{-}C_7)$-Cycloalkyl oder Oxo mono- oder disubstituierte gesättigte niedere Kohlenwasserstoffgruppe und Ar einen monocyclischen aromatischen Kohlenwasserstoffrest, welcher durch niederes Alkyl, niederes Alkoxy oder Halogen substituiert sein kann, bedeuten, umsetzt; oder

b) eine Verbindung der allgemeinen Formel

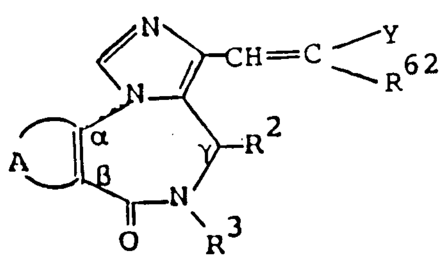

IV

worin $R^{62}$ Wasserstoff oder Halogen und Y Halogen bedeuten, und A, $R^2$ und $R^3$ obige Bedeutung besitzen, dehydrohalogeniert; oder

c) eine Verbindung der Formel I, worin $R^1$ die Gruppe (e) und $R^6$ Wasserstoff bedeuten, mit einem einen gegebenenfalls durch Hydroxy, niederes Alkoxy, $(C_3\text{-}C_7)$-Cycloalkyl oder Oxo mono- oder disubstituierten gesättigten niederen Kohlenwasserstoff- oder einen monocyclischen aromatischen Kohlenwasserstoffrest, welcher durch niederes Alkyl, niederes Alkoxy oder Halogen substituiert sein kann, oder Halogen liefernden Mittel behandelt; oder

d) eine Verbindung der allgemeinen Formel

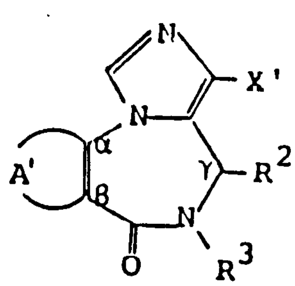

VI

worin $R^2$ und $R^3$ obige Bedeutung besitzen und X' Brom oder Jod und A' einen Rest der Formel (a), (b) oder (c) bedeutet, wobei aber im Falle, dass A' einen Rest der Formel (a) und $R^4$ und/oder $R^5$

Halogen bedeuten, dieses Halogen Fluor oder Chlor ist, wenn X Brom bedeutet, bzw. Fluor, Chlor oder Brom ist, wenn X Jod bedeutet,
mit einer Verbindung der allgmeinen Formel

$HC \equiv C\text{-}R^{64}$ VII

worin $R^{64}$ Wasserstoff, einen monocyclischen aromatischen Kohlenwasserstoffrest, welcher durch niederes Alkyl, niederes Alkoxy oder Halogen substituiert sein kann, oder eine gegebenenfalls durch Hydroxy, niederes Alkoxy, ($C_3$-$C_7$)-Cycloalkyl oder Oxo mono- oder disubstituierte gesättigte niedere Kohlenwasserstoffgruppe bedeutet,
umsetzt; oder
e) aus einer Verbindung der allgemeinen Formel

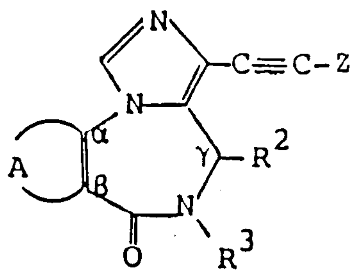

VIII

worin $R^2$, $R^3$ und A obige Bedeutung besitzen und Z eine Schutzgruppe bedeutet,
die Schutzgruppe abspaltet; oder
f) in einer Verbindung der allgemeinen Formel

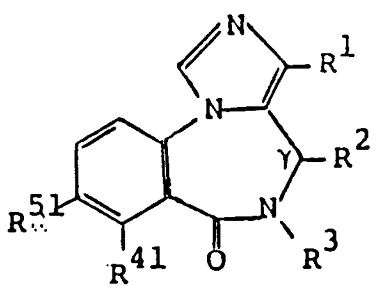

IX

worin R, $R^2$ $R^3$ und "nieder" obige Bedeutung besitzen und eines von $R^{41}$ und $R^{51}$ Amino und das andere Wasserstoff, Halogen, Trifluormethyl oder niederes Alkyl bedeutet,
die Aminogruppe durch ein Wasserstoff- oder Halogenatom ersetzt; oder
g) eine Verbindung der Formel I, worin $R^1$ einen Rest der Formel (e) bedeutet und worin im Falle, dass A einen Rest der Formel (a) bedeutet, $R^4$ und/oder $R^5$ nicht Jod bedeutet, zur entsprechenden Verbindung der Formel I, worin $R^1$ einen Rest der Formel (d) bedeutet, reduziert; oder
h) eine Verbindung der Formel I, worin $R^1$ einen Rest der Formel (d) oder (e) und $R^6$ eine durch Hydroxy substituierte gesättigte niedere Kohlenwasserstoffgruppe bedeuten, mit einem einen niederen Alkylrest liefernden Mittel behandelt; oder
i) einer Verbindung der Formel I, worin $R^1$ die Gruppe (d) oder (e) und $R^6$ eine durch Oxo substituierte gesättigte niedere Kohlenwasserstoffgruppe bedeuten, die Carbonylgruppe reduziert.

**2.** Verfahren gemäss Anspruch 1 zur Herstellung von Verbindungen der Formel I, worin $R^1$ die Gruppe $-CH=CH-R^6$ (d) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl, Phenyl oder Halogen bedeuten, oder $R^1$ die Gruppe $-C \equiv C-R^6$ (e) und $R^6$ Wasserstoff, niederes Alkyl, niederes Hydroxyalkyl, niederes Alkoxy-niederes Alkyl, ($C_3$-$C_7$)-Cycloalkyl, Hydroxy-($C_4$-$C_7$)-cycloalkyl, niederes Alkoxy-($C_4$-$C_7$)-cycloalkyl, ($C_3$-$C_7$)-Cycloalkyl-niederes Alkyl oder Phenyl bedeuten, dadurch gekennzeichnet, dass man eine der Verfahrensvarianten a) bis h) anwendet

und in Verfahrensvariante a) eine Verbindung der Formel III, worin $R^{61}$ Wasserstoff, niederes Alkyl, niederes Alkoxy-niederes Alkyl, ($C_3$-$C_7$)-Cycloalkyl, niederes Alkoxy-($C_4$-$C_7$)-cycloalkyl, ($C_3$-$C_7$)-Cycloalkyl-niederes Alkyl, Phenyl oder Halogen bedeutet, in Verfahrensvariante b) eine Verbindung der Formel IV, worin $R^{62}$ Wasserstoff bedeutet, in Verfahrensvariante c) ein einen niederen Alkyl-, einen niederen Hydroxyalkyl-, einen niederen Alkoxy-niederen Alkyl-, einen ($C_4$-$C_7$)-Cycloalkyl-, einen Hydroxy-($C_4$-$C_7$)-cycloalkyl-, einen niederen Alkoxy-($C_4$-$C_7$)-cycloalkyl-, einen ($C_3$-$C_7$)-Cycloalkyl-niederen Alkyl- oder ein einen Phenylrest lieferndes Mittel, in Verfahrensvariante d) eine Verbindung der Formel VII, worin $R^{64}$ die obige im Falle der Gruppe (e) für $R^6$ angegebene Bedeutung besitzt, und in Verfahrensvariante h) eine Verbindung der Formel I, worin $R^1$ einen Rest der Formel (d) oder (e) und $R^6$ niederes Hydroxyalkyl oder Hydroxy-($C_4$-$C_7$)-cycloalkyl bedeuten, als Ausgangsstoff verwendet.

**3.** Verfahren gemäss Anspruch 1 oder 2, worin $R^1$ die Gruppe $-C{\equiv}C-R^6$ (e) bedeutet.

**4.** Verfahren gemäss Anspruch 3, worin $R^6$ Wasserstoff, niederes Alkyl, niederes Hydroxyalkyl, niederes Alkoxyalkyl, ($C_3$-$C_7$)-Cycloalkyl, Hydroxy-($C_4$-$C_7$)-Cycloalkyl, niederes Alkoxy-($C_4$-$C_7$)-Cycloalkyl, ($C_3$-$C_7$)-Cycloalkyl-niederes Alkyl, ($C_3$-$C_7$)-Cycloalkyl-niederes Hydroxyalkyl oder ($C_3$-$C_7$)-Cycloalkyl-niederes Alkoxyalkyl bedeutet.

**5.** Verfahren gemäss Anspruch 4, worin $R^6$ Wasserstoff, niederes Alkyl, niederes 1-Hydroxyalkyl, niederes 1-Alkoxyalkyl, ($C_3$-$C_7$)-Cycloalkyl, 1-Hydroxy-($C_4$-$C_7$)-cycloalkyl, 1-(niederes Alkoxy)-($C_4$-$C_7$)-cycloalkyl oder 1-[($C_3$-$C_7$)-Cycloalkyl]-niederes 1-Hydroxyalkyl, insbesondere niederes Alkyl, niederes 1-Hydroxyalkyl oder ($C_3$-$C_7$)-Cycloalkyl bedeutet.

**6.** Verfahren gemäss einem der Ansprüche 1 bis 5, worin $R^2$ Wasserstoff und $R^3$ Methyl bedeuten oder worin $R^2$ und $R^3$ zusammen Dimethylen oder Trimethylen bedeuten und das mit $\gamma$ bezeichnete Kohlenstoffatom die (S)-Konfiguration aufweist.

**7.** Verfahren gemäss einem der Ansprüche 1 bis 6, worin A einen Rest der Formel (a) und eines von $R^4$ und $R^5$ Wasserstoff und das andere Wasserstoff oder Halogen bedeuten.

**8.** Verfahren gemäss Anspruch 7, worin $R^4$ und $R^5$ beide Wasserstoff oder $R^4$ Wasserstoff und $R^5$ Fluor oder $R^4$ Chlor oder Brom und $R^5$ Wasserstoff bedeuten.

**9.** Verfahren nach Anspruch 1, dadurch gekennzeichnet, dass man 7-Chlor-4,5-dihydro-5-methyl-3-(1-propinyl)-6H-imidazo[1,5-a][1,4]benzodiazepin-6-on herstellt.

**10.** Verfahren nach Anspruch 1, dadurch gekennzeichnet, dass man 7-Chlor-4,5-dihydro-3-(3-hydroxy-3-methyl-1-butinyl)-5-methyl-6H-imidazo[1,5-a][1,4]benzodiazepin-6-on herstellt.

**11.** Verfahren nach Anspruch 1, dadurch gekennzeichnet, dass man 7-Brom-4,5-dihydro-3-(3-hydroxy-3-methyl-1-butinyl)-5-methyl-6H-imidazo[1,5-a][1,4]benzodiazepin-6-on herstellt.

**12.** Verfahren nach Anspruch 1, dadurch gekennzeichnet, dass man 7-Chlor-4,5-dihydro-3-(3-hydroxy-1-butinyl)-5-methyl-6H-imidazo[1,5-a][1,4]benzodiazepin-6-on herstellt.

**13.** Verfahren nach Anspruch 1, dadurch gekennzeichnet, dass man 4,5-Dihydro-5-methyl-3-(1-propinyl)-6H-imidazo[1,5-a][1,4]benzodiazepin-6-on herstellt.

**14.** Verfahren nach Anspruch 1, dadurch gekennzeichnet, dass man 7-Chlor-4,5-dihydro-5-methyl-3-(3-methyl-1-butinyl)-6H-imidazo[1,5-a][1,4]benzodiazepin-6-on herstellt.

**15.** Verfahren nach Anspruch 1, dadurch gekennzeichnet, dass man 7-Chlor-4,5-dihydro-3-(3-hydroxy-1-propinyl)-5-methyl-6H-imidazo[1,5-a][1,4]benzodiazepin-6-on herstellt.

**16.** Verfahren nach Anspruch 1, dadurch gekennzeichnet, dass man 7-Chlor-3-(cyclopropyläthinyl)-4,5-dihydro-5-methyl-6H-imidazo[1,5-a][1,4]benzodiazepin-6-on herstellt.

**17.** Verfahren zur Herstellung von Arzneimitteln, insbesondere von Mitteln, welche antikonvulsiv, anxiolytisch, muskelrelaxierend und sedativ-hypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren, dadurch gekennzeichnet, dass man eine oder mehrere Verbindungen der in Anspruch 1 definierten Formel I und erwünschtenfalls eine oder mehrere andere therapeutisch wertvolle Stoffe zusammen mit einem therapeutisch inerten Träger in eine galenische Darreichungsform bringt.

**18.** Verwendung von Verbindungen der in Anspruch 1 definierten Formel I für die Herstellung von Arzneimitteln zur Anwendung bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

**19.** Verbindungen der allgemeinen Formel

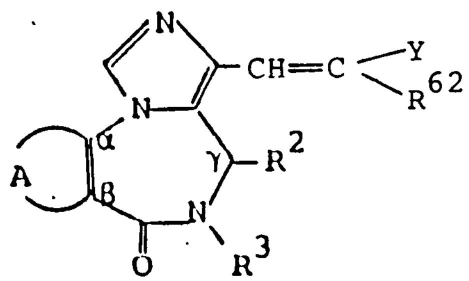

IVa

worin Y und $R^{62}$ je Halogen bedeuten, und A, $R^2$ und $R^3$ die in Anspruch 1 angegebene Bedeutung haben.

**20.** Verbindungen der allgemeinen Formel

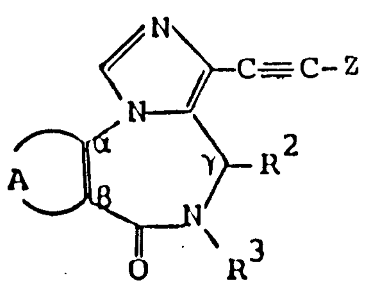

VIII

worin Z eine Schutzgruppe bedeutet, und A, $R^2$ und $R^3$ die in Anspruch 1 angegebene Bedeutung haben.

**21.** Verbindungen der allgemeinen Formel

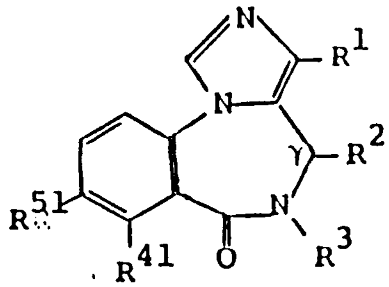

IX

worin eines von $R^{41}$ und $R^{51}$ Amino und das andere Wasserstoff, Halogen, Trifluormethyl oder niederes Alkyl bedeutet, und $R^1$, $R^2$ und $R^3$ die in Anspruch 1 angegebene Bedeutung haben.

**Claims**

**Claims for the following Contracting States: AT, BE, CH, DE, FR, GB, IT, LI, LU, NL, SE**

**1.** Compounds of the general formula

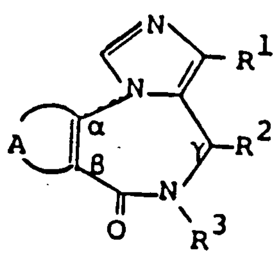

I

wherein A together with the two carbon atoms denoted by $\alpha$ and $\beta$ signifies one of the groups

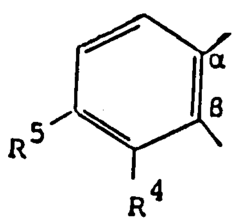

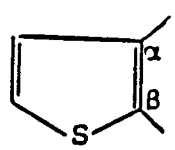

and

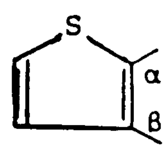

(a) (b) (c)

$R^1$ signifies one of the groups

$$-CH=CH-R^6 \quad \text{and} \quad -C\equiv C-R^6,$$

(d) (e)

$R^2$ signifies hydrogen and $R^3$ signifies lower alkyl or $R^2$ and $R^3$ together signify dimethylene or trimethylene, $R^4$ and $R^5$ each signify hydrogen, halogen, trifluoromethyl or lower alkyl and $R^6$ signifies hydrogen, halogen, a monocyclic aromatic hydrocarbon residue which can be substituted by lower

alkyl, lower alkoxy or halogen or a saturated lower hydrocarbon group which is optionally mono- or disubstituted by hydroxy, lower alkoxy, ($C_3$-$C_7$)-cycloalkyl or oxo, whereby the compounds of formula I have the (S)- or (R,S)-configuration with reference to the carbon atom denoted by $\gamma$ when $R^2$ and $R^3$ together signify dimethylene or trimethylene and whereby the double bond present in group (d) has the E- and/or Z-configuration when $R^6$ is different from hydrogen, and the residue denoted as "lower" have up to 7 carbon atoms.

**2.** Compounds in accordance with claim 1, wherein $R^1$ signifies the group -CH=CH-$R^6$ (d) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl, phenyl or halogen or $R^1$ signifies the group -C≡C-$R^6$ (e) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl or phenyl.

**3.** Compounds in accordance with claim 1 or 2, wherein $R^1$ signifies the group -C≡C-$R^6$ (e).

**4.** Compounds in accordance with claim 3, wherein $R^6$ signifies hydrogen, lower alkyl, lower hydroxyalkyl, lower alkoxyalkyl, ($C_3$-$C_7$)-cycloalkyl, hydroxy-($C_4$--$C_7$)-cycloalkyl, lower alkoxy-($C_4$-$C_7$)-cycloalkyl, ($C_3$-$C_7$)-cycloalkyl-lower alkyl, ($C_3$-$C_7$)-cycloalkyl-lower hydroxyalkyl or ($C_3$-$C_7$)-cycloalkyl-lower alkoxyalkyl.

**5.** Compounds in accordance with claim 4, wherein $R^6$ signifies hydrogen, lower alkyl, lower 1-hydroxyalkyl, lower 1-alkoxyalkyl, ($C_3$-$C_7$)-cycloalkyl, 1-hydroxy-($C_4$-$C_7$)-cycloalkyl, 1-(lower alkoxy)-($C_4$-$C_7$)-cycloalkyl or 1-[($C_3$-$C_7$)-cycloalkyl]-lower 1-hydroxyalkyl, especially lower alkyl, lower 1-hydroxyalkyl or ($C_3$-$C_7$)-cycloalkyl.

**6.** Compounds in accordance with any one of claims 1 to 5, wherein $R^2$ signifies hydrogen and $R^3$ signifies methyl or wherein $R^2$ and $R^3$ together signify dimethylene or trimethylene and the carbon atom denoted by $\gamma$ has the (S)-configuration.

**7.** Compounds in accordance with any one of claims 1 to 6, wherein A signifies a residue of formula (a) and one of $R^4$ and $R^5$ signifies hydrogen and the other signifies hydrogen or halogen.

**8.** Compounds in accordance with claim 7, wherein $R^4$ and $R^5$ both signify hydrogen or $R^4$ signifies hydrogen and $R^5$ signifies fluorine or $R^4$ signifies chlorine or bromine and $R^5$ signifies hydrogen.

**9.** 7-Chloro-4,5-dihydro-5-methyl-3-(1-propynyl)-6H-imidazo[1,5-a][1,4]benzodiazepin-6-one.

**10.** 7-Chloro-4,5-dihydro-3-(3-hydroxy-3-methyl-1-butynyl)-5-methyl-6H-imidazo[1,5-a][1,4]benzodiazepin-6-one.

**11.** 7-Bromo-4,5-dihydro-3-(3-hydroxy-3-methyl-1-butynyl)-5-methyl-6H-imidazo[1,5-a][1,4]benzodiazepin-6-one.

**12.** 7-Chloro-4,5-dihydro-3-(3-methyl-1-butynyl)-5-methyl-6H-imidazo[1,5-a][1,4]benzodiazepin-6-one.

**13.** 4,5-Dihydro-5-methyl-3-(1-propynyl)-6H-imidazo[1,5-a][1,4]benzodiazepin-6-one.

**14.** 7-Chloro-4,5-dihydro-5-methyl-3-(3-methyl-1-butynyl)-6H-imidazo[1,5-a][1,4]benzodiazepin-6-one.

**15.** 7-Chloro-4,5-dihydro-3-(3-hydroxy-1-propynyl)-5-methyl-6H-imidazo[1,5-a][1,4]benzodiazepin-6-one.

**16.** 7-Chloro-3-(cyclopropylethynyl)-4,5-dihydro-5-methyl-6H-imidazo[1,5-a][1,4]benzodiazepin-6-one.

**17.** Compounds of the general formula

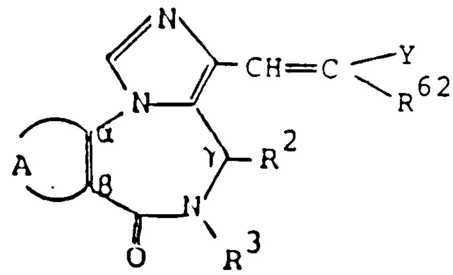

IVa

wherein Y and $R^{62}$ each signify halogen and A, $R^2$ and $R^3$ have the significance given in claim 1.

**18.** Compounds of the general formula

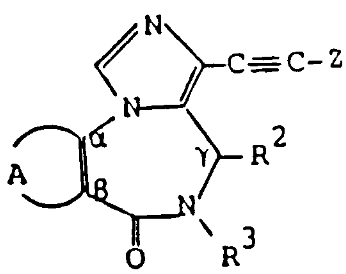

VIII

wherein Z signifies a protecting group and A, $R^2$ and $R^3$ have the significance given in claim 1.

**19.** Compounds of the general formula

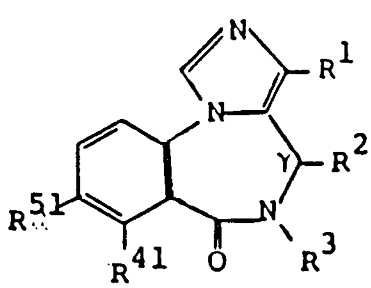

IX

wherein one of $R^{41}$ and $R^{51}$ signifies amino and the other signifies hydrogen, halogen, trifluoromethyl or lower alkyl and $R^1$, $R^2$, $R^3$ and "lower" have the significance given in claim 1.

**20.** Compounds in accordance with any one of claims 1 to 16 for use as therapeutically active substances.

**21.** Compounds in accordance with any one of claims 1 to 16 for use as therapeutically active substances which have anticonvulsant, anxiolytic, muscle relaxant and sedative-hypnotic activity and/or which partially or completely selectively antagonize some or all activities which 1,4-benzodiazepines having tranquillizing activity or other substances display via the central benzodiazepine receptors.

**22.** A process for the manufacture of compounds in accordance with any one of claims 1 to 16, characterized by

a) reacting a compound of the general formula

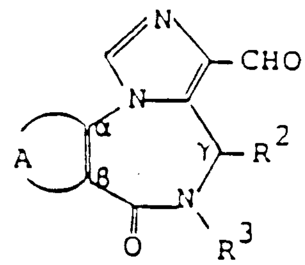

II

wherein A, $R^2$ and $R^3$ have the significance given in claim 1, with a compound of the general formula

$(Ar)_3P=CH\text{-}R^{61}$ III

wherein $R^{61}$ signifies hydrogen, halogen, a monocyclic aromatic hydrocarbon residue which can be substituted by lower alkyl, lower alkoxy or halogen or a saturated lower hydrocarbon group which is optionally mono- or disubstituted by lower alkoxy, $(C_3\text{-}C_7)$-cycloalkyl or oxo and Ar signifies a monocyclic aromatic hydrocarbon residue which can be substituted by lower alkyl, lower alkoxy or halogen;

or

b) dehydrohalogenating a compound of the general formula

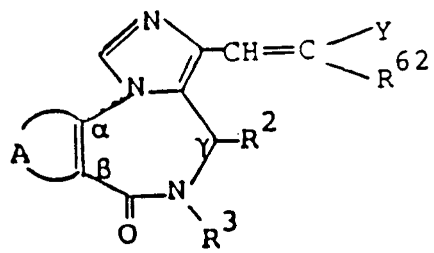

IV

wherein $R^{62}$ signifies hydrogen or halogen and Y signifies halogen, and A, $R^2$ and $R^3$ have the significance given in claim 1;

or

c) treating a compound of formula I in which $R^1$ signifies group (e) and $R^6$ signifies hydrogen with an agent yielding a saturated lower hydrocarbon residue which is optionally mono- or disubstituted by hydroxy, lower alkoxy, $(C_3\text{-}C_7)$-cycloalkyl or oxo or a monocyclic aromatic hydrocarbon residue which can be substituted by lower alkyl, lower alkoxy or halogen; or

d) reacting a compound of the general formula

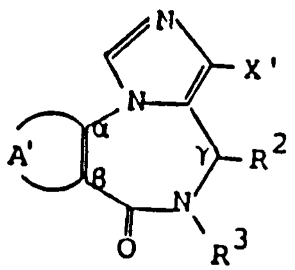

VI

wherein $R^2$ and $R^3$ have the significance given in claim 1 and X' signifies bromine or iodine and A' signifies a residue of formula (a), (b) or (c), with the proviso that where A' signifies a residue of formula (a) and $R^4$ and/or $R^5$ signify halogen, this halogen is fluorine or chlorine when X' signifies bromine and is fluorine, chlorine or bromine when X' signifies iodine,

with a compound of the general formula

$HC \equiv C\text{-}R^{64}$ VII

wherein $R^{64}$ signifies hydrogen, a monocyclic aromatic hydrocarbon residue which can be substituted by lower alkyl, lower alkoxy or halogen or a saturated lower hydrocarbon group which is optionally mono- or disubstituted by hydroxy, lower alkoxy, ($C_3$-$C_7$)-cycloalkyl or oxo;
or
e) cleaving off the protecting group from a compound of the general formula

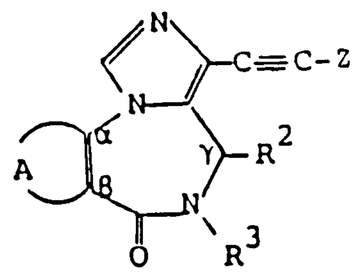

VIII

wherein $R^2$, $R^3$ and A have the significance given in claim 1 and Z signifies a protecting group;
or
f) replacing the amino group in a compound of the general formula

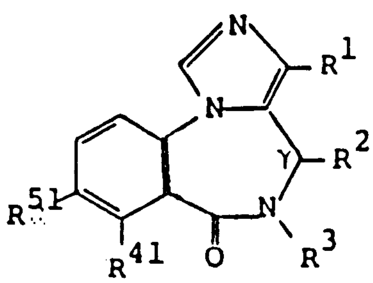

IX

wherein $R^1$, $R^2$ and $R^3$ have the significance given in claim 1 and one of $R^{41}$ and $R^{51}$ signifies amino and the other signifies hydrogen, halogen, trifluoromethyl or lower alkyl,
by a hydrogen or halogen atom; or
g) reducing a compound of formula I in which $R^1$ signifies a residue of formula (e) and in which, where A signifies a residue of formula (a), $R^4$ and/or $R^5$ do not signify iodine, to the corresponding compound of formula I in which $R^1$ signifies a residue of formula (d); or
h) treating a compound of formula I in which $R^1$ signifies a residue of formula (d) or (e) and $R^6$ signifies a saturated lower hydrocarbon group which is substituted by hydroxy with an agent yielding a lower alkyl residue; or
i) reducing the carbonyl group in a compound of formula I in which $R^1$ signifies group (d) or (e) and $R^6$ signifies a saturated lower hydrocarbon group which is substituted by oxo.

**23.** Medicaments containing a compound in accordance with any one of claims 1 to 16 and a therapeutically inert carrier.

**24.** Medicaments in accordance with claim 23, which have anticonvulsant, anxiolytic, muscle relaxant and sedative-hypnotic activity and/or which partially or completely selectively antagonize some or all activities which 1,4-benzodiazepines having tranquillizing activity or other substances display via the central benzodiazepine receptors.

**25.** The use of compounds in accordance with any one of claims 1 to 16 for the manufacture of medicaments for use in the control or prevention of convulsions, anxiety states, stress conditions,

excitation conditions and sleep disorders and/or in the partial or complete selective antagonization of some or all activities which 1,4-benzodiazepines having tranquillizing activity or other substances display via the central benzodiazepine receptors.

**Claims for the following Contracting State : ES**

**1.** A process for the manufacture of compounds of the general formula

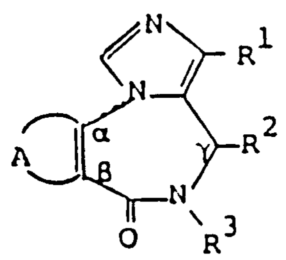

I

wherein A together with the two carbon atoms denoted by $\alpha$ and $\beta$ signifies one of the groups

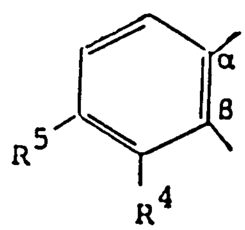

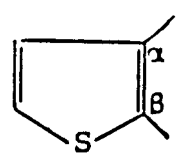

and

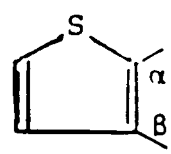

(a) (b) (c)

$R^1$ signifies one of the groups

$$-CH{=}CH{-}R^6 \quad \text{and} \quad -C{\equiv}C{-}R^6,$$

(d) (e)

$R^2$ signifies hydrogen and $R^3$ signifies lower alkyl or $R^2$ and $R^3$ together signify dimethylene or trimethylene, $R^4$ and $R^5$ each signify hydrogen, halogen, trifluoromethyl or lower alkyl and $R^6$ signifies hydrogen, halogen, a monocyclic aromatic hydrocarbon residue which can be substituted by lower alkyl, lower alkoxy or halogen or a saturated lower hydrocarbon group which is optionally mono- or disubstituted by hydroxy, lower alkoxy, ($C_3$-$C_7$)-cycloalkyl or oxo, whereby the compounds of formula I have the (S)- or (R,S)-configuration with reference to the carbon atom denoted by $\gamma$ when $R^2$ and $R^3$ together signify dimethylene or trimethylene and whereby the double bond present in group (d) has the E- and/or Z-configuration when $R^6$ is different from hydrogen, and the residue denoted as "lower" have up to 7 carbon atoms,
characterized by

a) reacting a compound of the general formula

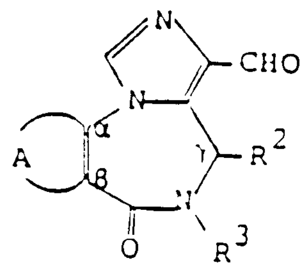

II

wherein A, $R^2$ and $R^3$ have the significance given as above, with a compound of the general formula

$(Ar)_3P=CH-R^{61}$ III

wherein $R^{61}$ signifies hydrogen, halogen, a monocyclic aromatic hydrocarbon residue which can be substituted by lower alkyl, lower alkoxy or halogen or a saturated lower hydrocarbon group which is optionally mono- or disubstituted by lower alkoxy, ($C_3$-$C_7$)-cycloalkyl or oxo and Ar signifies a monocyclic aromatic hydrocarbon residue which can be substituted by lower alkyl, lower alkoxy or halogen;

or

b) dehydrohalogenating a compound of the general formula

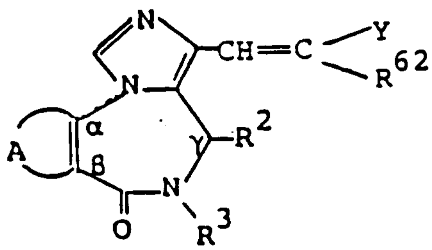

IV

wherein $R^{62}$ signifies hydrogen or halogen and Y signifies halogen, and A, $R^2$ and $R^3$ have the significance given as above,

or

c) treating a compound of formula I in which $R^1$ signifies group (e) and $R^6$ signifies hydrogen with an agent yielding a saturated lower hydrocarbon residue which is optionally mono- or disubstituted by hydroxy, lower alkoxy, ($C_3$-$C_7$)-cycloalkyl or oxo or a monocyclic aromatic hydrocarbon residue which can be substituted by lower alkyl, lower alkoxy or halogen; or

d) reacting a compound of the general formula

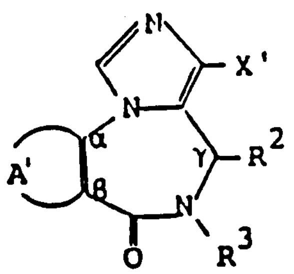

VI

wherein $R^2$ and $R^3$ have the significance given as above and X' signifies bromine or iodine and A' signifies a residue of formula (a), (b) or (c), with the proviso that where A' signifies a residue of formula (a) and $R^4$ and/or $R^5$ signify halogen, this halogen is fluorine or chlorine when X' signifies bromine and is fluorine, chlorine or bromine when X' signifies iodine,

with a compound of the general formula

HC≡C-$R^{64}$ VII

wherein $R^{64}$ signifies hydrogen, a monocyclic aromatic hydrocarbon residue which can be substituted by lower alkyl, lower alkoxy or halogen or a saturated lower hydrocarbon group which is optionally mono- or disubstituted by hydroxy, lower alkoxy, ($C_3$-$C_7$)-cycloalkyl or oxo;
or
e) cleaving off the protecting group from a compound of the general formula

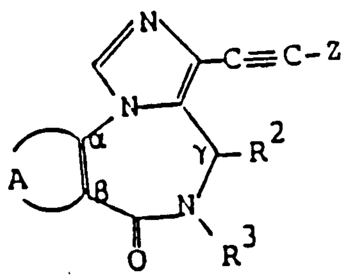

VIII

wherein $R^2$, $R^3$ and A have the significance given as above and Z signifies a protecting group;
or
f) replacing the amino group in a compound of the general formula

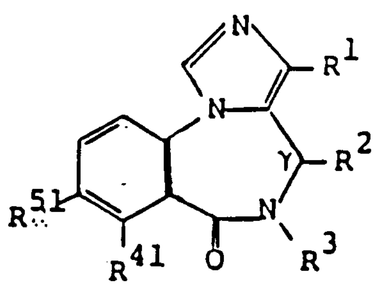

IX

wherein $R^1$, $R^2$, $R^3$ and "lower" have the significance given as above and one of $R^{41}$ and $R^{51}$ signifies amino and the other signifies hydrogen, halogen, trifluoromethyl or lower alkyl,
by a hydrogen or halogen atom; or
g) reducing a compound of formula I in which $R^1$ signifies a residue of formula (e) and in which, where A signifies a residue of formula (a), $R^4$ and/or $R^5$ do not signify iodine, to the corresponding compound of formula I in which $R^1$ signifies a residue of formula (d); or
h) treating a compound of formula I in which $R^1$ signifies a residue of formula (d) or (e) and $R^6$ signifies a saturated lower hydrocarbon group which is substituted by hydroxy with an agent yielding a lower alkyl residue; or
i) reducing the carbonyl group in a compound of formula I in which $R^1$ signifies group (d) or (e) and $R^6$ signifies a saturated lower hydrocarbon group which is substituted by oxo.

**2.** A process in accordance with claim 1 for the manufacture of compounds of formula I in which $R^1$ signifies the group -CH=CH-$R^6$ (d) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl, phenyl or halogen or $R^1$ signifies the group -C≡C-$R^6$ (e) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl or phenyl, characterized by using one of process variants a) to h) and in process variant a) using as the starting material a compound of formula III in which $R^{61}$ signifies hydrogen, lower alkyl, lower alkoxy-lower alkyl, ($C_3$-$C_7$)-cycloalkyl, lower alkoxy-($C_4$-$C_7$)-cycloalkyl, ($C_3$-$C_7$)-cycloalkyl-lower alkyl, phenyl or halogen, using in process variant b) as the starting material a compound of formula IV in which $R^{62}$ signifies hydrogen,

using in process variant c) as the starting material an agent yielding a lower alkoxy-lower alkyl, a ($C_4$-$C_7$)-cycloalkyl, a hydroxy-($C_4$-$C_7$)-cycloalkyl, a lower alkoxy-($C_4$-$C_7$)-cycloalkyl, a ($C_3$-$C_7$)-cycloalkyl-lower alkyl or a phenyl residue, using in process variant d) as the starting material a compound of formula VII in which $R^{64}$ has the significance given above for $R^6$ in the case of group (e) and using in process variant h) as the starting material a compound of formula I in which $R^1$ signifies a residue of formula (d) or (e) and $R^6$ signifies lower hydroxyalkyl or hydroxy-($C_4$-$C_7$)-cycloalkyl.

**3.** A process in accordance with claim 1 or 2, wherein $R^1$ signifies the group -C≡C-$R^6$ (e).

**4.** A process in accordance with claim 3, wherein $R^6$ signifies hydrogen, lower alkyl, lower hydroxyalkyl, lower alkoxyalkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl, ($C_3$-$C_7$)-cycloalkyl-lower hydroxyalkyl or ($C_3$-$C_7$)-cycloalkyl-lower alkoxyalkyl.

**5.** A process in accordance with claim 4, wherein $R^6$ signifies hydrogen, lower alkyl, lower 1-hydroxyalkyl, lower 1-alkoxyalkyl, ($C_3$-$C_7$)-cycloalkyl, 1-hydroxy-($C_4$-$C_7$)-cycloalkyl, 1-(lower alkoxy)-($C_4$-$C_7$)-cycloalkyl or 1-[($C_3$-$C_7$)-cycloalkyl]-lower 1-hydroxyalkyl, especially lower alkyl, lower 1-hydroxyalkyl or ($C_3$-$C_7$)-cycloalkyl.

**6.** A process in accordance with any one of claims 1 to 5, wherein $R^2$ signifies hydrogen and $R^3$ signifies methyl or wherein $R^2$ and $R^3$ together signify dimethylene or trimethylene and the carbon atom denoted by $\gamma$ has the (S)-configuration.

**7.** A process in accordance with any one of claims 1 to 6, wherein A signifies a residue of formula (a) and one of $R^4$ and $R^5$ signifies hydrogen and the other signifies hydrogen or halogen.

**8.** A process in accordance with claim 7, wherein $R^4$ and $R^5$ both signify hydrogen or $R^4$ signifies hydrogen and $R^5$ signifies fluorine or $R^4$ signifies chlorine or bromine and $R^5$ signifies hydrogen.

**9.** A process in accordance with claim 1, wherein 7-chloro-4,5-dihydro-5-methyl-3-(1-propynyl)-6H-imidazo[1,5-a][1,4]benzodiazepin-6-one is manufactured.

**10.** A process in accordance with claim 1, wherein 7-chloro-4,5-dihydro-3-(3-hydroxy-3-methyl-1-butynyl)-5-methyl-6H-imidazo[1,5-a][1,4]benzodiazepin- -6-one is manufactured.

**11.** A process in accordance with claim 1, wherein 7-bromo-4,5-dihydro-3-(3-hydroxy-3-methyl-1-butynyl)-5-methyl-6H-imidazo[1,5-a][1,4]benzodiazepin-6-one is manufactured.

**12.** A process in accordance with claim 1, wherein 7-chloro-4,5-dihydro-3-(3-methyl-1-butynyl)-5-methyl-6H-imidazo[1,5-a][1,4]benzodiazepin-6-one is manufactured.

**13.** A process in accordance with claim 1, wherein 4,5-dihydro-5-methyl-3-(1-propynyl)-6H-imidazo[1,5-a]-[1,4]benzodiazepin-6-one is manufactured.

**14.** A process in accordance with claim 1, wherein 7-chloro-4,5-dihydro-5-methyl-3-(3-methyl-1-butynyl)-6H-imidazo[1,5-a][1,4]benzodiazepin-6-one is manufactured.

**15.** A process in accordance with claim 1, wherein 7-chloro-4,5-dihydro-3-(3-hydroxy-1-propynyl)-5-methyl-6H-imidazo[1,5-a][1,4]benzodiazepin-6-one is manufactured.

**16.** A process in accordance with claim 1, wherein 7-chloro-3-(cyclopropylethynyl)-4,5-dihydro-5-methyl-6H-imidazo[1,5-a][1,4]benzodiazepin-6-one is manufactured.

**17.** The use of compounds of formula I defined in claim 1 for the manufacture of medicaments for use in the control or prevention of convulsions, anxiety states, stress conditions, excitation conditions and sleep disorders and/or in the partial or complete selective antagonization of some or all activities which 1,4-benzodiazepines having tranquillizing activity or other substances display via the central benzodiazepine receptors.

**Claims for the following Contracting State : GR**

**1.** A process for the manufacture of compounds of the general formula

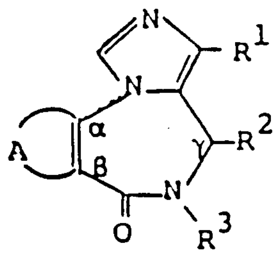

I

wherein A together with the two carbon atoms denoted by $\alpha$ and $\beta$ signifies one of the groups

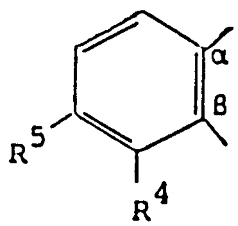

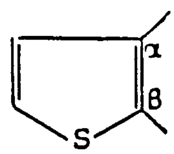

and

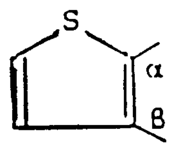

(a) (b) (c)

$R^1$ signifies one of the groups

$-CH{=}CH{-}R^6$ and $-C{\equiv}C{-}R^6$,

(d) (e)

$R^2$ signifies hydrogen and $R^3$ signifies lower alkyl $R^2$ signifies hydrogen and $R^3$ signifies lower alkyl or $R^2$ and $R^3$ together signify dimethylene or trimethylene, $R^4$ and $R^5$ each signify hydrogen, halogen, trifluoromethyl or lower alkyl and $R^6$ signifies hydrogen, halogen, a monocyclic aromatic hydrocarbon residue which can be substituted by lower alkyl, lower alkoxy or halogen or a saturated lower hydrocarbon group which is optionally mono- or disubstituted by hydroxy, lower alkoxy, ($C_3$-$C_7$)-cycloalkyl or oxo, whereby the compounds of formula I have the (S)- or (R,S)-configuration with reference to the carbon atom denoted by $\gamma$ when $R^2$ and $R^3$ together signify dimethylene or trimethylene and whereby the double bond present in group (d) has the E- and/or Z-configuration when $R^6$ is different from hydrogen, and the residue denoted as "lower" have up to 7 carbon atoms, characterized by

a) reacting a compound of the general formula

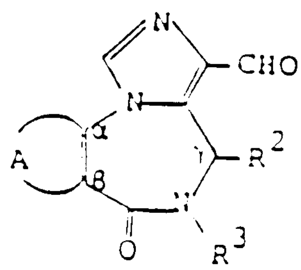

II

wherein A, $R^2$ and $R^3$ have the significance given as above, with a compound of the general formula

$(Ar)_3P=CH-R^{61}$ III

wherein $R^{61}$ signifies hydrogen, halogen, a monocyclic aromatic hydrocarbon residue which can be substituted by lower alkyl, lower alkoxy or halogen or a saturated lower hydrocarbon group which is optionally mono- or disubstituted by lower alkoxy, ($C_3$-$C_7$)-cycloalkyl or oxo and Ar signifies a monocyclic aromatic hydrocarbon residue which can be substituted by lower alkyl, lower alkoxy or halogen;

or

b) dehydrohalogenating a compound of the general formula

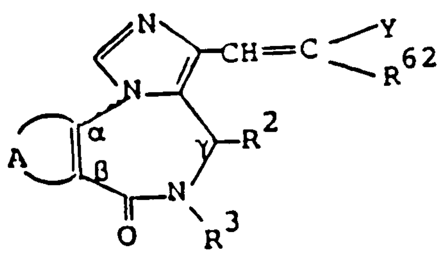

IV

wherein $R^{62}$ signifies hydrogen or halogen and Y signifies halogen, and A, $R^2$ and $R^3$ have the significance given as above;

or

c) treating a compound of formula I in which $R^1$ signifies group (e) and $R^6$ signifies hydrogen with an agent yielding a saturated lower hydrocarbon residue which is optionally mono- or disubstituted by hydroxy, lower alkoxy, ($C_3$-$C_7$)-cycloalkyl or oxo or a monocyclic aromatic hydrocarbon residue which can be substituted by lower alkyl, lower alkoxy or halogen; or

d) reacting a compound of the general formula

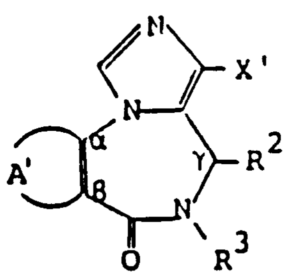

VI

wherein $R^2$ and $R^3$ have the significance given as above and X' signifies bromine or iodine and A' signifies a residue of formula (a), (b) or (c), with the proviso that where A' signifies a residue of formula (a) and $R^4$ and/or $R^5$ signify halogen, this halogen is fluorine or chlorine when X' signifies

bromine and is fluorine, chlorine or bromine when X' signifies iodine, with a compound of the general formula

$HC\equiv C\text{-}R^{64}$ VII

wherein $R^{64}$ signifies hydrogen, a monocyclic aromatic hydrocarbon residue which can be substituted by lower alkyl, lower alkoxy or halogen or a saturated lower hydrocarbon group which is optionally mono- or disubstituted by hydroxy, lower alkoxy, ($C_3$-$C_7$)-cycloalkyl or oxo; or

e) cleaving off the protecting group from a compound of the general formula

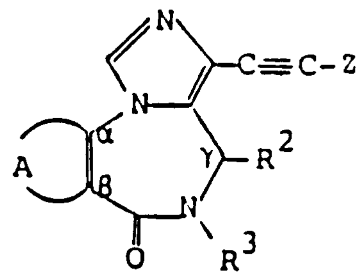

VIII

wherein $R^2$, $R^3$ and A have the significance given as above and Z signifies a protecting group; or

f) replacing the amino group in a compound of the general formula

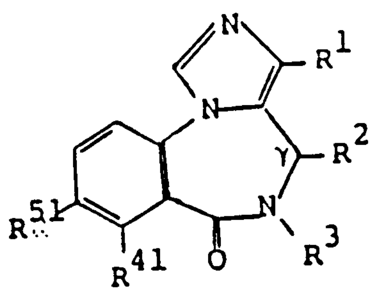

IX

wherein $R^1$, $R^2$, $R^3$ and "lower" have the significance given as above and one of $R^{41}$ and $R^{51}$ signifies amino and the other signifies hydrogen, halogen, trifluoromethyl or lower alkyl, by a hydrogen or halogen atom; or

g) reducing a compound of formula I in which $R^1$ signifies a residue of formula (e) and in which, where A signifies a residue of formula (a), $R^4$ and/or $R^5$ do not signify iodine, to the corresponding compound of formula I in which $R^1$ signifies a residue of formula (d); or

h) treating a compound of formula I in which $R^1$ signifies a residue of formula (d) or (e) and $R^6$ signifies a saturated lower hydrocarbon group which is substituted by hydroxy with an agent yielding a lower alkyl residue; or

i) reducing the carbonyl group in a compound of formula I in which $R^1$ signifies group (d) or (e) and $R^6$ signifies a saturated lower hydrocarbon group which is substituted by oxo.

**2.** A process in accordance with claim 1 for the manufacture of compounds of formula I in which $R^1$ signifies the group $-CH=CH-R^6$ (d) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl, phenyl or halogen or $R^1$ signifies the group $-C\equiv C-R^6$ (e) and $R^6$ signifies hydrogen, lower alkyl, lower hydroxyalkyl, lower alkoxy-lower alkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl or phenyl, characterized by using one of process variants a) to h) and in process variant a) using as the starting material a compound of formula III in which $R^{61}$ signifies hydrogen, lower alkyl, lower alkoxy-lower alkyl, ($C_3$-$C_7$)-cycloalkyl, lower alkoxy-($C_4$-$C_7$)-cycloalkyl, ($C_3$-$C_7$)-cycloalkyl-lower alkyl, phenyl or halogen, using in process variant b) as the starting material a compound of formula IV in which $R^{62}$ signifies hydrogen,

using in process variant c) as the starting material an agent yeilding a lower alkoxy-lower alkyl, a ($C_4$-$C_7$)-cycloalkyl, a hydroxy-($C_4$-$C_7$)-cycloalkyl, a lower alkoxy-($C_4$-$C_7$)-cycloalkyl, a ($C_3$-$C_7$)-cycloalkyl-lower alkyl or a phenyl residue, using in process variant d) as the starting material a compound of formula VII in which $R^{64}$ has the significance given above for $R^6$ in the case of group (e) and using in process variant h) as the starting material a compound of formula I in which $R^1$ signifies a residue of formula (d) or (e) and $R^6$ signifies lower hydroxy alkyl or hydroxy-($C_4$-$C_7$)-cycloalkyl.

**3.** A process in accordance with claim 1 or 2, wherein $R^1$ signifies the group -C≡C-$R^6$ (e).

**4.** A process in accordance with claim 3, wherein $R^6$ signifies hydrogen, lower alkyl, lower hydroxyalkyl, lower alkoxyalkyl, ($C_3$-$C_7$)-cycloalkyl, hydroxy-($C_4$-$C_7$)-cycloalkyl, lower alkoxy-($C_4$-$C_7$)-cycloalkyl, ($C_3$-$C_7$)-cycloalkyl-lower alkyl, ($C_3$-$C_7$)-cycloalkyl-lower hydroxyalkyl or ($C_3$-$C_7$)-cycloalkyl-lower alkoxyalkyl.

**5.** A process in accordance with claim 4, wherein $R^6$ signifies hydrogen, lower alkyl, lower 1-hydroxyalkyl, lower 1-alkoxyalkyl, ($C_3$-$C_7$)-cycloalkyl, 1-hydroxy-($C_4$-$C_7$)-cycloalkyl, 1-(lower alkoxy)-($C_4$-$C_7$)-cycloalkyl or 1-[($C_3$-$C_7$)-cycloalkyl]-lower 1-hydroxyalkyl, especially lower alkyl, lower 1-hydroxyalkyl or ($C_3$-$C_7$)-cycloalkyl.

**6.** A process in accordance with any one of claims 1 to 5, wherein $R^2$ signifies hydrogen and $R^3$ signifies methyl or wherein $R^2$ and $R^3$ together signify dimethylene or trimethylene and the carbon atom denoted by $\gamma$ has the (S)-configuration.

**7.** A process in accordance with any one of claims 1 to 6, wherein A signifies a residue of formula (a) and one of $R^4$ and $R^5$ signifies hydrogen and the other signifies hydrogen or halogen.

**8.** A process in accordance with claim 7, wherein $R^4$ and $R^5$ both signify hydrogen or $R^4$ signifies hydrogen and $R^5$ signifies fluorine or $R^4$ signifies chlorine or bromine and $R^5$ signifies hydrogen.

**9.** A process in accordance with claim 1, wherein 7-chloro-4,5-dihydro-5-methyl-3-(1-propynyl)-6H-imidazo[1,5-a][1,4]benzodiazepin-6-one is manufactured.

**10.** A process in accordance with claim 1, wherein 7-chloro-4,5-dihydro-3-(3-hydroxy-3-methyl-1-butynyl)-5-methyl-6H-imidazo[1,5-a][1,4]benzodiazepin- -6-one is manufactured.

**11.** A process in accordance with claim 1, wherein 7-bromo-4,5-dihydro-3-(3-hydroxy-3-methyl-1-butynyl)-5-methyl-6H-imidazo[1,5-a][1,4]benzodiazepin-6- -one is manufactured.

**12.** A process in accordance with claim 1, wherein 7-chloro-4,5-dihydro-3-(3-methyl-1-butynyl)-5-methyl-6H-imidazo[1,5-a][1,4]benzodiazepin-6-one is manufactured.

**13.** A process in accordance with claim 1, wherein 4,5-dihydro-5-methyl-3-(1-propynyl)-6H-imidazo[1,5-a]-[1,4]benzodiazepin-6-one is manufactured.

**14.** A process in accordance with claim 1, wherein 7-chloro-4,5-dihydro-5-methyl-3-(3-methyl-1-butynyl)-6H-imidazo[1,5-a][1,4]benzodiazepin-6-one is manufactured.

**15.** A process in accordance with claim 1, wherein 7-chloro-4,5-dihydro-3-(3-hydroxy-1-propyny])-5-methyl-6H-imidazo[1,5-a][1,4]benzodiazepin-6-one is manufactured.

**16.** A process in accordance with claim 1, wherein 7-chloro-3-(cyclopropylethynyl)-4,5-dihydro-5-methyl-6H-imidazo[1,5-a][1,4]benzodiazepin-6-one is manufactured.

**17.** A process for the manufacture of medicaments especially of medicaments which have anticonvulsive, anxiolytic, muscle relaxant and sedative-hypnotic activity and/or which partially or completely selectively antagonize some or all activities which 1,4-benzodiazepines having tranquillizing activity or other substances display via the central benzodiazepine receptors, characterized by bringing one or more compounds of formula I defined in claim 1 and, if desired, one or more other therapeutically valuable substances into a galenical administration form together with a therapeutically inert carrier.

**18.** The use of compounds of formula I defined in claim 1 for the manufacture of medicaments for use in the control or prevention of convulsions, anxiety states, stress conditions, excitation conditions and sleep disorders and/or in the partial or complete selective antagonization of some or all activities which 1,4-benzodiazepines having tranquillizing activity or other substances display via the central benzodiazepine receptors.

**19.** Compounds of the general formula

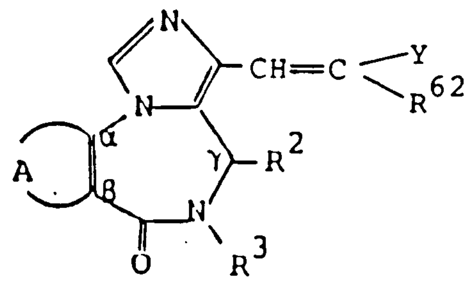

IVa

wherein Y and $R^{62}$ each signify halogen and A, $R^2$ and $R^3$ have the significance given in claim 1.

**20.** Compounds of the general formula

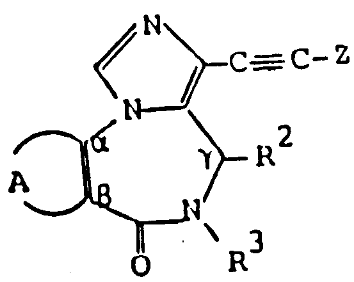

VIII

wherein Z signifies a protecting group and A, $R^2$ and $R^3$ have the significance given in claim 1.

**21.** Compounds of the general formula

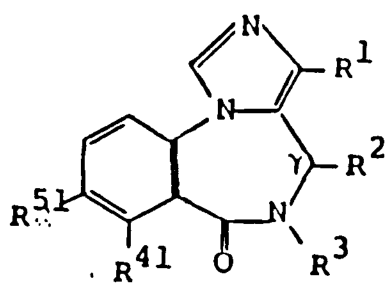

IX

wherein one of $R^{41}$ and $R^{51}$ signifies amino and the other signifies hydrogen, halogen, trifluoromethyl or lower alkyl and $R^1$, $R^2$ and $R^3$ have the significance given in claim 1.

**Revendications**

**Revendications pour les Etats contractants suivants : AT, BE, CH, DE, FR, GB, IT, LI, LU, NL, SE**

**1.** Composés de formule générale

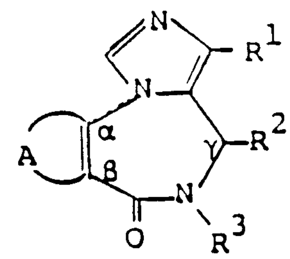

I

où A représente conjointement avec les deux atomes de carbone désignés par α et β l'un des groupes

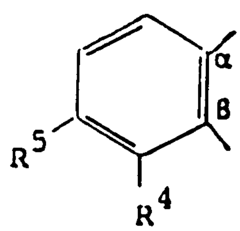

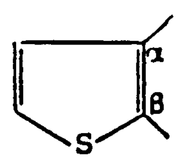

et

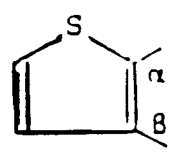

(a) (b) (c)

$R^1$ est un des groupes

$-CH{=}CH-R^6$ et $-C{\equiv}C-R^6$,

(d) (e)

$R^2$ désigne l'hydrogène et $R^3$ un groupe alkyle inférieur ou $R^2$ et $R^3$ représentent ensemble un groupe diméthylène ou triméthylène, $R^4$ et $R^5$ sont chacun l'hydrogène, un halogène ou un groupe trifluorométhyle ou alkyle inférieur et $R^6$ est l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo, tandis que les composés de formule I présentent en ce qui concerne l'atome de carbone désigné par γ la configuration (S) ou (R,S), lorsque $R^2$ et $R^3$ représentent ensemble un groupe diméthylène ou triméthylène, et tandis que la double liaison présente dans le groupe (d) présente la configuration E et/ou Z, lorsque $R^6$ est différent de l'hydrogène, et les restes désignés par "inférieur" présentent jusqu'à 7 atomes de carbone.

**2.** Composés selon la revendication 1, dans lesquels $R^1$ représente le groupe $-CH=CH-R^6$ (d) et $R^6$ est l'hydrogène ou un groupe alkyle inférieur, hydroxyalkyle inférieur, alcoxy inférieur-alkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur, phényle ou halogène, ou $R^1$ est le groupe $-C\equiv C-R^6$ (e) et $R^6$ est l'hydrogène ou un groupe alkyle inférieur, hydroxyalkyle inférieur, alcoxy-inférieur-alkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur ou phényle.

**3.** Composés selon l'une des revendications 1 ou 2, dans lesquels $R^1$ représente le groupe $-C\equiv C-R^6$ (e).

**4.** Composés selon la revendication 3, dans lesquels $R^6$ représente l'hydrogène ou un groupe alkyle inférieur, hydroxyalkyle inférieur, alcoxyalkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur, cycloalkyl en $C_3$-$C_7$-hydroxyalkyle inférieur ou cycloalkyl en $C_3$-$C_7$-alcoxyalkyle inférieur.

**5.** Composés selon la revendication 4, dans lesquels $R^6$ représente l'hydrogène ou un groupe alkyle inférieur, 1-hydroxyalkyle inférieur, 1-alcoxyalkyle inférieur, cycloalkyle en $C_3$-$C_7$, 1-hydroxy-cycloalkyle en $C_4$-$C_7$, 1-alcoxy inférieur-cycloalkyle en $C_4$-$C_7$ ou 1-(cycloalkyl en $C_3$-$C_7$)-1-hydroxyalkyle inférieur, en particulier alkyle inférieur, 1-hydroxyalkyle inférieur ou cycloalkyle en $C_3$-$C_7$.

**6.** Composés selon l'une quelconque des revendications 1 à 5, dans lesquels $R^2$ représente l'hydrogène et $R^3$ un groupe méthyle ou dans lesquels $R^2$ et $R^3$ représentent ensemble un groupe diméthylène ou triméthylène et l'atome de carbone désigné par $\gamma$ présente la configuration (S).

**7.** Composés selon l'une quelconque des revendications 1 à 6, dans lesquels A représente un reste de formule (a) et l'un des groupes $R^4$ et $R^5$ est l'hydrogène et l'autre l'hydrogène ou un halogène.

**8.** Composés selon la revendication 7, dans lesquels $R^4$ et $R^5$ sont tous deux l'hydrogène ou $R^4$ représente l'hydrogène et $R^5$ le fluor ou $R^4$ le chlore ou le brome et $R^5$ l'hydrogène.

**9.** 7-chloro-4,5-dihydro-5-méthyl-3-(1-propinyl)-6H-imidazo[1,5-a][1,4]benzodiazépine-6-one.

**10.** 7-chloro-4,5-dihydro-3-(3-hydroxy-3-méthyl-1-butinyl)-5-méthyl-6H-imidazo[1,5-a][1,4]benzodiazépine-6-one.

**11.** 7-bromo-4,5-dihydro-3-(3-hydroxy-3-méthyl-1-butinyl)-5-méthyl-6H-imidazo[1,5-a][1,4]benzodiazépine-6-one.

**12.** 7-chloro-4,5-dihydro-3-(3-hydroxy-1-butinyl)-5-méthyl-6H-imidazo[1,5-a][1,4]benzodiazépine-6-one.

**13.** 4,5-dihydro-5-méthyl-3-(1-propinyl)-6H-imidazo[1,5-a][1,4]benzodiazépine-6-one.

**14.** 7-chloro-4,5-dihydro-5-méthyl-3-(3-méthyl-1-butinyl)-6H-imidazo[1,5-a][1,4]benzodiazépine-6-one.

**15.** 7-chloro-4,5-dihydro-3-(3-hydroxy-1-propinyl)-5-méthyl-6H-imidazo[1,5-a][1,4]benzodiazépine-6-one.

**16.** 7-chloro-3-(cyclopropyléthinyl)-4,5-dihydro-5-méthyl-6H-imidazo[1,5-a][1,4]benzodiazépine-6-one.

**17.** Composés de formule générale

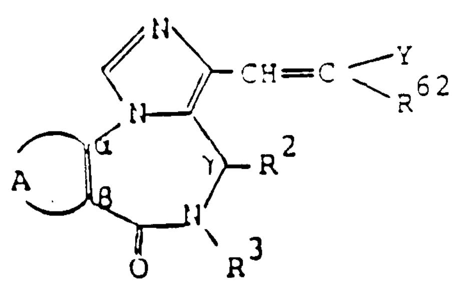

IVa

où Y et $R^{62}$ représentent chacun un halogène, et A, $R^2$ et $R^3$ ont la signification indiquée dans la revendicarion 1.

**18.** Composés de formule générale

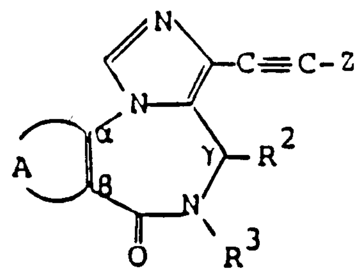

VIII

où Z représente un groupe protecteur Z, et A, $R^2$ et $R^3$ ont la signification indiquée dans la revendication 1.

**19.** Composés de formule générale

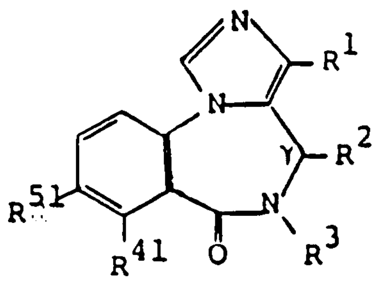

IX

où l'un des groupes $R^{41}$ et $R^{51}$ est un radical amino et l'autre est l'hydrogène, un halogène ou un groupe trifluorométhyle ou alkyle inférieur, et $R^1$ $R^2$, $R^3$ et "inférieur" ont la signification indiquée dans la revendication 1.

**20.** Composés selon l'une quelconque des revendications 1 à 16 pour une utilisation comme agents actifs thérapeutiques.

**21.** Composés selon l'une quelconque des revendications 1 à 16 pour une utilisation comme agents actifs thérapeutiques, qui ont une activité anticonvulsivante, anxiolytique, de relaxation musculaire et sédative-hypnotique et/ou sont partiellement ou totalement des antagonistes de certaines ou de la totalité des actions que présentent les 1,4-benzodiazépines ou autres substances à activité tranquillisante sur les récepteurs centraux de benzodiazépine.

**22.** Procédé pour la préparation de composés selon l'une quelconque des revendications 1 à 16, caractérisé en ce qu'

a) on fait réagir un composé de formule générale

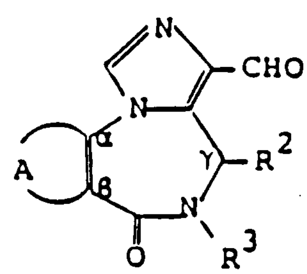

II

où A, $R^2$ et $R^3$ ont la signification indiquée dans la revendication 1, avec un composé de formule générale

$(Ar)_3P=CH-R^{61}$ III

où $R^{61}$ représente l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe alcoxy inférieur, cycloalkyle en $C_3$-$C_7$ ou oxo, et Ar est un reste hydrocarboné aromatique monocyclique, qui peut être substitué par un groupe alkyle inférieur, par un groupe alcoxy inférieur ou par un halogène; ou

b) on déshalogénhydrate un composé de formule générale

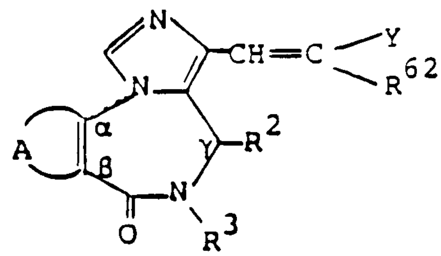

IV

où $R^{62}$ représente l'hydrogène ou un halogène et Y un halogène, et A, $R^2$ et $R^3$ ont la signification indiquée dans la revendication 1; ou

c) on traite un composé de formule I, ou $R^1$ représente le groupe (e) et $R^6$ l'hydrogène, avec un agent fournissant un reste hydrocarboné inférieur saturé éventuellement mono- ou disubstitué par un groupe hydroxy, alcoxy inférieur, cycloalkyle en $C_3$-$C_7$ ou oxo, ou un reste hydrocarboné aromatique monocyclique, qui peut être substitué par un groupe alkyle inférieur, par un groupe alcoxy inférieur ou par un halogène, ou un halogène; ou

d) on fait réagir un composé de formule générale

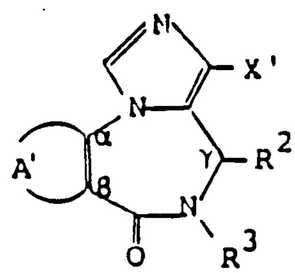

VI

où $R^2$ et $R^3$ ont la signification indiquée dans la revendication I et X' représente le brome ou l'iode et A' est un reste de formule (a), (b) ou (c), mais dans le cas où A' est un reste de formule (a) et $R^4$ et/ou $R^5$ un halogène, cet halogène est le fluor ou le chlore lorsque X est le brome, ou il est le fluor, le chlore ou le brome lorsque X est l'iode,

avec un composé de formule générale

$HC{\equiv}C-R^{64}$ VII

où $R^{64}$ représente l'hydrogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo; ou

e) on élimine le groupe protecteur d'un composé de formule générale

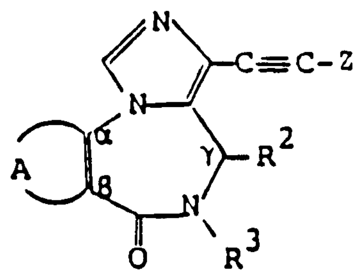

VIII

où $R^2$, $R^3$ et A ont la signification indiquée dans la revendication 1 et Z représente un groupe protecteur; ou

f) on remplace dans un groupe de formule générale

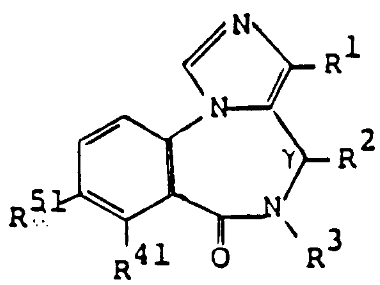

IX

où $R^1$, $R^2$ et $R^3$ ont la signification indiquée dans la revendication 1 et l'un des groupes $R^{41}$ et $R^{51}$ est un radical amino et l'autre est l'hydrogène, un halogène ou un groupe trifluorométhyle ou alkyle inférieur,

le groupe amino par un atome d'hydrogène ou d'halogène; ou

g) on réduit un composé de formule I, où $R^1$ représente un reste de formule (e) et où dans le cas où A est un reste de formule (a), $R^4$ et/ou $R^5$ ne sont pas l'iode, en le composé correspondant de formule I, ou $R^1$ est un reste de formule (d); ou

h) on traite un composé de formule I, ou $R^1$ représente un reste de formule (d) ou (e) et $R^6$ est un groupe hydrocarboné inférieur saturé substitué par un groupe hydroxy, avec un agent fournissant un reste alkyle inférieur; ou

i) on réduit le groupe carbonyle d'un composé de formule I, où $R^1$ représente le groupe (d) ou (e) et $R^6$ est un groupe hydrocarboné inférieur saturé substitué par un groupe oxo.

**23.** Médicaments, contenant un composé selon l'une quelconque des revendications 1 à 16 et un support thérapeutiquement inerte.

**24.** Médicaments selon la revendication 23, qui ont une activité anticonvulsivante, anxiolytique, de relaxation musculaire et sédative-hypnotique et/ou sont partiellement ou totalement des antagonistes sélectivement de certaines ou de la totalité des actions que présentent les 1,4-benzodiazépines ou autres substances à activité tranquillisante sur les récepteurs centraux de benzodiazépine.

**25.** Utilisation de composés selon l'une quelconque des revendications 1 à 16 pour la préparation de médicaments pour une utilisation dans la lutte contre les ou la prévention des convulsions, états d'anxiété, états de tension, états d'excitation et troubles du sommeil et/ou dans l'action antagoniste sélective partielle ou totale de certaines ou la totalité des actions que présentent les 1,4-benzodiazépines ou autres substances à activité tranquillisante sur les récepteurs centraux de benzodiazépine.

**Revendications pour l'Etat contractant suivant : ES**

**1.** Procédé pour la préparation de composés de formule générale

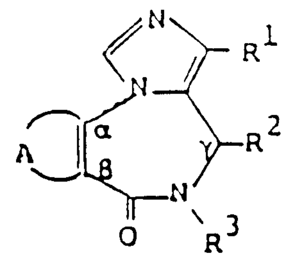

I

où A représente conjointement avec les deux atomes de carbone désignés par et l'un des groupes

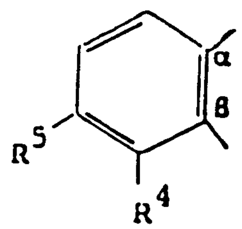

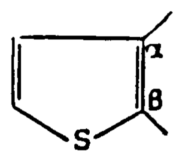

et

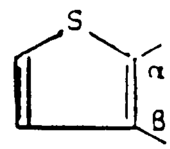

(a) (b) (c)

$R^1$ est un des groupes

$$-CH{=}CH-R^6 \quad \text{et} \quad -C{\equiv}C-R^6.$$

(d) (e)

$R^2$ désigne l'hydrogène et $R^3$ un groupe alkyle inférieur ou $R^2$ et $R^3$ représentent ensemble un groupe diméthylène ou triméthylène, $R^4$ et $R^5$ sont chacun l'hydrogène, un halogène ou un groupe trifluorométhyle ou alkyle inférieur et $R^6$ est l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo, tandis que les composés de formule I présentent en ce qui concerne l'atome de carbone désigné par $\gamma$ la configuration (S) ou (R,S), lorsque $R^2$ et $R^3$ représentent ensemble un groupe diméthylène ou triméthylène, et tandis que la double liaison présente dans le groupe (d) présente la configuration E et/ou Z, lorsque $R^6$ est différent de l'hydrogène, et les restes désignés par "inférieur" présentent jusqu'à 7 atomes de carbone,
caractérisé en ce qu'

a) on fait réagir un composé de formule générale

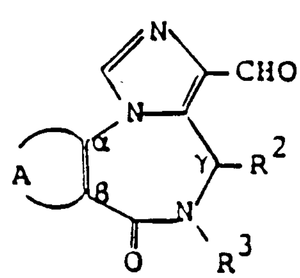

II

où A, $R^2$ et $R^3$ ont la signification susdite,
avec un composé de formule générale

$(Ar)_3P=CH-R^{61}$ III

où $R^{61}$ représente l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe alcoxy inférieur, cycloalkyle en $C_3$-$C_7$ ou oxo, et Ar est un reste hydrocarboné aromatique monocyclique, qui peut être substitué par un groupe alkyle inférieur, par un groupe alcoxy inférieur ou par un halogène; ou
b) on déshalogénhydrate un composé de formule générale

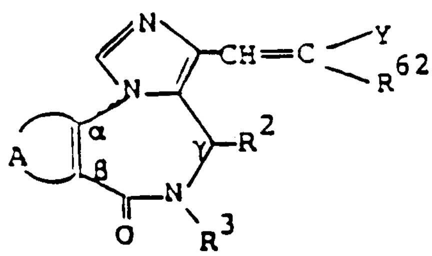

IV

où $R^{62}$ représente l'hydrogène ou un halogène et Y un halogène, et A, $R^2$ et $R^3$ ont la signification susdite; ou
c) on traite un composé de formule I, où $R^1$ représente le groupe (e) et $R^6$ l'hydrogène, avec un agent fournissant un reste hydrocarboné inférieur saturé éventuellement mono- ou disubstitué par un groupe hydroxy, alcoxy inférieur, cycloalkyle en $C_3$-$C_7$ ou oxo, ou un reste hydrocarboné aromatique monocyclique, qui peut être substitué par un groupe alkyle inférieur, par un groupe alcoxy inférieur ou par un halogène, ou un halogène; ou
d) on fait réagir un composé de formule générale

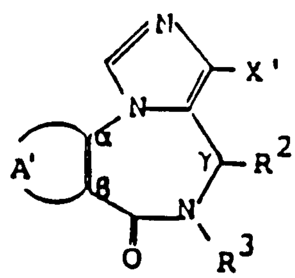

VI

où $R^2$, $R^3$ et A ont la signification susdite et X' représente le brome ou l'iode et A' est un reste de formule (a), (b) ou (c), mais dans le cas où A' est un reste de formule (a) et $R^4$ et/ou $R^5$ un halogène, cet halogène est le fluor ou le chlore lorsque X est le brome, ou il est le fluor, le chlore ou le brome lorsque X est l'iode,
avec un composé de formule générale

$HC\equiv C-R^{64}$ VII

où $R^{64}$ représente l'hydrogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo; ou
e) on élimine le groupe protecteur d'un composé de formule générale

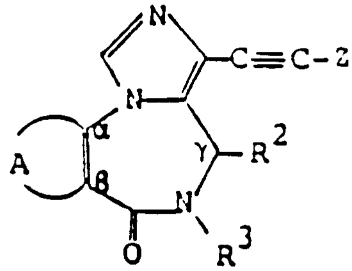

VIII

où $R^2$, $R^3$ et A ont la signification susdite et Z représente un groupe protecteur; ou
f) on remplace dans un groupe de formule générale

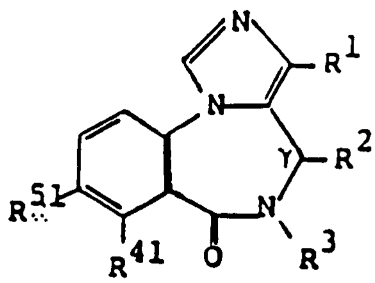

IX

où $R^1$, $R^2$, $R^3$ et "inférieur" ont la signification susdite et l'un des groupes $R^{41}$ et $R^{51}$ est un radical amino et l'autre est l'hydrogène, un halogène ou un groupe trifluorométhyle ou alkyle inférieur,
le groupe amino par un atome d'hydrogène ou d'halogène; ou
g) on réduit un composé de formule I, où $R^1$ représente un reste de formule (e) et où dans le cas où A est un reste de formule (a), $R^4$ et/ou $R^5$ ne sont pas l'iode, en le composé correspondant de formule I, ou $R^1$ est un reste de formule (d); ou
h) on traite un composé de formule I, où $R^1$ représente un reste de formule (d) ou (e) et $R^6$ est un groupe hydrocarboné inférieur saturé substitué par un groupe hydroxy, avec un agent fournissant un reste alkyle inférieur; ou
i) on réduit le groupe carbonyle d'un composé de formule I, ou $R^1$ représente le groupe (d) ou (e) et $R^6$ est un groupe hydrocarboné inférieur saturé substitué par un groupe oxo.

**2.** Procédé selon la revendication 1 pour la préparation de composés de formule I, ou $R^1$ représente le groupe $-CH=CH-R^6$ (d) et $R^6$ est l'hydrogène ou un groupe alkyle inférieur, hydroxyalkyle inférieur, alcoxy inférieur-alkyle inférieur, cycloalkyle en $C_3$-$C_7$,hydroxy-cycloalkyle en $C_4$-$C_7$, alcoxy inférieur-cycloalkyle en $C_4$-$C_7$, cycloalkyl en $C_3$-$C_7$-alkyle inférieur, phényle ou halogène, ou $R^1$ est le groupe $-C\equiv C-R^6$ (e) et $R^6$ est l'hydrogène ou un groupe alkyle inférieur, hydroxyalkyle inférieur, alcoxy inférieur-alkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur ou phényle, caractérisé en ce qu'on utilise l'une des variantes opératoires (a) à (h) et on utilise comme produit de départ dans la variante opératoire a) un composé de formule III, où $R^{61}$ représente l'hydrogène ou un groupe alkyle inférieur, alcoxy inférieur-alkyle inférieur, cycloalkyle en $C_3$-$C_7$,alcoxy inférieur-cycloalkyle en $C_4$-$C_7$, cycloalkyl en $C_3$-$C_7$-alkyle inférieur, phényle ou halogène, dans la variante opératoire b) un composé de formule IV, où $R^{62}$ représente l'hydrogène, dans la variante opératoire c) un agent fournissant un reste alkyle inférieur, un reste hydroxyalkyle inférieur, un reste alcoxy inférieur-alkyle inférieur, un reste cycloalkyle en $C_4$-$C_7$, un reste hydroxy-cycloalkyle en $C_4$-$C_7$, un reste alcoxy inférieur-cycloalkyle en $C_4$-$C_7$, un reste cycloalkyl en $C_3$-$C_7$-alkyle inférieur ou un reste phényle, dans la variante opératoire d) un composé de formule VII, où $R^{64}$ possède la signification indiquée plus haut dans le cas du groupe (e) pour $R^6$, et dans la variante opératoire h) un composé de formule I, où $R^1$ représente un reste de formule (d) ou (e) et $R^6$ est un groupe hydroxyalkyle inférieur ou hydroxy-cycloalkyle en $C_4$-$C_7$.

**3.** Procédé selon l'une des revendications 1 ou 2, dans lequel $R^1$ représente le groupe $-C\equiv C-R^6$ (e).

**4.** Procédé selon la revendication 3, dans lequel $R^6$ représente l'hydrogène ou un groupe alkyle inférieur, hydroxyalkyle inférieur, alcoxyalkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur, cycloalkyl en $C_3$-$C_7$-hydroxyalkyle inférieur ou cycloalkyl en $C_3$-$C_7$-alcoxyalkyle inférieur.

**5.** Procédé selon la revendication 4, dans lequel $R^6$ représente l'hydrogène ou un groupe alkyle inférieur, 1-hydroxyalkyle inférieur, 1-alcoxyalkyle inférieur, cycloalkyle en $C_3$-$C_7$, 1-hydroxy-cycloalkyle en $C_4$-$C_7$, 1-alcoxy inférieur-cycloalkyle en $C_4$-$C_7$ ou 1-(cycloalkyl en $C_3$-$C_7$)-hydroxyalkyle inférieur, en particulier alkyle inférieur, 1-hydroxyalkyle inférieur ou cycloalkyle en $C_3$-$C_7$.

**6.** Procédé selon l'une quelconque des revendications 1 à 5, dans lequel $R^2$ représente l'hydrogène et $R^3$ un groupe méthyle ou dans lequel $R^2$ et $R^3$ représentent ensemble un groupe diméthylène ou triméthylène et l'atome de carbone désigné par $\gamma$ présente la configuration (S).

**7.** Procédé selon l'une quelconque des revendications 1 à 6, dans lequel A représente un reste de formule (a) et l'un des groupes $R^4$ et $R^5$ est l'hydrogène et l'autre l'hydrogène ou un halogène.

**8.** Procédé selon la revendication 7, dans lequel $R^4$ et $R^5$ sont tous deux l'hydrogène ou $R^4$ représente l'hydrogène et $R^5$ le fluor ou $R^4$ le chlore ou le brome et $R^5$ l'hydrogène.

**9.** Procédé selon la revendication 1, caractérisé en ce qu'on prépare la 7-chloro-4,5-dihydro-5-méthyl-3-(1-propionyl)-6H-imidazo[1,5-a][1,4]benzodiazépine-6-one.

**10.** Procédé selon la revendication 1, caractérisé en ce qu'on prépare la 7-chloro-4,5-dihydro-3-(3-hydroxy-3-méthyl-1-butinyl)-5-méthyl-6H-imidazo[1,5-a][1,4]benzodiazépine-6-one.

**11.** Procédé selon la revendication 1, caractérisé en ce qu'on prépare la 7-bromo-4,5-dihydro-3(3-hydroxy-3-méthyl-1-butinyl)-5-méthyl-6H-imidazo[1,5-a][1,4]benzodiazépine-6-one.

**12.** Procédé selon la revendication 1, caractérisé en ce qu'on prépare la 7-chloro-4,5-dihydro-3-(3-hydroxy-1-butinyl)-5-méthyl-6H-imidazo[1,5-a][1,4]benzodiazépine-6-one.

**13.** Procédé selon la revendication 1, caractérisé en ce qu'on prépare la 4,5-dihydro-5-méthyl-3-(1-propinyl)-6H-imidazo[1,5-a][1,4]benzodiazépine-6-one.

**14.** Procédé selon la revendication 1, caractérisé en ce qu'on prépare la 7-chloro-4,5-dihydro-5-méthyl-3-(3-méthyl-1-butinyl)-6H-imidazo[1,5-a][1,4]benzodiazépine-6-one.

**15.** Procédé selon la revendication 1, caractérisé en ce qu'on prépare la 7-chloro-4,5-dihydro-3-(3-hydroxy-1-propinyl)-5-méthyl-6H-imidazo[1,5-a][1,4]benzodiazépine-6-one.

**16.** Procédé selon la revendication 1, caarctérisé en ce qu'on prépare la 7-chloro-3-(cyclopropyléthinyl)-4,5-dihydro-5-méthyl-6H-imidazo[1,5-a][1,4]benzodiazépine-6-one.

**17.** Utilisation de composés de formule I définie dans la revendication 1 pour la préparation de médicaments pour une utilisation dans la lutte contre les ou la prévention des convulsions, états d'anxiété, états de tension, états d'excitation et troubles du sommeil et/ou dans l'action antagoniste sélective partielle ou totale de certaines ou la totalité des actions que présentent les 1,4-benzodiazépines ou autres substances à activité tranquillisante sur les récepteurs centraux de benzodiazépine.

**Revendications pour l'Etat contractant suivant : GR**

**1.** Procédé pour la préparation de composés de formule générale

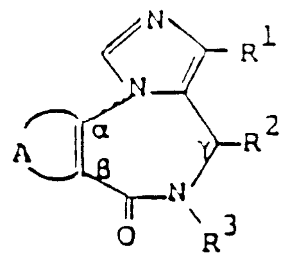

I

où A représente conjointement avec les deux atomes de carbone désignés par et l'un des groupes

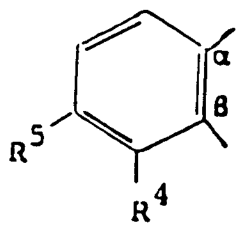

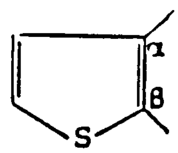

et

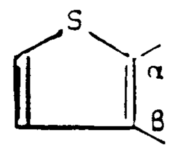

(a) (b) (c)

$R^1$ est un des groupes

$-CH{=}CH-R^6$ et $-C{\equiv}C-R^6$,

(d) (e)

$R^2$ désigne l'hydrogène et $R^3$ un groupe alkyle inférieur ou $R^2$ et $R^3$ représentent ensemble un groupe diméthylène ou triméthylène, $R^4$ et $R^5$ sont chacun l'hydrogène, un halogène ou un groupe trifluorométhyle ou alkyle inférieur et $R^6$ est l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo, tandis que les composés de formule I présentent en ce qui concerne l'atome de carbone désigné par $\gamma$ la configuration (S) ou (R,S), lorsque $R^2$ et $R^3$ représentent ensemble un groupe diméthylène ou triméthylène, et tandis que la double liaison présente dans le groupe (d) présente la configuration E et/ou Z, lorsque $R^6$ est différent de l'hydrogène, et les restes désignés par "inférieur" présentent jusqu'à 7 atomes de carbone,
caractérisé en ce qu'

a) on fait réagir un composé de formule générale

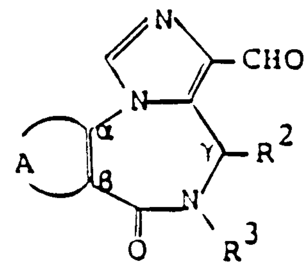

II

où A, $R^2$ et $R^3$ ont la signification susdite,
avec un composé de formule générale

$(Ar)_3P = CH\text{-}R^{61}$ III

ou $R^{61}$ représente l'hydrogène, un halogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe alcoxy inférieur, cycloalkyle en $C_3$-$C_7$ ou oxo, et Ar est un reste hydrocarboné aromatique monocyclique, qui peut être substitué par un groupe alkyle inférieur, par un groupe alcoxy inférieur ou par un halogène; ou

b) on déshalogénhydrate un composé de formule générale

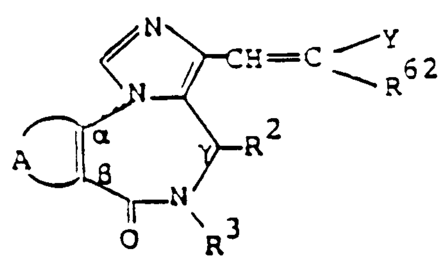

IV

où $R^{62}$ représente l'hydrogène ou un halogène et Y un halogène, et A, $R^2$ et $R^3$ ont la signification susdite; ou

c) on traite un composé de formule I, ou $R^1$ représente le groupe (e) et $R^6$ l'hydrogène, avec un agent fournissant un reste hydrocarboné inférieur saturé éventuellement mono- ou disubstitué par un groupe hydroxy, alcoxy inférieur, cycloalkyle en $C_3$-$C_7$ ou oxo, ou un reste hydrocarboné aromatique monocyclique, qui peut être substitué par un groupe alkyle inférieur, par un groupe alcoxy inférieur ou par un halogène, ou un halogène; ou

d) on fait réagir un composé de formule générale

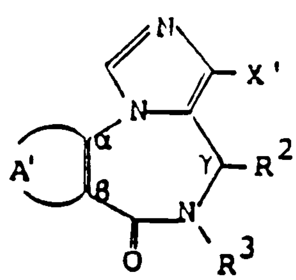

VI

où $R^2$, $R^3$ et A ont la signification susdite et X' représente le brome ou l'iode et A' est un reste de formule (a), (b) ou (c), mais dans le cas où A' est un reste de formule (a) et $R^4$ et/ou $R^5$ un

halogène, cet halogène est le fluor ou le chlore lorsque X est le brome, ou il est le fluor, le chlore ou le brome lorsque X est l'iode,
avec un composé de formule générale

$HC\equiv C-R^{64}$ VII

où $R^{64}$ représente l'hydrogène, un reste hydrocarboné aromatique monocyclique, qui peut être substitué par un groupe alkyle inférieur, par un groupe alcoxy inférieur ou par un halogène, ou un groupe hydrocarboné inférieur saturé éventuellement mono- ou disubstitué par un groupe hydroxy, alcoxy inférieur, cycloalkyle en $C_3$-$C_7$ ou oxo; ou
e) on élimine le groupe protecteur d'un composé de formule générale

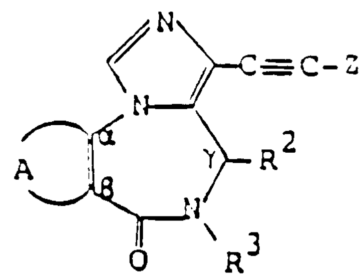

VIII

ou $R^2$, $R^3$ et A ont la signification susdite et Z représente un groupe protecteur; ou
f) on remplace dans un groupe de formule générale

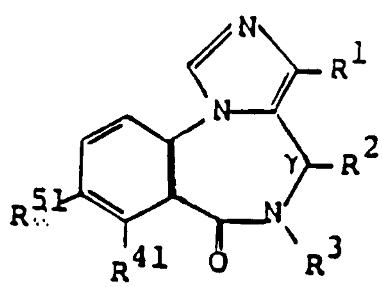

IX

où $R^1$, $R^2$, $R^3$ et "inférieur" ont la signification susdite et l'un des groupes $R^{41}$ et $R^{51}$ est un radical amino et l'autre est l'hydrogène, un halogène ou un groupe trifluorométhyle ou alkyle inférieur,
le groupe amino par un atome d'hydrogène ou d'halogène; ou
g) on réduit un composé de formule I, où $R^1$ représente un reste de formule (e) et où dans le cas où A est un reste de formule (a), $R^4$ et/ou $R^5$ ne sont pas l'iode, en le composé correspondant de formule I, où $R^1$ est un reste de formule (d); ou
h) on traite un composé de formule I, où $R^1$ représente un reste de formule (d) ou (e) et $R^6$ est un groupe hydrocarboné inférieur saturé substitué par un groupe hydroxy, avec un agent fournissant un reste alkyle inférieur; ou
i) on réduit le groupe carbonyle d'un composé de formule I, ou $R^1$ représente le groupe (d) ou (e) et $R^6$ est un groupe hydrocarboné inférieur saturé substitué par un groupe oxo.

**2.** Procédé selon la revendication 1 pour la préparation de composés de formule I, où $R^1$ représente le groupe $-CH=CH-R^6$ (d) et $R^6$ est l'hydrogène ou un groupe alkyle inférieur, hydroxyalkyle inférieur, alcoxy inférieur-alkyle inférieur, cycloalkyle en $C_3$-$C_7$,hydroxy-cycloalkyle en $C_4$-$C_7$, alcoxy inférieur-cycloalkyle en $C_4$-$C_7$, cycloalkyl en $C_3$-$C_7$-alkyle inférieur, phényle ou halogène, ou $R^1$ est le groupe $-C\equiv C-R^6$ (e) et $R^6$ est l'hydrogène ou un groupe alkyle inférieur, hydroxyalkyle inférieur, alcoxy inférieur-alkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur ou phényle, caractérisé en ce qu'on utilise l'une des variantes opératoires (a) à (h) et on utilise comme produit de départ dans la variante opératoire a) un composé de formule III, ou $R^{61}$ représente l'hydrogène ou un groupe alkyle inférieur, alcoxy inférieur-alkyle inférieur, cycloalkyle en $C_3$-$C_7$,alcoxy inférieur-cycloalkyle en $C_4$-$C_7$, cycloalkyl en $C_3$-$C_7$-alkyle

inférieur, phényle ou halogène, dans la variante opératoire b) un composé de formule IV, où $R^{62}$ représente l'hydrogène, dans la variante opératoire c) un agent fournissant un reste alkyle inférieur, un reste hydroxyalkyle inférieur, un reste alcoxy inférieur-alkyle inférieur, un reste cycloalkyle en $C_4$-$C_7$, un reste hydroxy-cycloalkyle en $C_4$-$C_7$, un reste alcoxy inférieur-cycloalkyle en $C_4$-$C_7$, un reste cycloalkyl en $C_3$-$C_7$-alkyle inférieur ou un reste phényle, dans la variante opératoire d) un composé de formule VII, où $R^{64}$ possède la signification indiquée plus haut dans le cas du groupe (e) pour $R^6$, et dans la variante opératoire h) un composé de formule I, où $R^1$ représente un reste de formule (d) ou (e) et $R^6$ est un groupe hydroxyalkyle inférieur ou hydroxy-cycloalkyle en $C_4$-$C_7$.

**3.** Procédé selon l'une des revendications 1 ou 2, dans lequel $R^1$ représente le groupe -C≡C-$R^6$ (e).

**4.** Procédé selon la revendication 3, dans lequel $R^6$ représente l'hydrogène ou un groupe alkyle inférieur, hydroxyalkyle inférieur, alcoxyalkyle inférieur, cycloalkyle en $C_3$-$C_7$, hydroxy-cycloalkyle en $C_4$-$C_7$, alcoxy inférieur-cycloalkyle en $C_4$-$C_7$, cycloalkyl en $C_3$-$C_7$-alkyle inférieur, cycloalkyl en $C_3$-$C_7$-hydroxyalkyle inférieur ou cycloalkyl en $C_3$-$C_7$-alcoxyalkyle inférieur.

**5.** Procédé selon la revendication 4, dans lequel $R^6$ représente l'hydrogène ou un groupe alkyle inférieur, 1-hydroxyalkyle inférieur, 1-alcoxyalkyle inférieur, cycloalkyle en $C_3$-$C_7$, 1-hydroxy-cycloalkyle en $C_4$-$C_7$, 1-alcoxy inférieur-cycloalkyle en $C_4$-$C_7$ ou 1-(cycloalkyl en $C_3$-$C_7$)-hydroxyalkyle inférieur, en particulier alkyle inférieur, 1-hydroxyalkyle inférieur ou cycloalkyle en $C_3$-$C_7$.

**6.** Procédé selon l'une quelconque des revendications 1 à 5, dans lequel $R^2$ représente l'hydrogène et $R^3$ un groupe méthyle ou dans lequel $R^2$ et $R^3$ représentent ensemble un groupe diméthylène ou triméthylène et l'atome de carbone désigné par $\gamma$ présente la configuration (S).

**7.** Procédé selon l'une quelconque des revendications 1 à 6, dans lequel A représente un reste de formule (a) et l'un des groupes $R^4$ et $R^5$ est l'hydrogène et l'autre l'hydrogène ou un halogène.

**8.** Procédé selon la revendication 7, dans lequel $R^4$ et $R^5$ sont tous deux l'hydrogène ou $R^4$ représente l'hydrogène et $R^5$ le fluor ou $R^4$ le chlore ou le brome et $R^5$ l'hydrogène.

**9.** Procédé selon la revendication 1, caractérisé en ce qu'on prépare la 7-chloro-4,5-dihydro-5-méthyl-3-(1-propionyl)-6H-imidazo[1,5-a][1,4]benzodiazépine-6-one.

**10.** Procédé selon la revendication 1, caractérisé en ce qu'on prépare la 7-chloro-4,5-dihydro-3-(3-hydroxy-3-méthyl-1-butinyl)-5-méthyl-6H-imidazo[1,5-a][1,4]benzodiazépine-6-one.

**11.** Procédé selon la revendication 1, caractérisé en ce qu'on prépare la 7-bromo-4,5-dihydro-3-(3-hydroxy-3-méthyl-1-butinyl)-5-méthyl-6H-imidazo[1,5-a][1,4]benzodiazépine-6-one.

**12.** Procédé selon la revendication 1, caractérisé en ce qu'on prépare la 7-chloro-4,5-dihydro-3-(3-hydroxy-1-butinyl)-5-méthyl-6H-imidazo[1,5-a][1,4]benzodiazépine-6-one.

**13.** Procédé selon la revendication 1, caractérisé en ce qu'on prépare la 4,5-dihydro-5-méthyl-3-(1-propinyl)-6H-imidazo[1,5-a][1,4]benzodiazépine-6-one.

**14.** Procédé selon la revendication 1, caractérisé en ce qu'on prépare la 7-chloro-4,5-dihydro-5-méthyl-3-(3-méthyl-1-butinyl)-6H-imidazo[1,5-a][1,4]benzodiazépine-6-one.

**15.** Procédé selon la revendication 1, caractérisé en ce qu'on prépare la 7-chloro-4,5-dihydro-3-(3-hydroxy-1-propinyl)-5-méthyl-6H-imidazo[1,5-a][1,4]benzodiazépine-6-one.

**16.** Procédé selon la revendication 1, caarctérisé en ce qu'on prépare la 7-chloro-3-(cyclopropyléthinyl)-4,5-dihydro-5-méthyl-6H-imidazo[1,5-a][1,4]benzodiazépine-6-one.

**17.** Procédé pour la préparation de médicaments, en particulier d'agents qui ont une activité anticonvulsivante, anxiolytique, de relaxation musculaire et sédative-hypnotique et/ou sont partiellement ou totalement des antagonistes sélectivement de certaines ou de la totalité des actions que présentent les 1,4-benzodiazépines ou autres substances à activité tranquillisante sur les récepteurs centraux de benzodiazépine, caractérisé en ce qu'on met sous une forme de présentation galénique un ou plusieurs composés de formule I définie dans la revendication 1 et, si on le souhaite, une ou plusieurs autres substances à activité thérapeutique, conjointement avec un support thérapeutiquement inerte.

**18.** Utilisation de composés de formule I définie dans la revendication 1 pour la préparation de médicaments pour une utilisation dans la lutte contre les ou la prévention des convulsions, états d'anxiété, états de tension, états d'excitation et troubles du sommeil et/ou dans l'action antagoniste sélective partielle ou totale de certaines ou la totalité des actions que présentent les 1,4-benzodiazépines ou autres substances à activité tranquillisante sur les récepteurs centraux de benzodiazépine.

**19.** Composés de formule générale

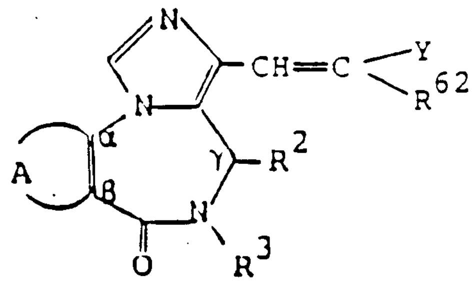

IVa

où Y et $R^{62}$ représentent chacun un halogène, et A, $R^2$ et $R^3$ ont la signification indiquée dans la revendication 1.

**20.** Composés de formule générale

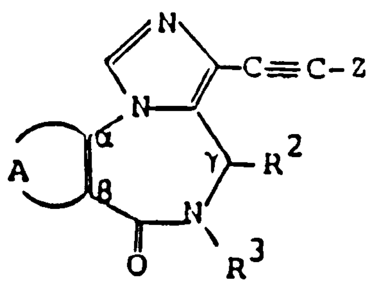

VIII

où Z représente un groupe protecteur, et A, $R^2$ et $R^3$ ont la signification indiquée dans la revendication 1.

**21.** Composés de formule générale

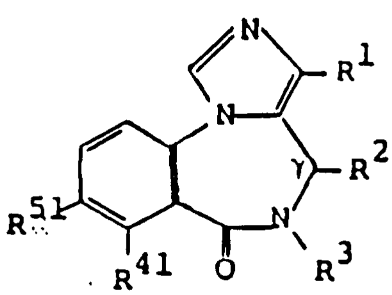

IX

où l'un des groupes $R^{41}$ et $R^{51}$ est un radical amino et l'autre est l'hydrogène, un halogène ou un groupe trifluorométhyle ou alkyle inférieur, et $R^1$, $R^2$ et $R^3$ ont la signification indiquée dans la

revendication 1.